# EUROPEAN PATENT APPLICATION

(11) **EP 4 847 501 A1**
(43) Date of publication of application: **02.12.2026**
(21) Application number: 25745278.9
(22) Date of filing: 20.01.2025
(51) Int. Cl.: C07D 403/10, H10K 85/60, H10K 50/11, C09K 11/06, H10K 101/00

(54) **ORGANIC OPTOELECTRONIC DIODE COMPOUND, ORGANIC OPTOELECTRONIC DIODE COMPOSITION, ORGANIC OPTOELECTRONIC DIODE, AND DISPLAY DEVICE**

(30) Priority: 22.01.2024 KR 20240009692
(71) Applicant: SAMSUNG SDI CO., LTD., Yongin-si, Gyeonggi-do 17084 (KR)
(72) Inventor: KIM, Changwoo, Suwon-si, Gyeonggi-do 16678 (KR); LEE, Mijin, Suwon-si, Gyeonggi-do 16678 (KR); KANG, Dong Min, Suwon-si, Gyeonggi-do 16678 (KR); KIM, Byungku, Suwon-si, Gyeonggi-do 16678 (KR); LEE, Byoungkwan, Suwon-si, Gyeonggi-do 16678 (KR); NOH, Wonho, Suwon-si, Gyeonggi-do 16678 (KR); LEE, Jihee, Suwon-si, Gyeonggi-do 16678 (KR); JANG, Jinseok, Suwon-si, Gyeonggi-do 16678 (KR); HUH, Dalho, Suwon-si, Gyeonggi-do 16678 (KR); KIM, Hyung Sun, Suwon-si, Gyeonggi-do 16678 (KR)
(74) Representative: Michalski Hüttermann & Partner mbB
(86) International application number: PCT/KR2025/099092
(87) International publication number: WO 2025/159613

(57) **Abstract**

Disclosed are a compound for an organic optoelectronic device represented by Chemical Formula 1A or Chemical Formula 1B, an composition for an organic optoelectronic device including the same, an organic optoelectronic device, and a display device.

Details of Chemical Formula 1A and Chemical Formula 1B are as defined in the specification.

## Description

### [Technical Field]

A compound for an organic optoelectronic device, a composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

### [Disclosure]

### [Technical Problem]

An embodiment provides a compound for an organic optoelectronic device capable of lowering a driving voltage and implementing an organic optoelectronic device with high-efficiency and long life-span.

Another embodiment provides a composition for an organic optoelectronic device including the compound for the organic optoelectronic device.

Another embodiment provides an organic optoelectronic device including the compound for the organic optoelectronic device or composition for the organic optoelectronic device.

Another embodiment provides a display device including the organic optoelectronic device.

### [Technical Solution]

According to an embodiment, a compound for an organic optoelectronic device represented by Chemical Formula 1A or Chemical Formula 1B.

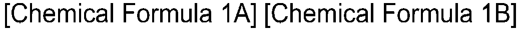

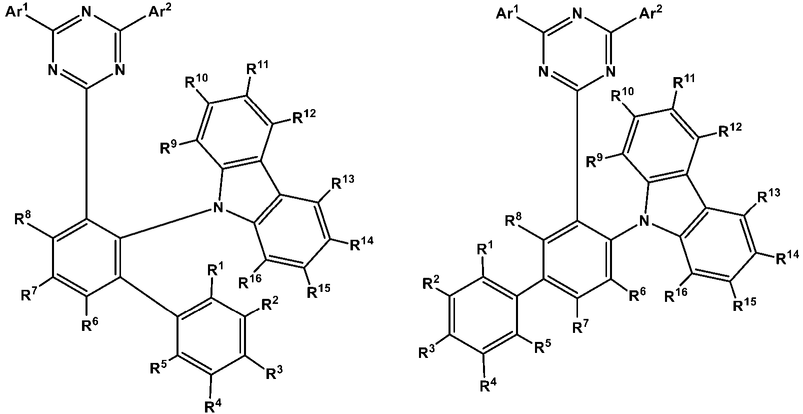

In Chemical Formula 1,
Ar¹ and Ar² are each independently a phenyl group unsubstituted or substituted with deuterium or a C6 to C20 aryl group; a biphenyl group unsubstituted or substituted with deuterium or a C6 to C20 aryl group; or a terphenyl group unsubstituted or substituted with deuterium or a C6 to C20 aryl group,
provided that at least one of Ar1 and Ar2 is a biphenyl group unsubstituted or substituted with deuterium or a C6 to C20 aryl group; or a terphenyl group unsubstituted or substituted with deuterium or a C6 to C20 aryl group,
R¹ to R⁵ are each independently hydrogen, deuterium, or a substituted or unsubstituted phenyl group,
R⁶ to R⁸ are each independently hydrogen or deuterium, and
R⁹ to R¹⁶ are each independently hydrogen, deuterium or a substituted or unsubstituted C6 to C20 aryl group.

According to another embodiment, a composition for an organic optoelectronic device including a first compound and a second compound is provided.

The first compound may be the aforementioned compound for the organic optoelectronic device, and the second compound may be represented by Chemical Formula 2; a combination of Chemical Formula 3 and Chemical Formula 4; or Chemical Formula 5.

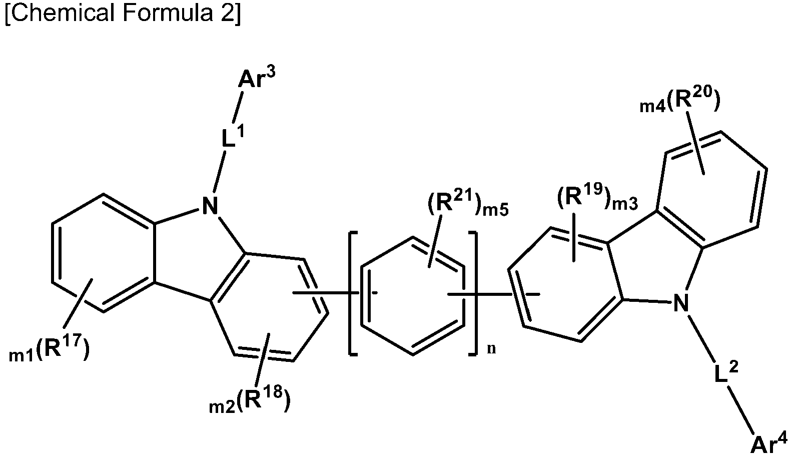

In Chemical Formula 2,
R¹⁷ to R²¹ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L¹ and L² are each independently a single bond or a substituted or unsubstituted C6 to C20 arylene group,
m1, m4, and m5 are each independently one of integers from 1 to 4,
m2 and m3 are each independently one of integers from 1 to 3, and
n is one of integers from 0 to 2; 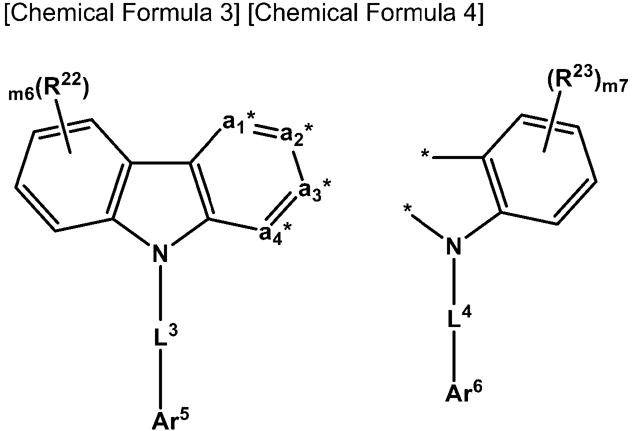
wherein, in Chemical Formula 3 and Chemical Formula 4,
among a₁* to a₄* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L³, and L⁴ are each independently a single bond or a substituted or unsubstituted C6 to C20 arylene group,
R^{a}, R²², and R²³ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m6 and m7 are each independently one of integers from 1 to 4; 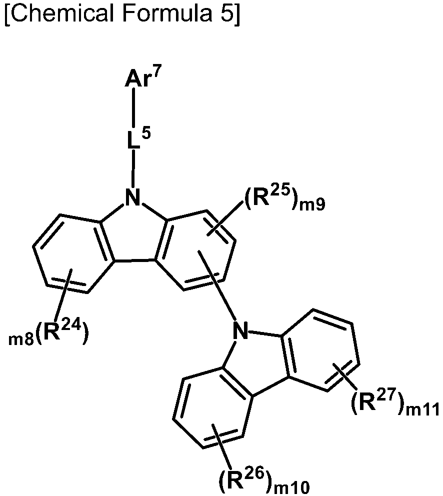
wherein, in Chemical Formula 5,
L⁵ is each independently a single bond or a substituted or unsubstituted C6 to C20 arylene group,
R²⁴ to R²⁷ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m8, m10, and m11 are each independently one of integers from 1 to 4, and
m9 is one of integers from 1 to 3.

According to another embodiment, an organic optoelectronic device includes an anode and a cathode facing each other, and at least one organic layer between the anode and the cathode, and the organic layer includes the compound for the organic optoelectronic device.

According to another embodiment, a display device including the organic optoelectronic device is provided.

### [Advantageous Effects]

An organic optoelectronic device with high efficiency and long life-span while lowering the driving voltage may be realized.

### [Description of the Drawings]

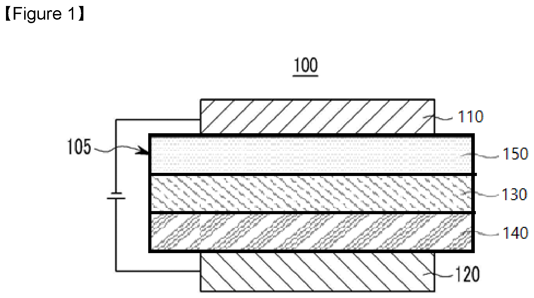

FIG. 1 is a cross-sectional view illustrating an organic light emitting diode according to an embodiment.

### <Description of Symbols>

100: organic light emitting diode
105: organic layer
110: cathode
120: anode
130: light emitting layer
140: hole transport region
150: electron transport region

### [Best Mode]

Hereinafter, embodiments of the present invention are described in detail. However, these embodiments are exemplary, and this disclosure is not limited thereto.

In the present specification, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In a specific example of the present invention, the "substituted" refers to replacement of at least one hydrogen of a substituent or a compound by deuterium, a C1 to C20 alkyl group, a C1 to C5 alkylsilyl group, a C6 to C20 aryl group, a C2 to C20 heteroaryl group, or a cyano group. In a specific example of the present invention, the "substituted" refers to replacement of at least one hydrogen of a substituent or a compound by deuterium, a C1 to C5 alkyl group, a C1 to C5 alkylsilyl group, a C6 to C18 aryl group, a C2 to C18 heteroaryl group, or a cyano group. In a specific example of the present invention, the "substituted" refers to replacement of at least one hydrogen of a substituent or a compound by deuterium, a cyano group, a methyl group, an ethyl group, an n-propyl group, an iso-propyl group, an n-butyl group, an iso-butyl group, a sec-butyl group, a tert-butyl group, a trimethylsilyl group, a phenyl group, a biphenyl group, a terphenyl group, or a naphthyl group.

In the present specification, "unsubstituted" refers to non-replacement of a hydrogen atom by another substituent and remaining of the hydrogen atom.

In the present specification, hydrogen (-H)" may include "deuterium substitution (-D)" or "tritium substitution (-T)."

In the present specification, when a definition is not otherwise provided, "hetero" refers to one including one to three heteroatoms selected from N, O, S, P, and Si, and remaining carbons in one functional group.

In the present specification, "aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quaterphenyl group, and the like, and two or more hydrocarbon aromatic moieties are fused directly or indirectly to provide a non-aromatic fused ring, for example a fluorenyl group.

The aryl group may include a monocyclic, polycyclic, or fused ring polycyclic (i.e., rings sharing adjacent pairs of carbon atoms) functional group.

In the present specification, "heterocyclic group" is a generic concept of a heteroaryl group, and may include at least one heteroatom selected from N, O, S, P, and Si instead of carbon (C) in a cyclic compound such as aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" may refer to aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted benzophenanthrenyl group, a substituted or unsubstituted triphenylene group, a substituted or unsubstituted perylenyl group, a substituted or unsubstituted fluorenyl group, a substituted or unsubstituted indenyl group, or a combination thereof, but is not limited thereto.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, a substituted or unsubstituted benzonaphthol furanyl group, a substituted or unsubstituted benzonaphthothiophenyl group, a substituted or unsubstituted benzofuranofluorenyl group, a substituted or unsubstituted benzothiophenefluorenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an embodiment is described.

A compound for an organic optoelectronic device according to an embodiment is represented by Chemical Formula 1A or Chemical Formula 1B.

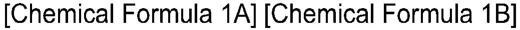

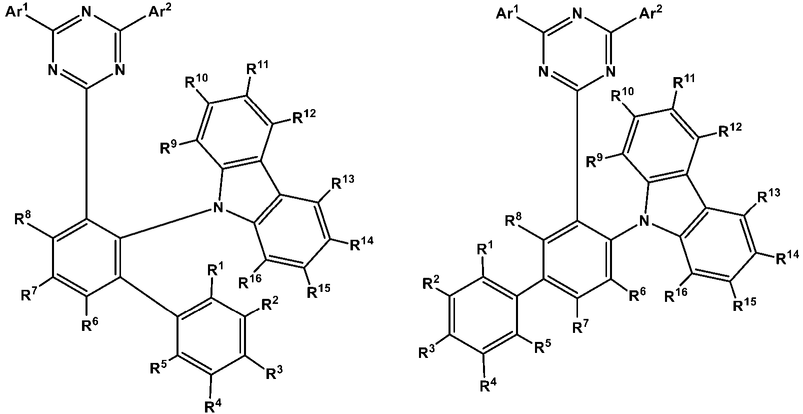

In Chemical Formula 1,
Ar¹ and Ar² are each independently a phenyl group unsubstituted or substituted with deuterium or a C6 to C20 aryl group; a biphenyl group unsubstituted or substituted with deuterium or a C6 to C20 aryl group; or a terphenyl group unsubstituted or substituted with deuterium or a C6 to C20 aryl group,
provided that at least one of Ar1 and Ar2 is a biphenyl group unsubstituted or substituted with deuterium or a C6 to C20 aryl group; or a terphenyl group unsubstituted or substituted with deuterium or a C6 to C20 aryl group,
R¹ to R⁵ are each independently hydrogen, deuterium, or a substituted or unsubstituted phenyl group,
R⁶ to R⁸ are each independently hydrogen or deuterium, and
R⁹ to R¹⁶ are each independently hydrogen, deuterium or a substituted or unsubstituted C6 to C20 aryl group.

The compound represented by Chemical Formula 1A and Chemical Formula 1B has an N-carbazole substituted at the ortho position of phenylene linked with triazine, a substituted or unsubstituted phenyl group substituted at the meta position of phenylene with respect to the triazine, and the triazine is substituted with at least one biphenyl group or terphenyl group.

The compound represented by Chemical Formula 1A or Chemical Formula 1B is a structure containing ortho-carbazole with respect to triazine and has particularly excellent energy transfer efficiency to a phosphorescent dopant, so it may be used as an advantageous material as a phosphorescent host. The meta-biphenyl structure substituted on the triazine included in Chemical Formula 1A or Chemical Formula 1B speeds up electron mobility and increases stability compared to mono-phenyl, thereby improving life-span when applied as a phosphorescent host. In addition, the phenyl group at the end of the meta-biphenyl structure is substituted at the ortho and para positions with respect to the carbazole moiety, respectively, thereby helping expand the resonance structure of the carbazole moiety, which helps stabilize the hole transport part in the molecule. As a result, a charge balance optimization effect between the electron transport part and the hole transport part in the molecule is obtained, further helping improve life-span.

In addition, by substituting carbazole at the ortho position of the triazine, the dihedral angle increases due to steric hindrance between triazine and carbazole, so the triazine moiety and the carbazole moiety are twisted with each other, increasing the dihedral angle. This soon appears in a form that is mostly separated without electron cloud overlap between the HOMO level and the LUMO level, and since it has a small ΔEst, fast energy transfer is possible, so it shows high efficiency characteristics, especially when applied as a phosphorescent host. In addition, the side reaction path in the excited state is reduced, so a further life-span increasing effect may be obtained.

In addition, by substituting at least one biphenyl group or terphenyl group on the triazine, electron mobility is accelerated and stability is increased, thereby improving driving improvement effect and life-span.

As an example, Ar¹ and Ar² may each independently be a phenyl group unsubstituted or substituted with deuterium, a phenyl group, or a biphenyl group; a biphenyl group unsubstituted or substituted with deuterium, a phenyl group, or a biphenyl group; or a terphenyl group unsubstituted or substituted with deuterium, a phenyl group, or a biphenyl group.

As an example, R⁹ to R¹⁶ may each independently be hydrogen, deuterium, or a substituted or unsubstituted C6 to C12 aryl group.

As a specific example, R⁹ to R¹⁶ may each independently be hydrogen, deuterium, a substituted or unsubstituted phenyl group, or a substituted or unsubstituted biphenyl group.

In a most specific embodiment, the compound represented by Chemical Formula 1 may be one selected from the compounds listed in Group 1, but is not limited thereto.

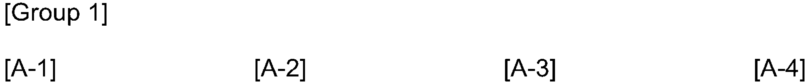

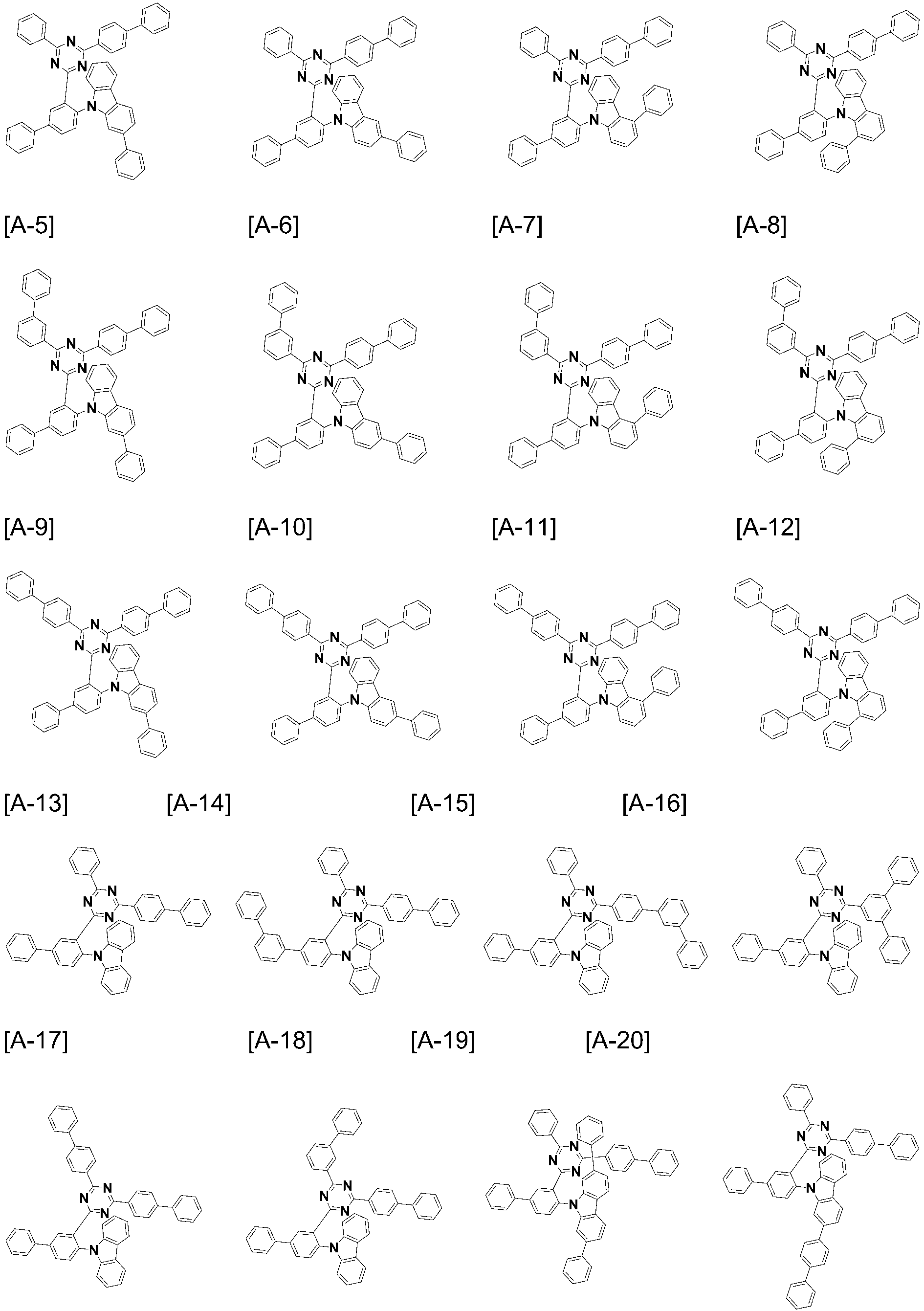

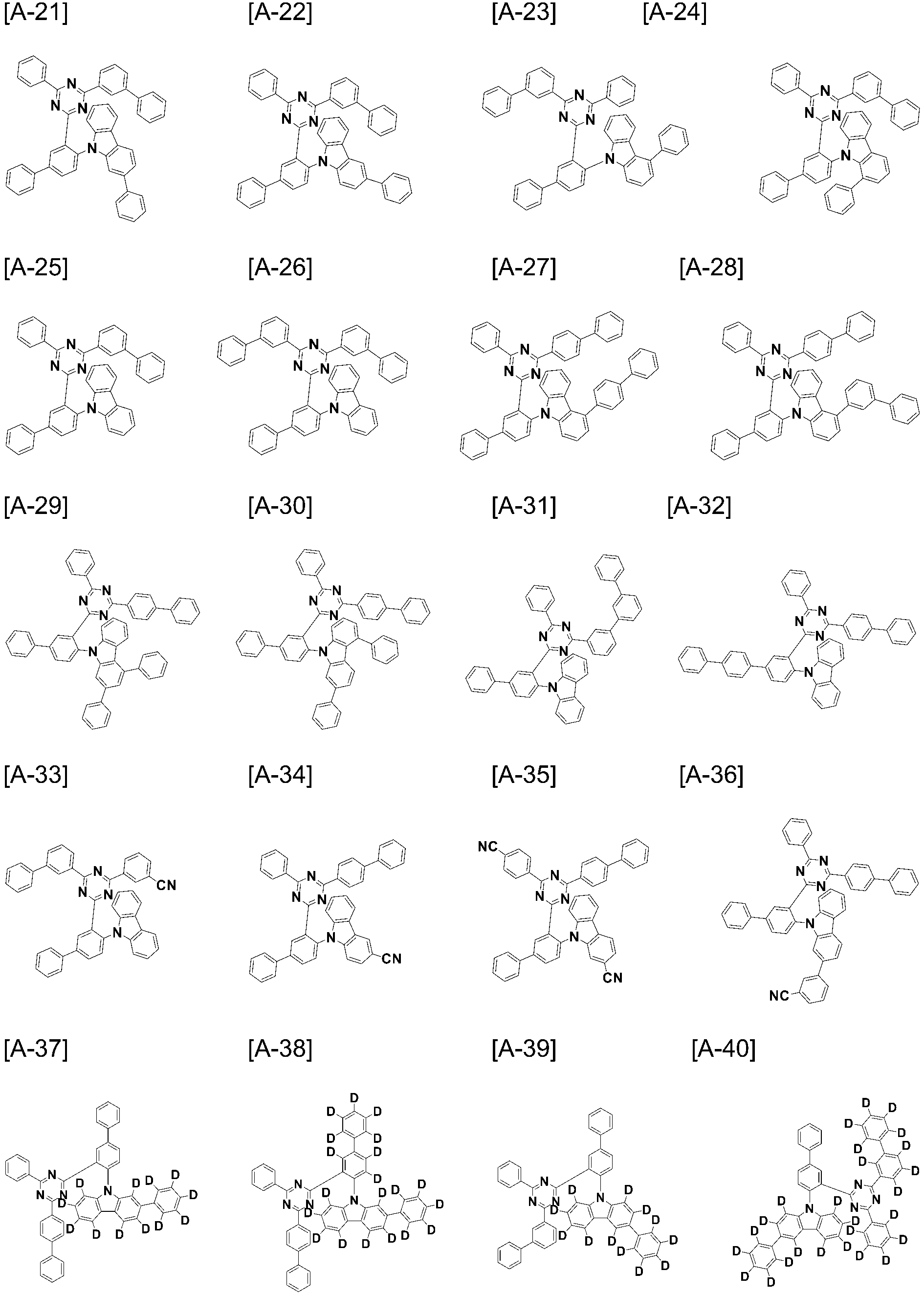

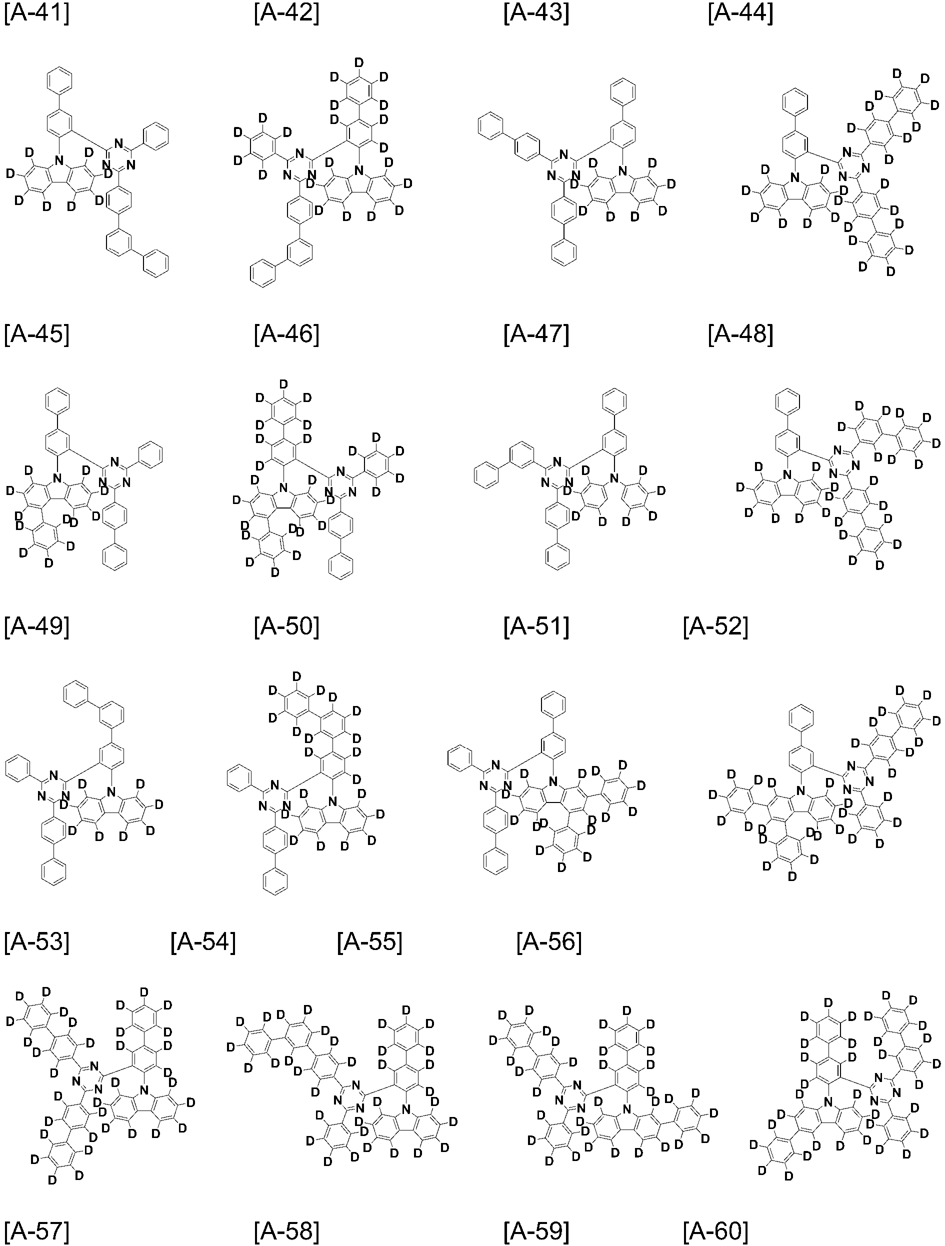

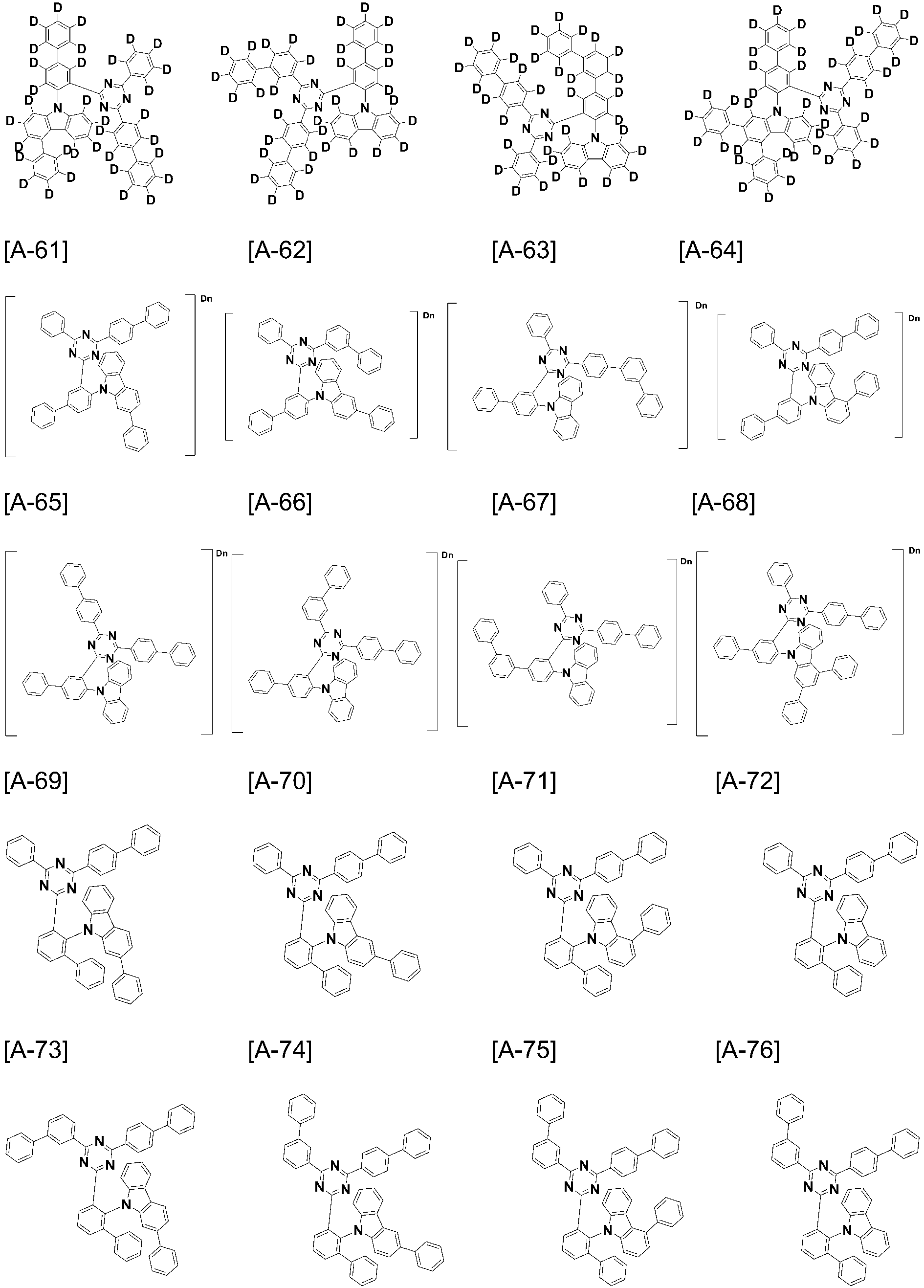

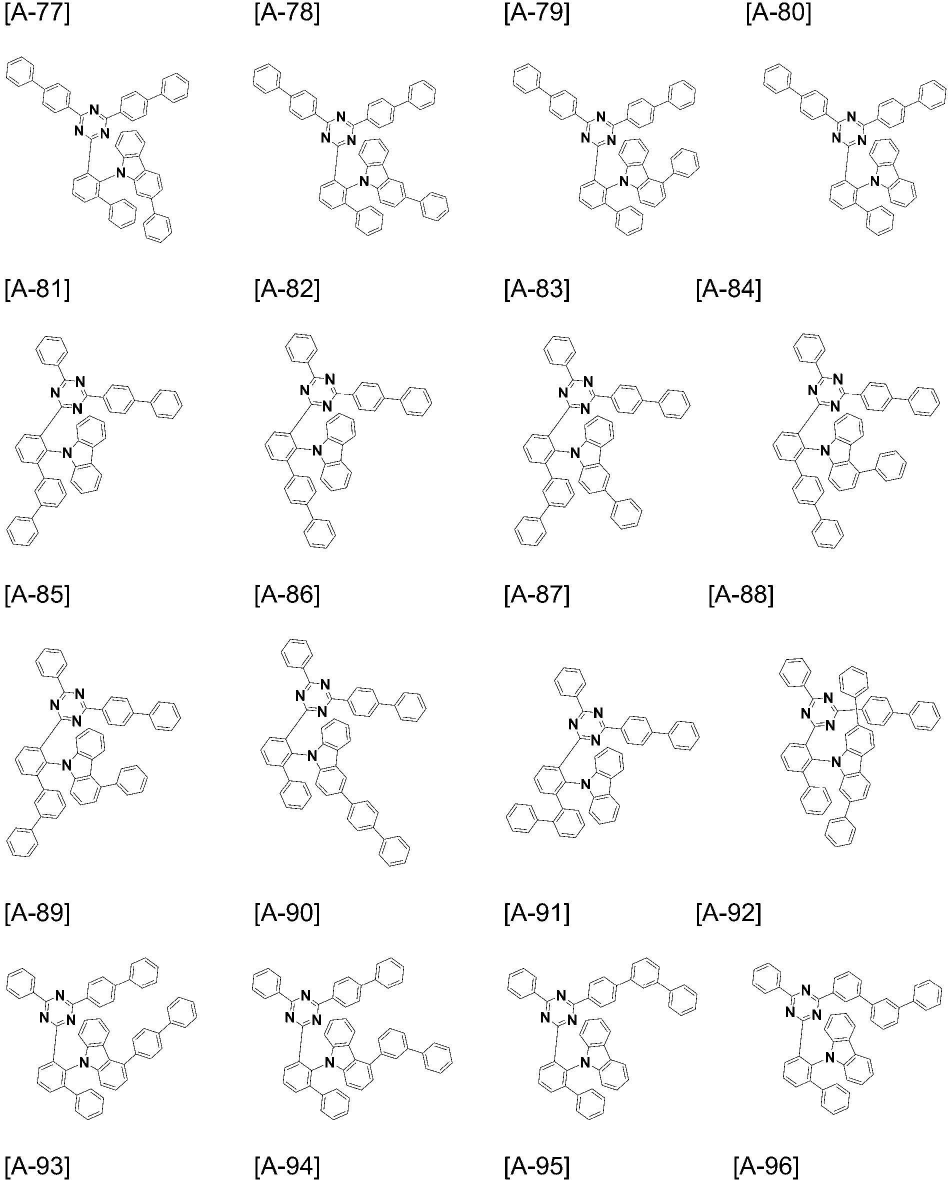

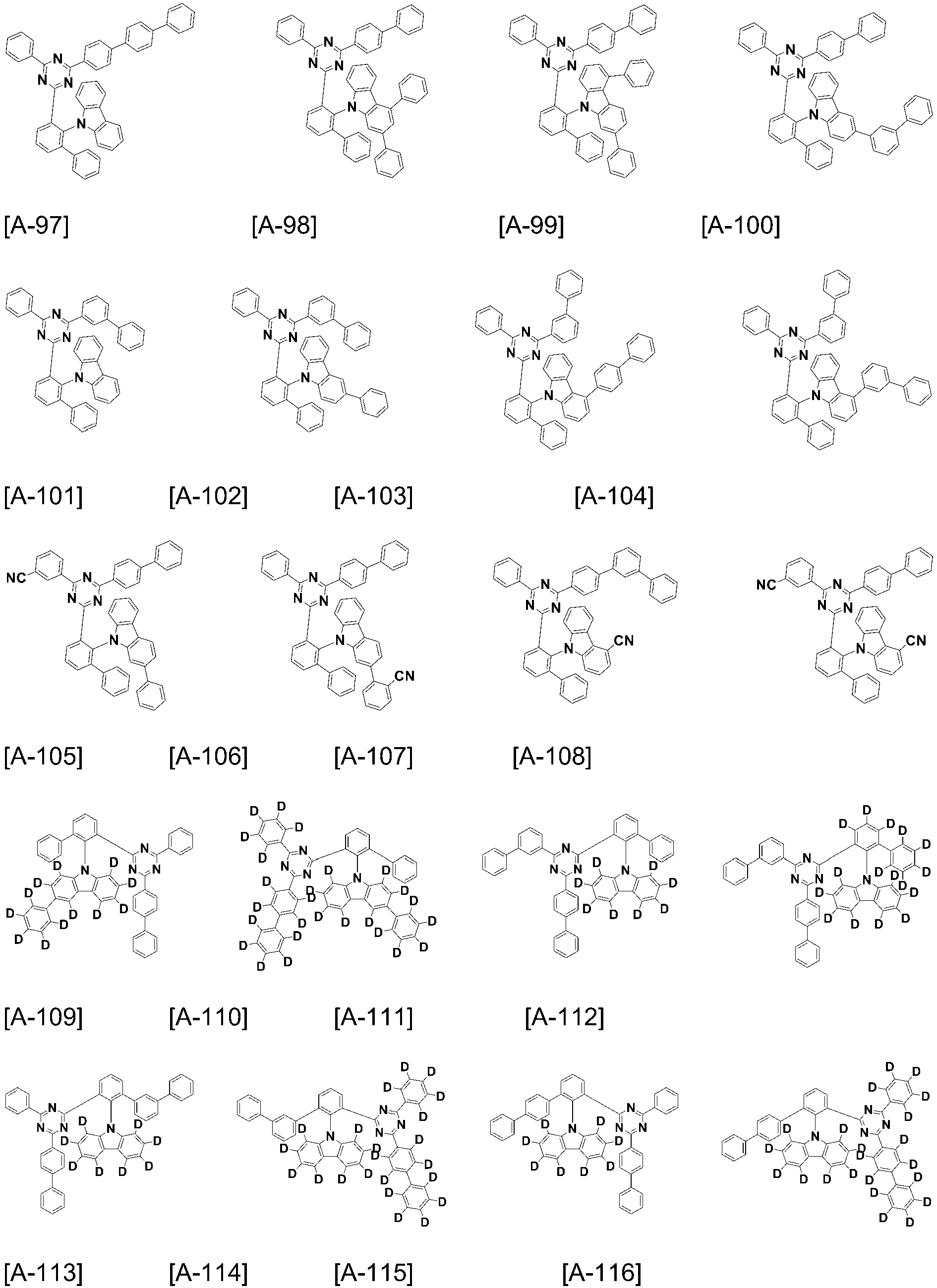

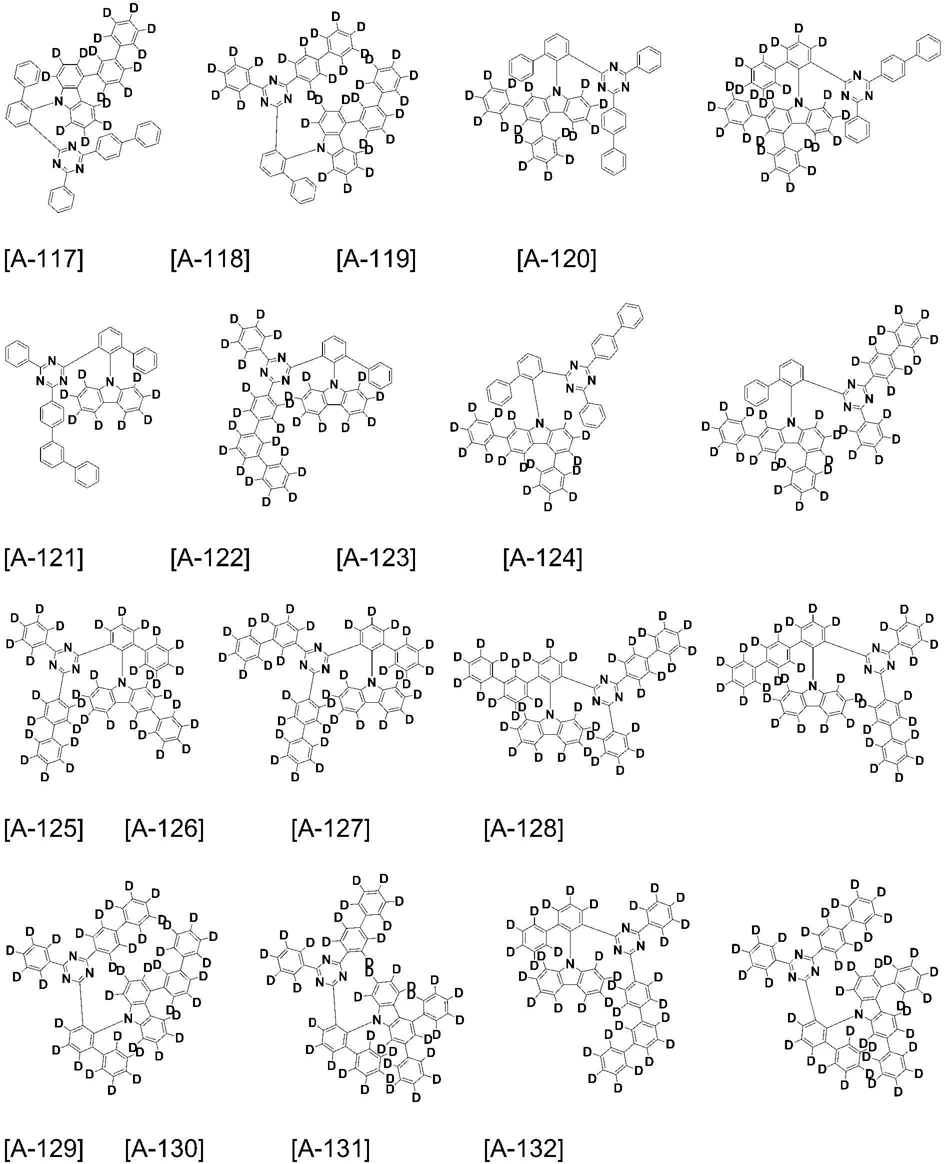

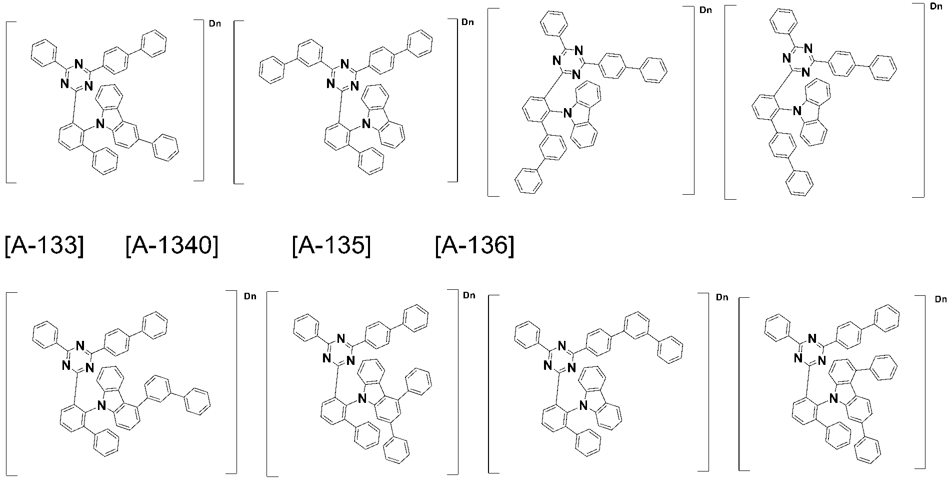

(Dn means the number of substituted deuteriums and indicates a structure in which one or more deuteriums are substituted).

A composition for an organic optoelectronic device according to another embodiment includes a first compound and a second compound, wherein the first compound is the aforementioned compound for the organic optoelectronic device, and the second compound may be represented by Chemical Formula 2; a combination of Chemical Formula 3 and Chemical Formula 4; or Chemical Formula 5.

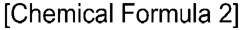

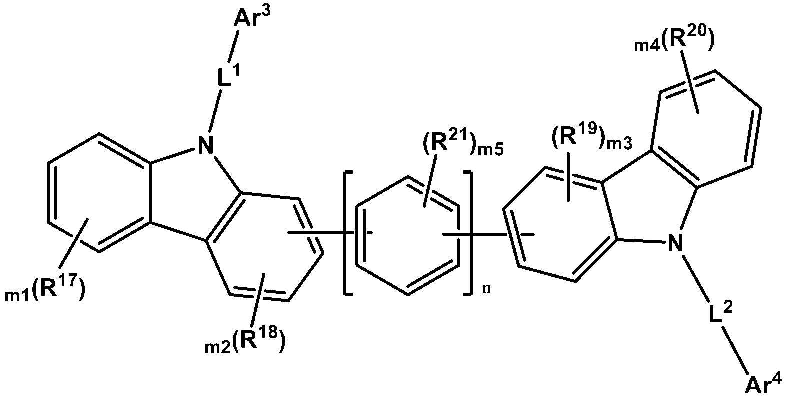

In Chemical Formula 2,
R¹⁷ to R²¹ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L¹ and L² are each independently a single bond or a substituted or unsubstituted C6 to C20 arylene group,
m1, m4, and m5 are each independently one of integers from 1 to 4,
m2 and m3 are each independently one of integers from 1 to 3, and
n is one of integers from 0 to 2; 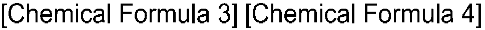 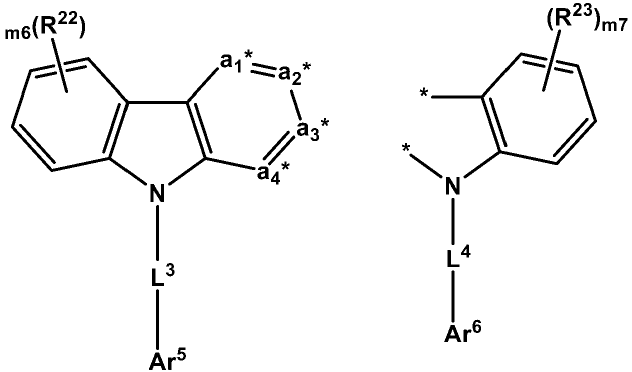
wherein, in Chemical Formula 3 and Chemical Formula 4,
among a₁* to a₄* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L³, and L⁴ are each independently a single bond or a substituted or unsubstituted C6 to C20 arylene group,
R^{a}, R²² and R²³ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m6 and m7 are each independently one of integers from 1 to 4; 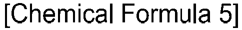 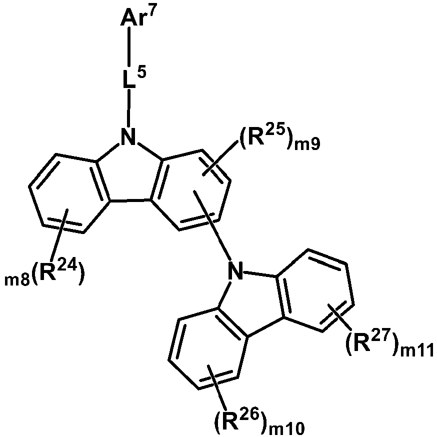
wherein, in Chemical Formula 5,
L⁵ is each independently a single bond or a substituted or unsubstituted C6 to C20 arylene group,
R²⁴ to R²⁷ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m8, m10, and m11 are each independently one of integers from 1 to 4, and
m9 is one of integers from 1 to 3.

The second compound may be used in a light emitting layer together with the first compound to increase the mobility of charges and increase stability, thereby improving luminous efficiency and life-span characteristics.

In Chemical Formula 2, when m1 is 2 or more, each R¹⁷ may be the same as or different from each other.

In Chemical Formula 2, when m2 is 2 or more, each R¹⁸ may be the same as or different from each other.

In Chemical Formula 2, when m3 is 2 or more, each R¹⁹ may be the same as or different from each other.

In Chemical Formula 2, when m4 is 2 or more, each R²⁰ may be the same as or different from each other.

In Chemical Formula 2, when m5 is 2 or more, each R²¹ may be the same as or different from each other.

In Chemical Formula 3 and Chemical Formula 4, when m6 is 2 or more, each R²² may be the same as or different from each other.

In Chemical Formula 3 and Chemical Formula 4, when m7 is 2 or more, each R²³ may be the same as or different from each other.

In Chemical Formula 3 and Chemical Formula 4, when substituted with 2 or more R^{a}s, each R^{a} may be the same as or different from each other.

In Chemical Formula 5, when m8 is 2 or more, each R²⁴ may be the same as or different from each other.

In Chemical Formula 5, when m9 is 2 or more, each R²⁵ may be the same as or different from each other.

In Chemical Formula 5, when m10 is 2 or more, each R²⁶ may be the same as or different from each other.

In Chemical Formula 5, when m11 is 2 or more, each R²⁷ may be the same as or different from each other.

As an example, in Chemical Formula 2, Ar³ and Ar⁴ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group,
in Chemical Formula 2, L¹ and L² may each independently be a single bond, a substituted or unsubstituted phenylene group, or a substituted or unsubstituted biphenylene group,
in Chemical Formula 2, R¹⁷ to R²¹ may each independently be hydrogen, deuterium, or a substituted or unsubstituted C6 to C12 aryl group, and
n may be 0 or 1.

As an example, in Chemical Formula 2, "substituted" refers to replacement of at least one hydrogen by deuterium, a C1 to C4 alkyl group, a C6 to C18 aryl group, or a C2 to C30 heteroaryl group.

For example, in Chemical Formula 2, Ar³ and Ar⁴ may each independently be a substituted or unsubstituted carbazolyl group, a substituted or unsubstituted dibenzothiophenyl group, a substituted or unsubstituted dibenzofuranyl group, or a substituted or unsubstituted fluorenyl group.

In another specific embodiment of the present invention, Chemical Formula 2 may be represented by any one of Chemical Formula 2-1 to Chemical Formula 2-15.

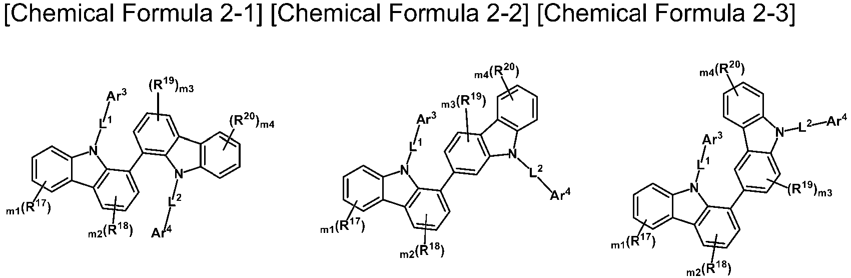

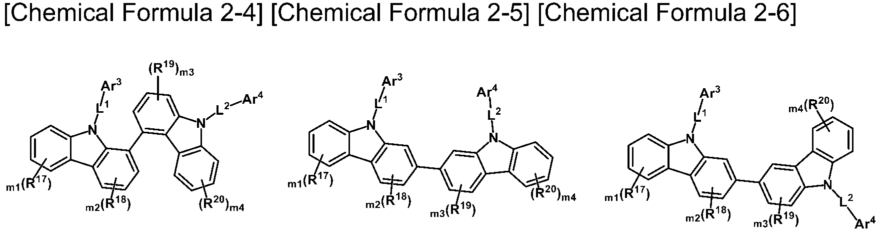

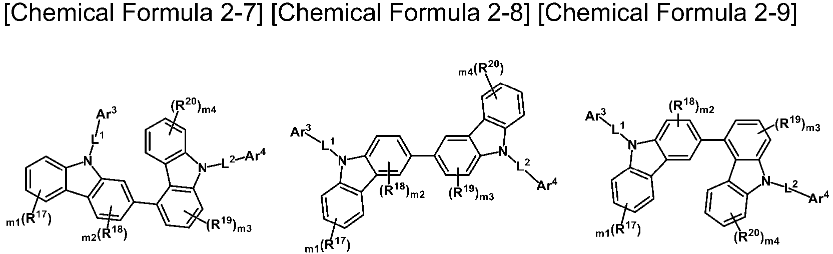

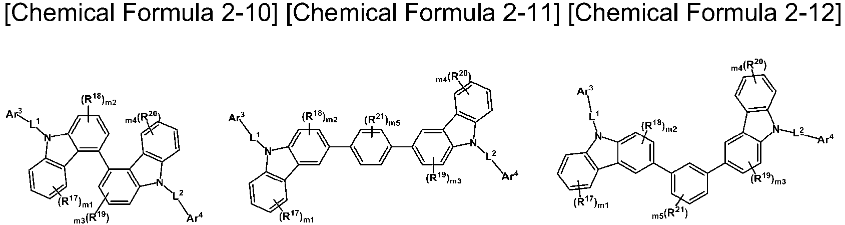

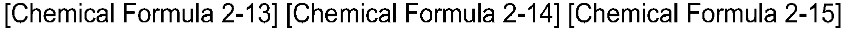

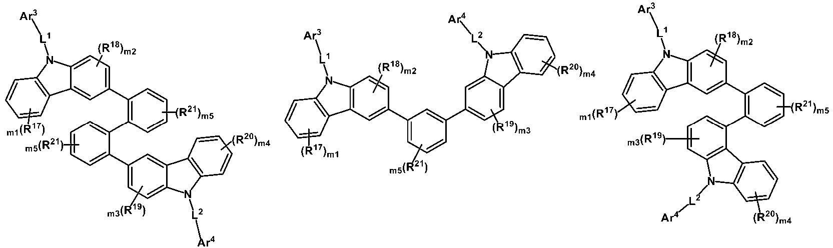

In Chemical Formula 2-1 to Chemical Formula 2-15, R¹⁷ to R²¹ are each independently hydrogen, deuterium, or a substituted or unsubstituted C6 to C12 aryl group, and L¹-Ar³ and L²-Ar⁴ may each independently be one of the substituents listed in Group I.

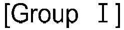

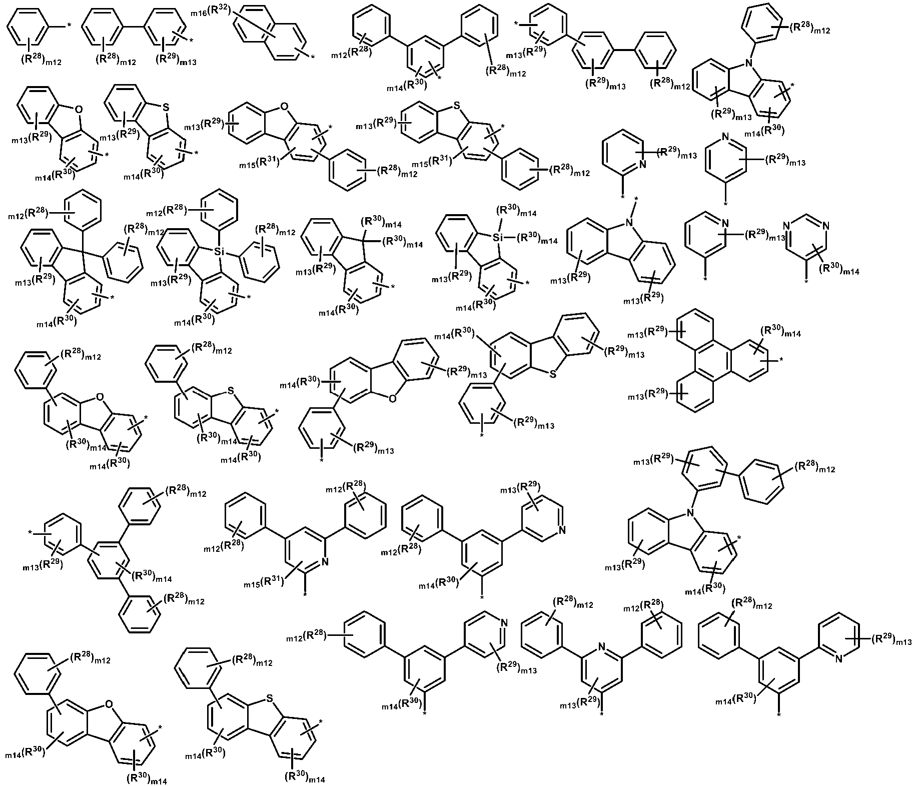

In Group I,
R²⁸ to R³² are each independently hydrogen, deuterium, a cyano group, a C1 to C10 alkyl group, or a C6 to C12 aryl group,
m12 is one of integers from 1 to 5,
m13 is one of integers from 1 to 4,
m14 is one of integers from 1 to 3,
m15 is an integer of 1 or 2,
m16 is one of integers from 1 to 7, and
* is a linking point.

In Group I, when m12 is 2 or more, each R²⁸ may be the same as or different from each other.

In Group I, when m13 is 2 or more, each R²⁹ may be the same as or different from each other.

In Group I, when m14 is 2 or more, each R³⁰ may be the same as or different from each other.

In Group I, when m15 is 2, each R³¹ may be the same as or different from each other.

In Group I, when m16 is 2 or more, each R³² may be the same as or different from each other.

The combination of Chemical Formula 3 and Chemical Formula 4 may be represented by, for example, any one of Chemical Formula 3A, Chemical Formula 3B, Chemical Formula 3C, Chemical Formula 3D and Chemical Formula 3E.

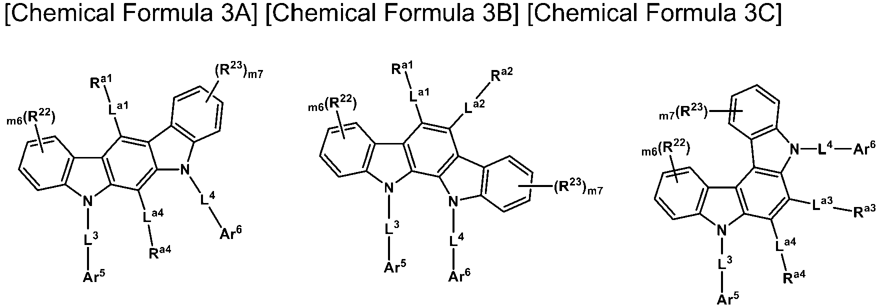

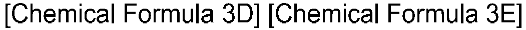

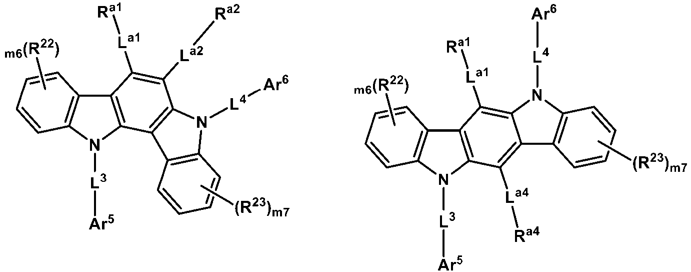

In Chemical Formula 3A to Chemical Formula 3E, L³, L⁴, Ar⁵, Ar⁶, R²², R²³, m6, and m7 are the same as described above,
L^{a1} to L^{a4} are as defined for L³ and L⁴ above, and
R^{a1} to R^{a4} are as defined for R²², and R²³ above.

For example, in Chemical Formulas 3 and 4, Ar⁴ and Ar⁵ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and
R^{a1} to R^{a4}, R²², and R²³ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In another specific embodiment of the present invention, in Chemical Formulas3 and 4, L³-Ar⁵ and L⁴-Ar⁶ may each independently be selected from the substituents listed in Group I .

In an embodiment, R^{a1} to R^{a4}, R²², and R²³ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, R^{a1} to R^{a4}, R²², and R²³ may each independently be hydrogen, deuterium, a cyano group, or a substituted or unsubstituted phenyl group,
in a specific embodiment, R^{a1} to R^{a4}, R²², and R²³ may each independently be hydrogen, deuterium, or a substituted or unsubstituted phenyl group.

In another specific embodiment of the present invention, the second compound may be represented by Chemical Formula 2-8, and in Chemical Formula 2-8, Ar³ and Ar⁴ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, L¹ and L² may each independently be a single bond, or a substituted or unsubstituted C6 to C20 arylene group, R¹⁷ to R²⁰ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 2-8, R¹⁷ to R²⁰ are each independently hydrogen, deuterium, or a substituted or unsubstituted C6 to C12 aryl group, and L¹-Ar³ and L²-Ar⁴ may each independently be one of substituents listed in Group I.

In another specific embodiment of the present invention, the second compound may be represented by Chemical Formula 3C, and in Chemical Formula 3C, L^{a3} and L^{a4} may be a single bond, L³ and L⁴ may each independently be a single bond or a substituted or unsubstituted C6 to C12 arylene group, R²², R²³, R^{a3}, and R^{a4} may each be hydrogen, deuterium or phenyl group, and Ar⁵ and Ar⁶ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 3C, L^{a3} and L^{a4} may be a single bond, R²², R²³, R^{a3}, and R^{a4} may each independently be hydrogen, deuterium, or a C6 to C12 aryl group, and L³-Ar⁵ and L⁴-Ar⁶ may each independently be one of substituents listed in Group I .

Chemical Formula 5 may be represented by, for example, any one of Chemical Formula 5-1 to Chemical Formula 5-4.

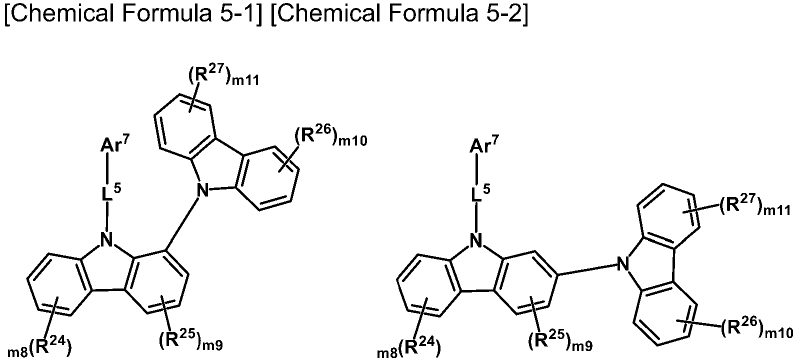

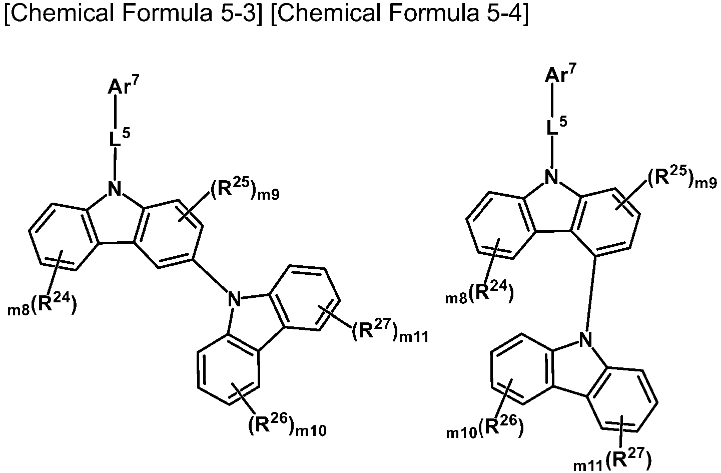

In Chemical Formula 5-1 to Chemical Formula 5-4, L⁵, Ar⁷, R²⁴ to R²⁷, and m8 to m11 aree same as described above.

For example, in Chemical Formula 5, Ar⁷ may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and
R²⁴ to R²⁷ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, in Chemical Formula 5, L⁵-Ar⁷ may be selected from the substituents listed in Group I .

For example, R²⁴ to R²⁷ may each independently be hydrogen, deuterium, a cyano group, or a substituted or unsubstituted phenyl group.

For example, the second compound for the organic optoelectronic device may be one selected from the compounds listed in Group 2, but is not limited thereto.

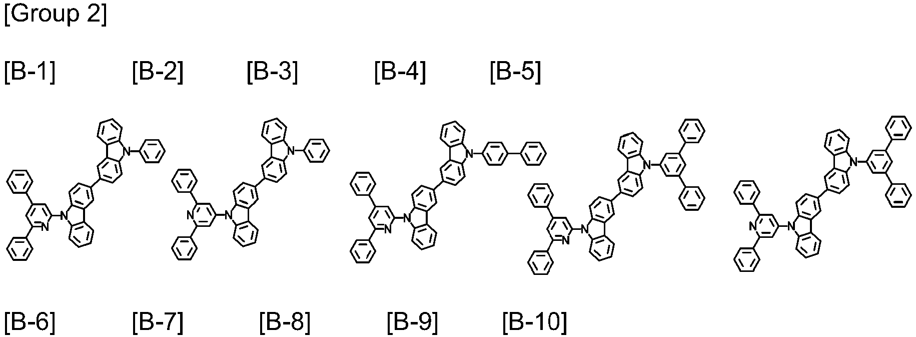

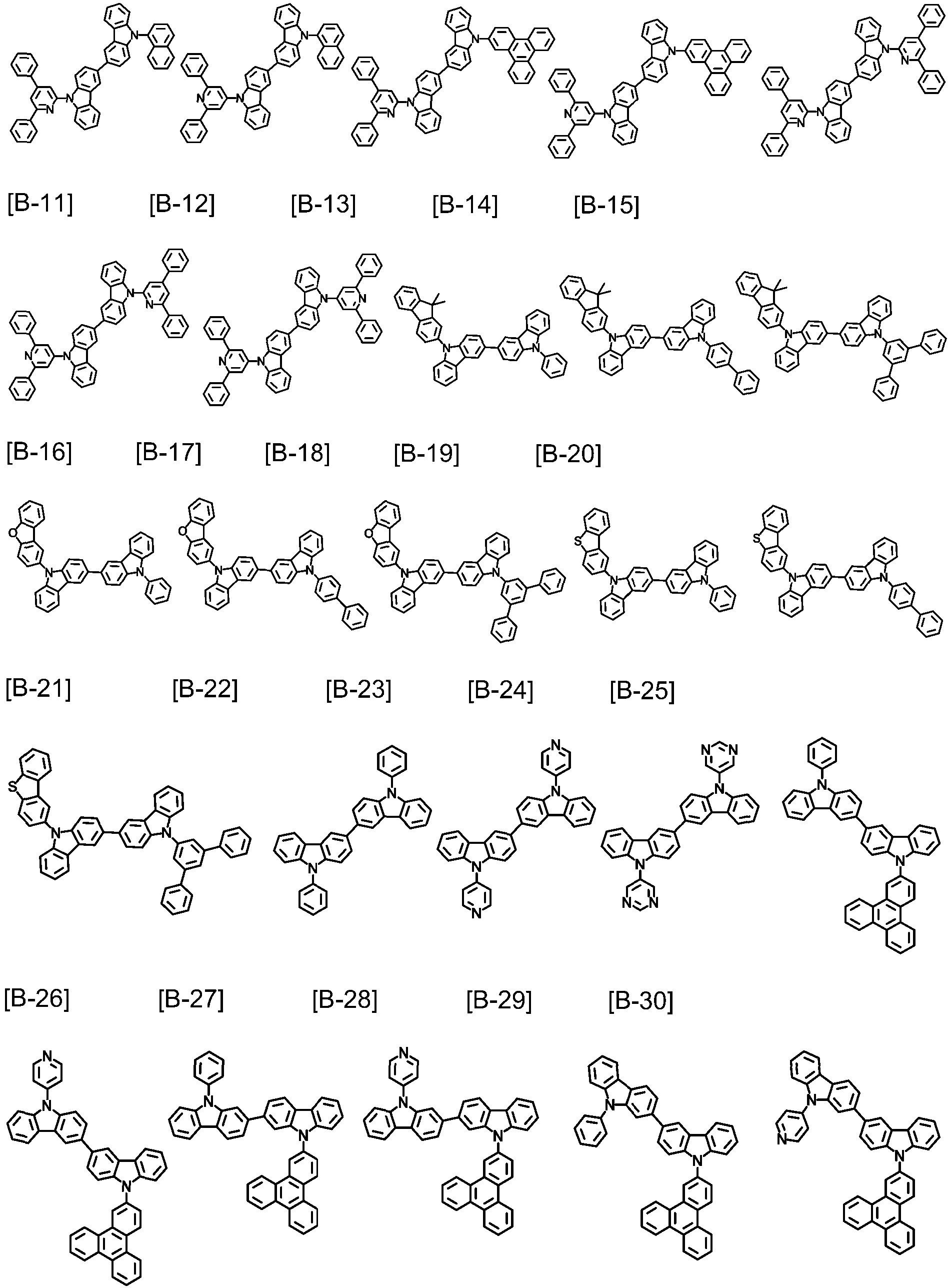

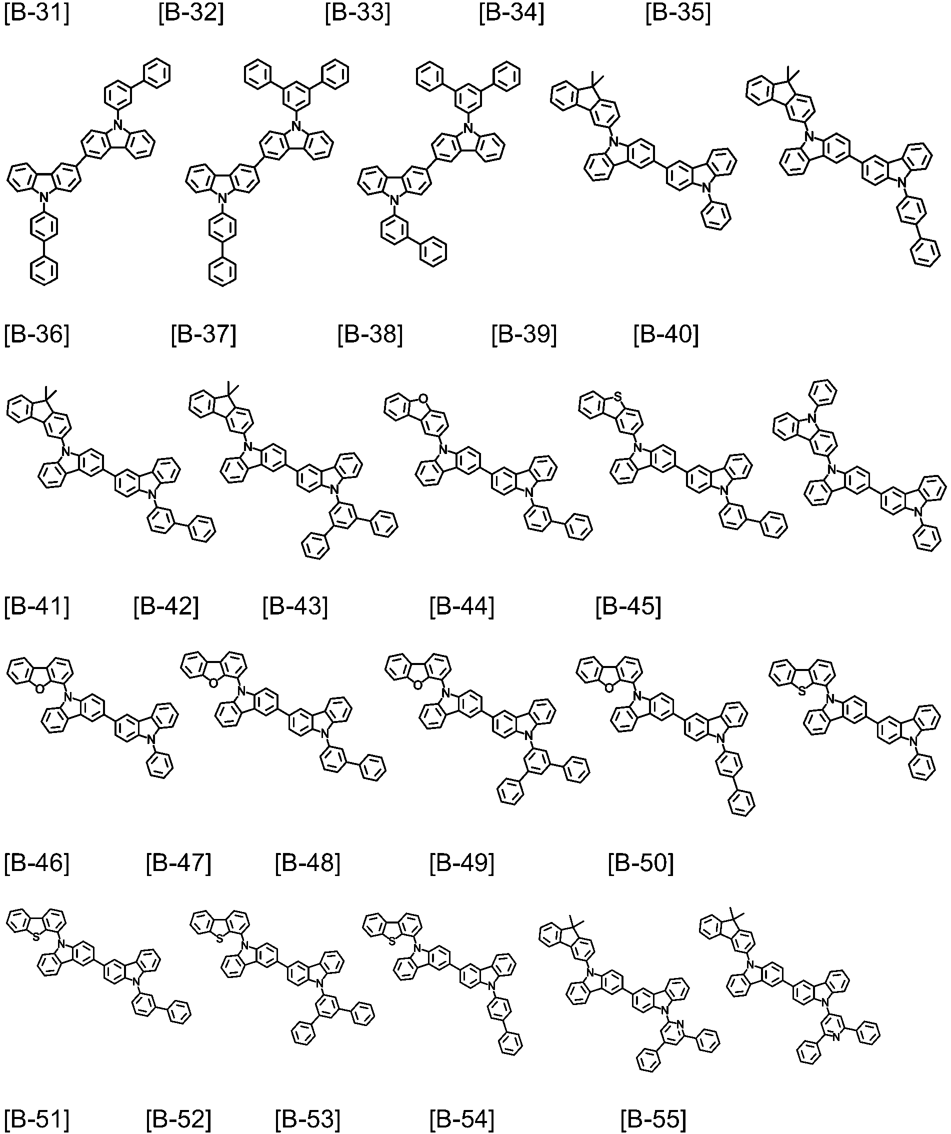

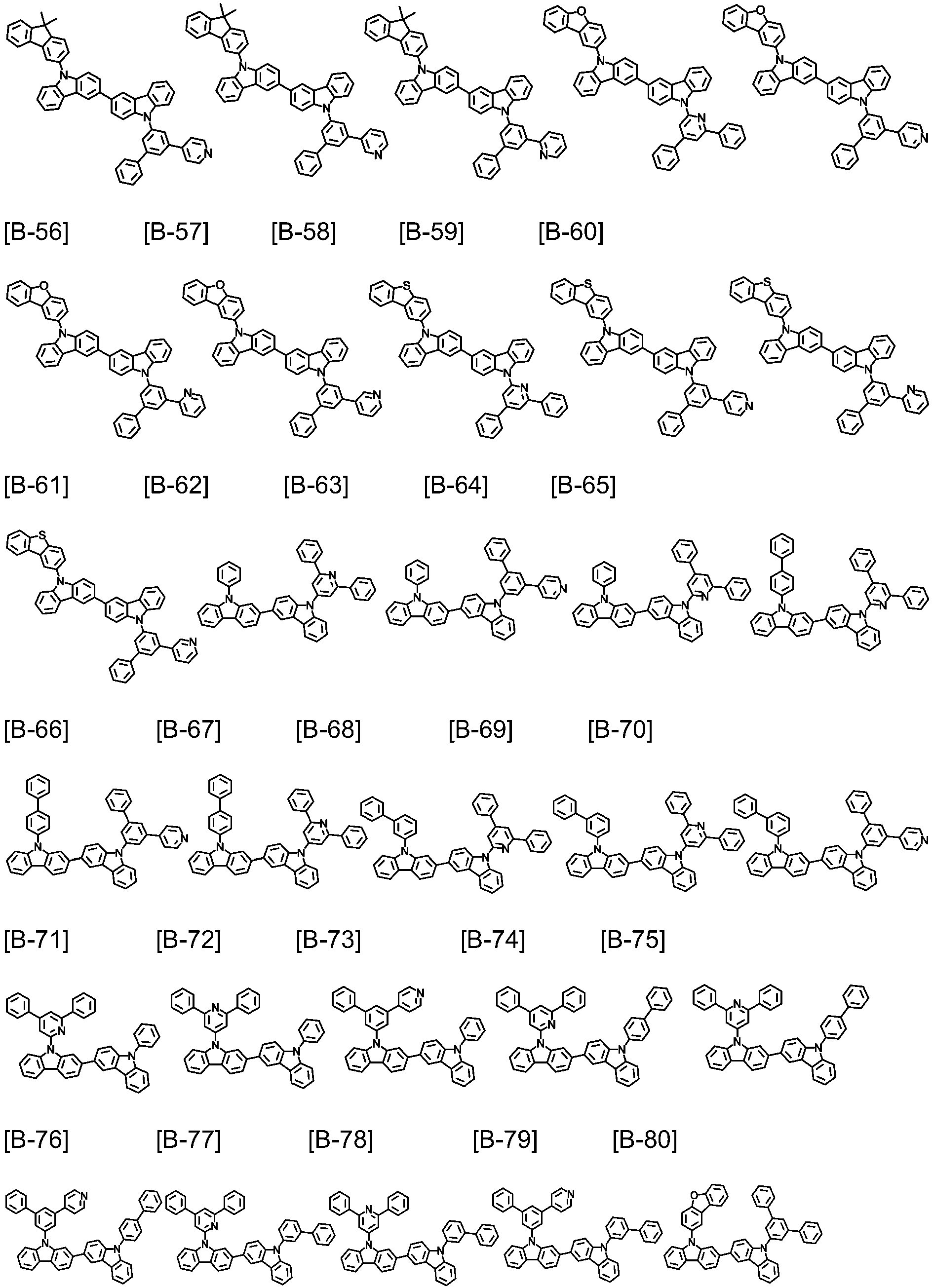

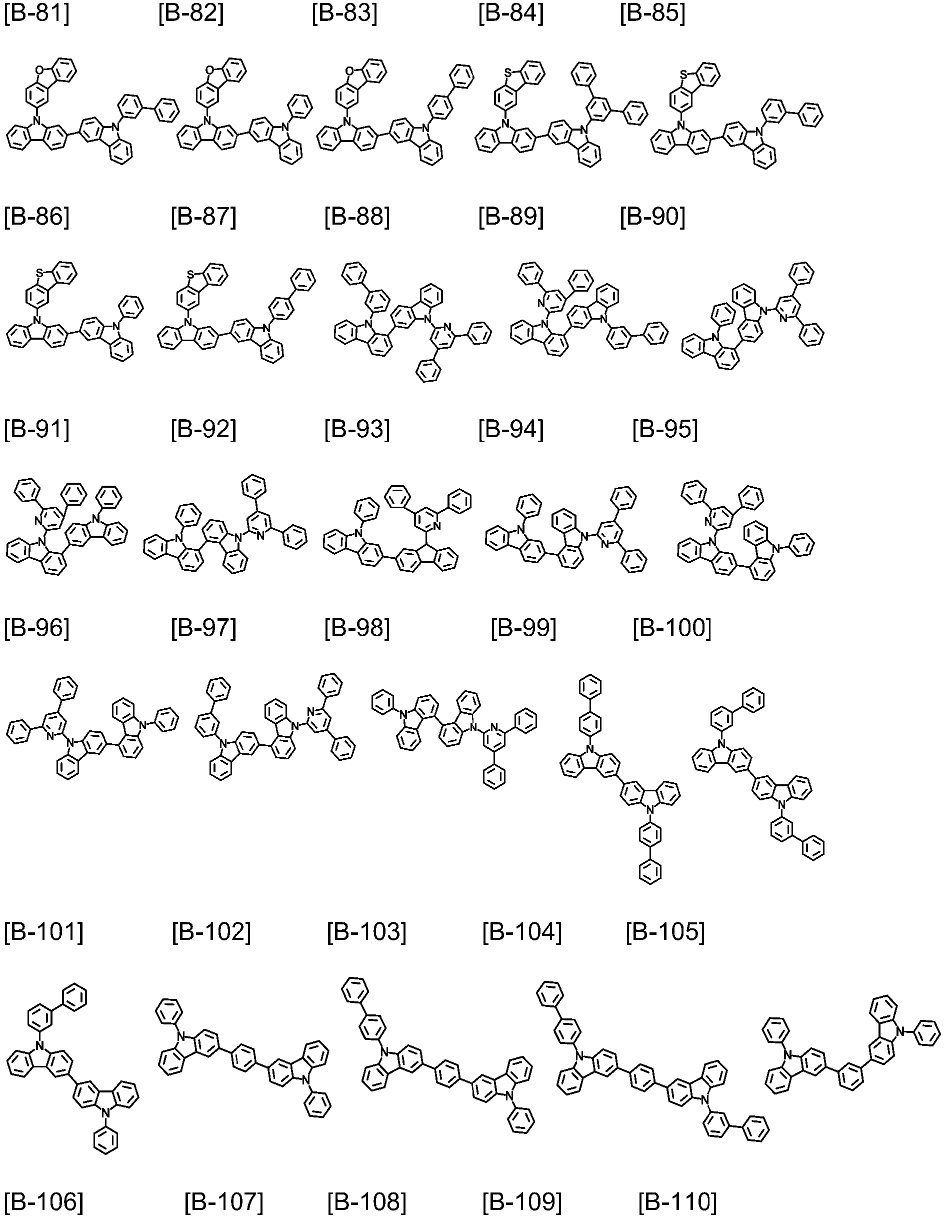

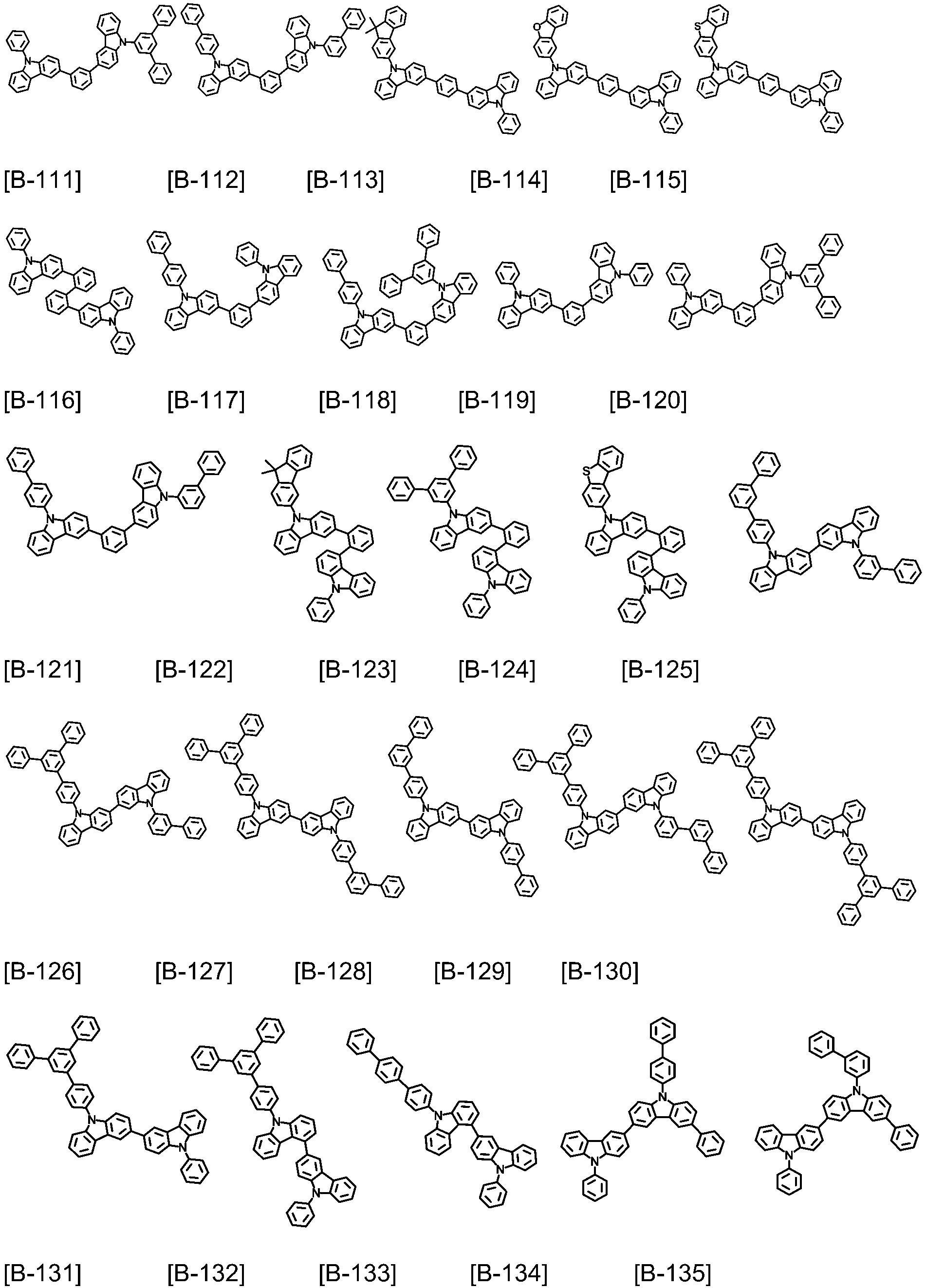

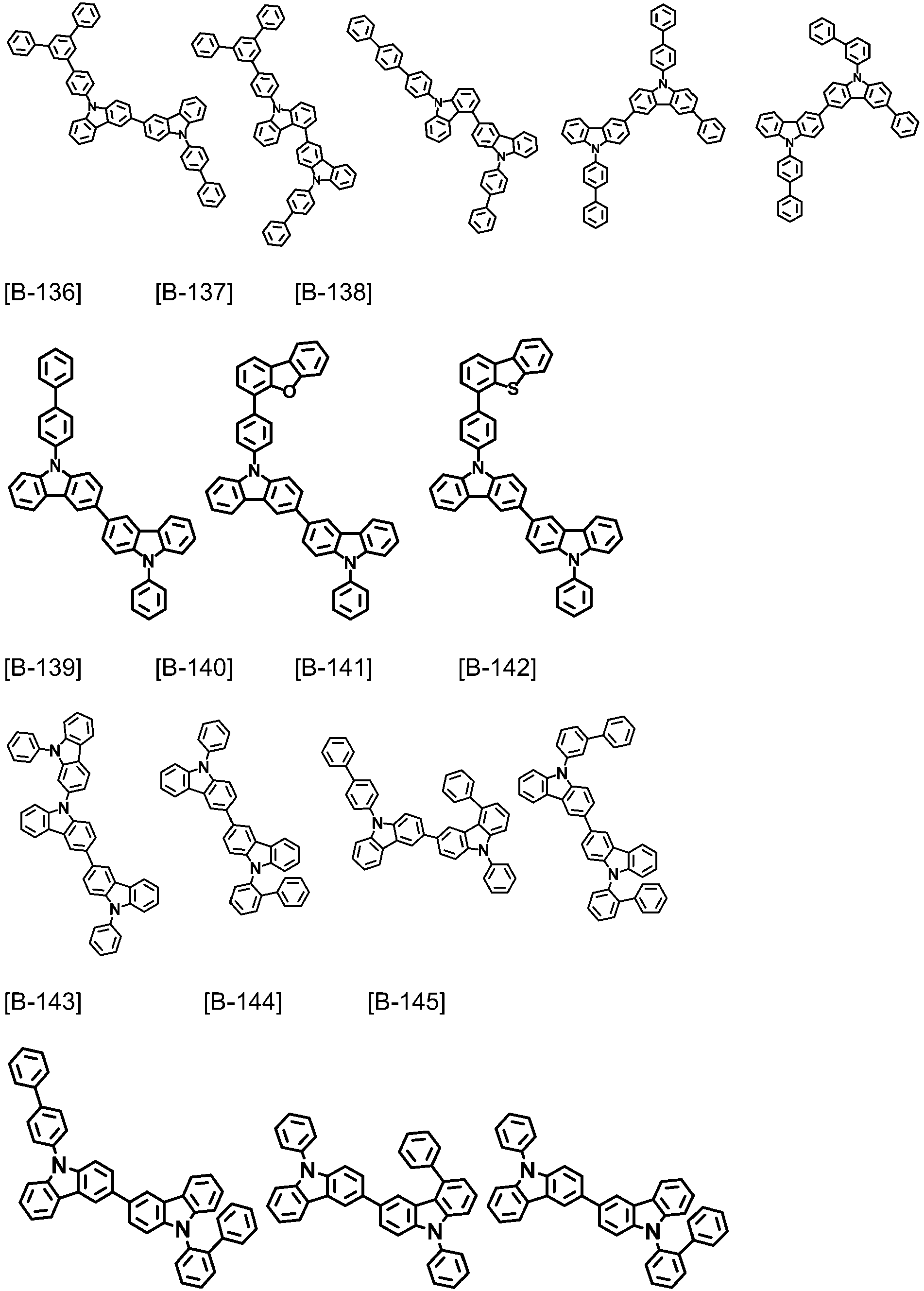

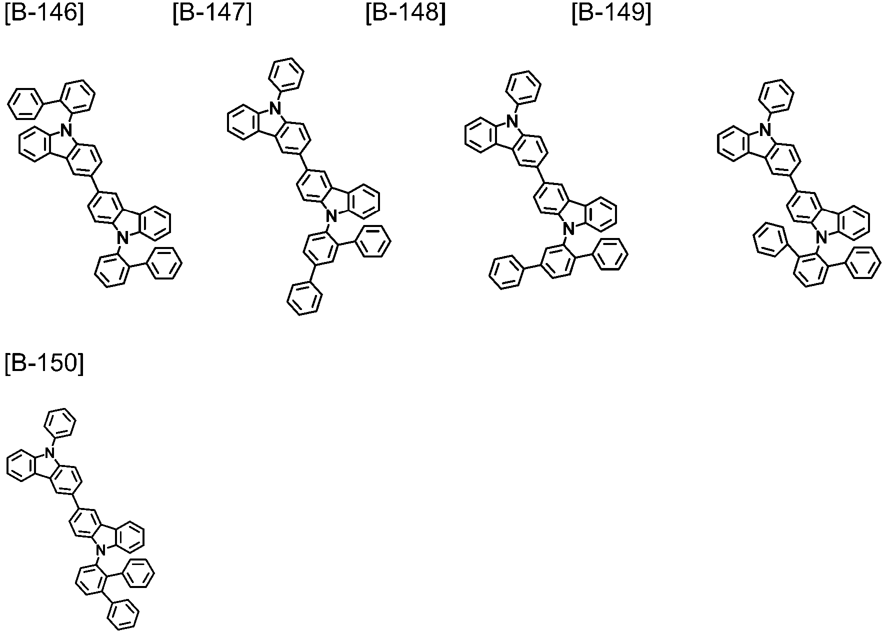

Additionally, examples of Compound B-1 to Compound B-150 listed in Group 2 in which at least one hydrogen is replaced with deuterium are shown below, but the present invention is not limited thereto.

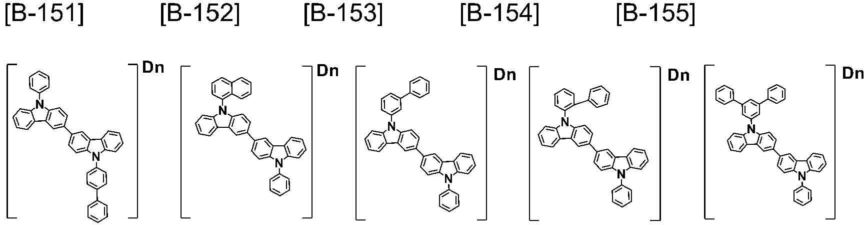

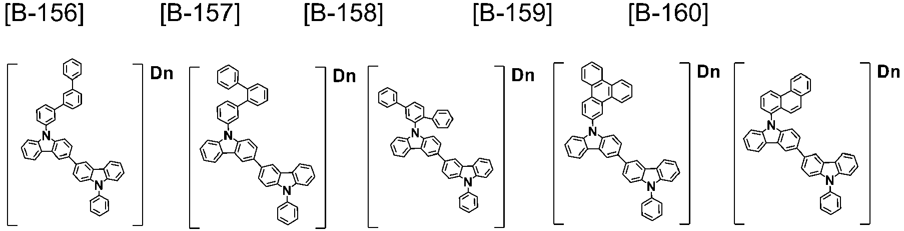

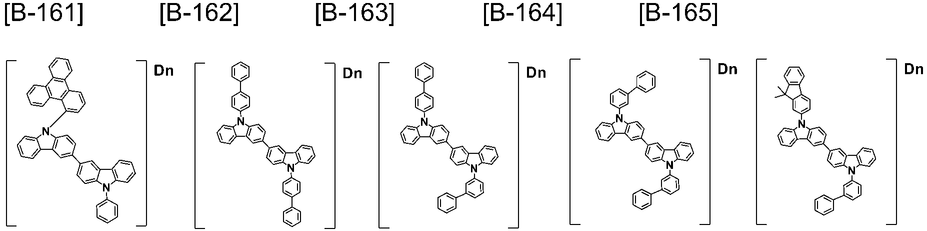

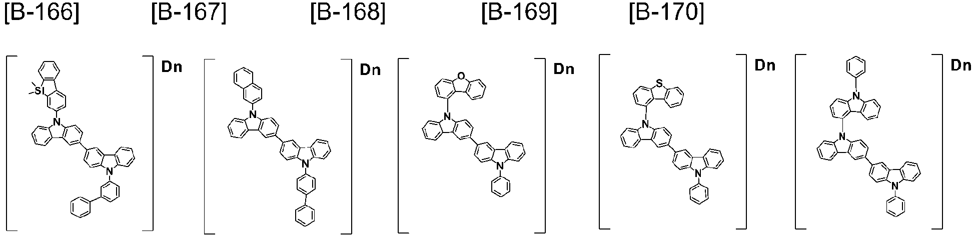

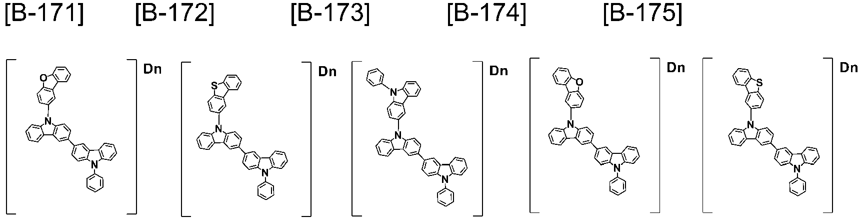

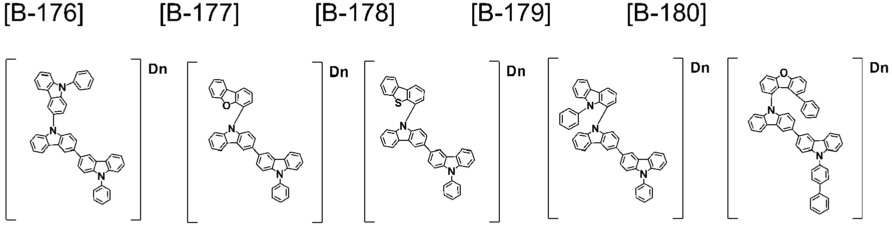

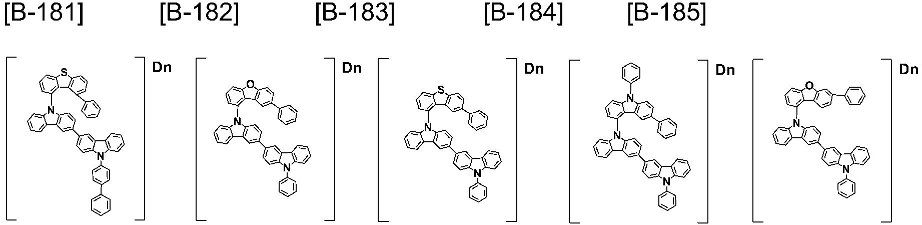

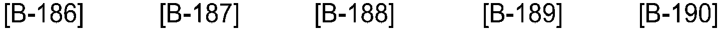

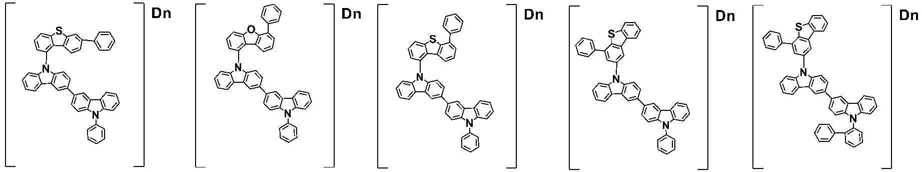

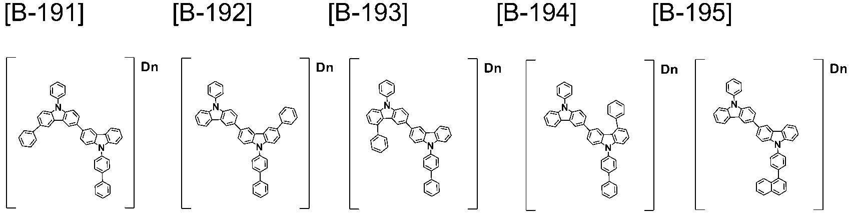

(Dn means the number of substituted deuteriums and indicates a structure in which one or more deuteriums are substituted)

The most specific structures for Compound B-151 to Compound B-195 of Group 2 are presented below as examples according to the position and substitution degree of deuterium substitution, which is not to limit the scope of rights to compounds not shown below.

The scope of the present invention is determined by the claims, and when deuterium is substituted, it is not limited to the compounds exemplified below, and the position of deuterium substitution and the substitution degree of deuterium substitution may include all changeable ranges within the range of Compound B-1 to Compound B-195.

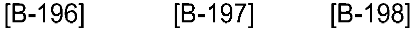

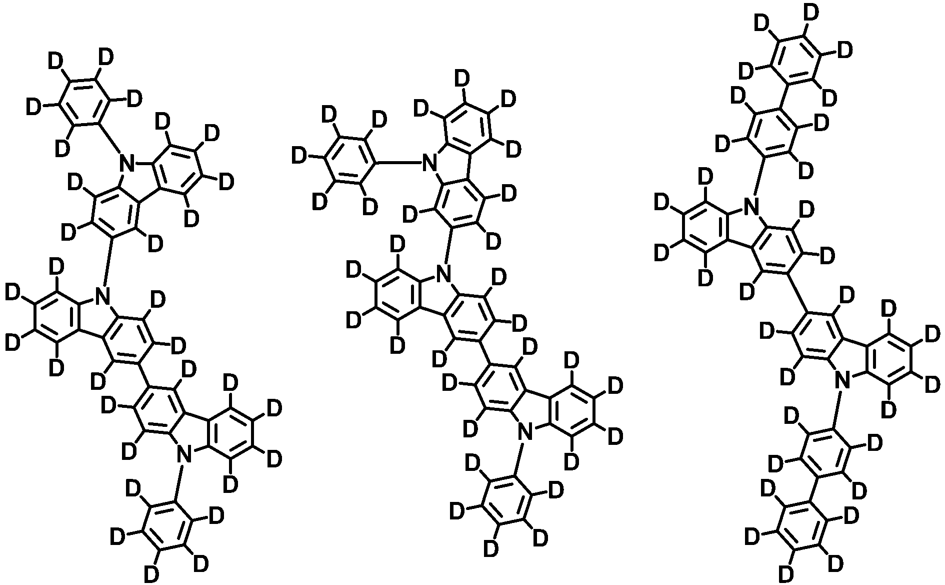

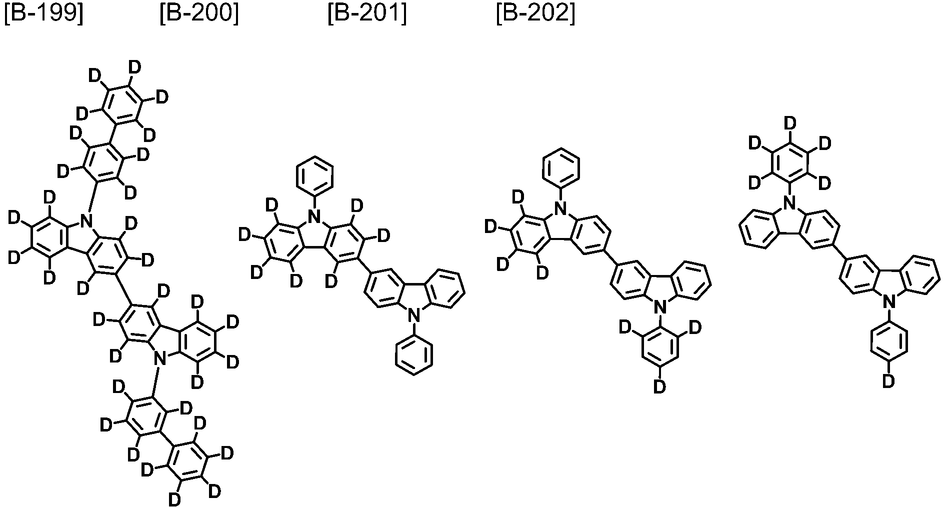

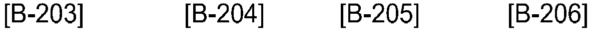

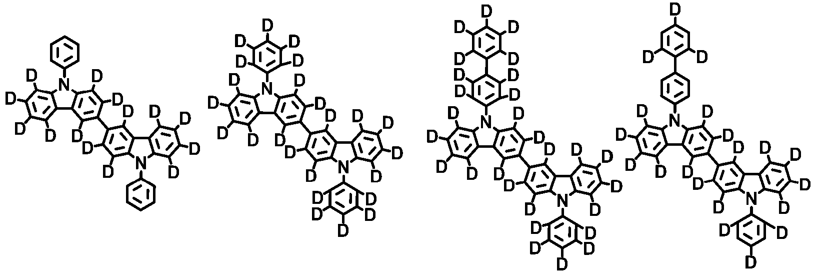

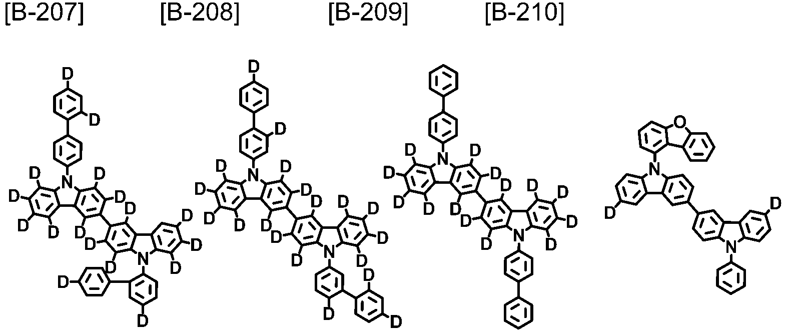

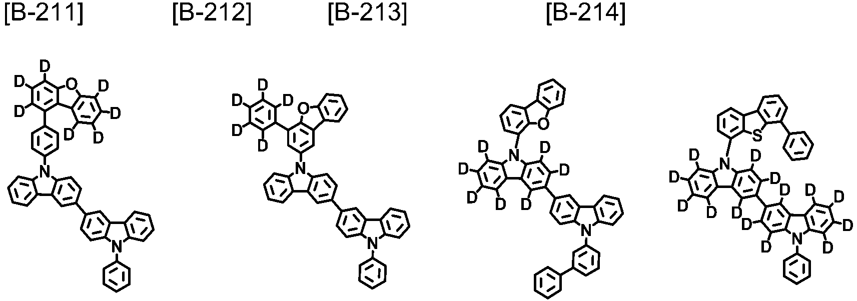

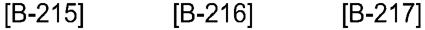

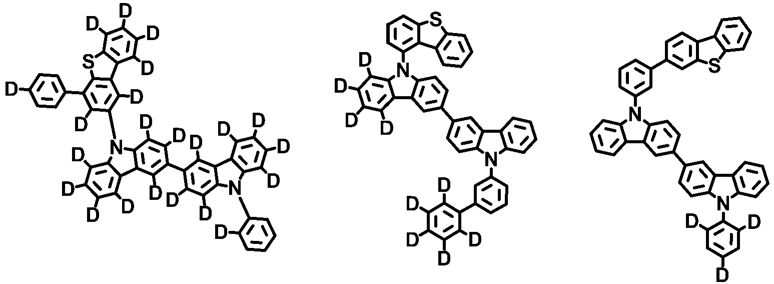

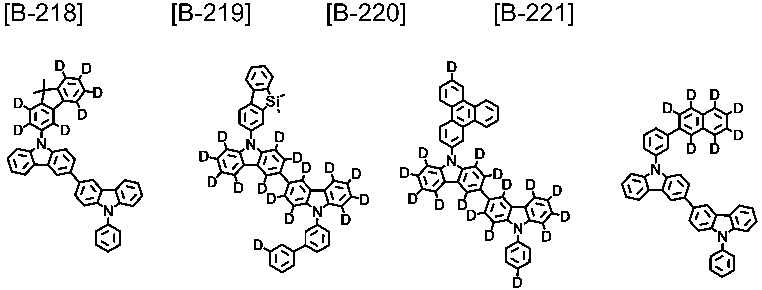

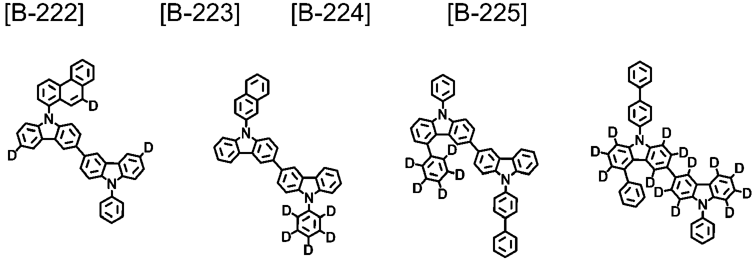

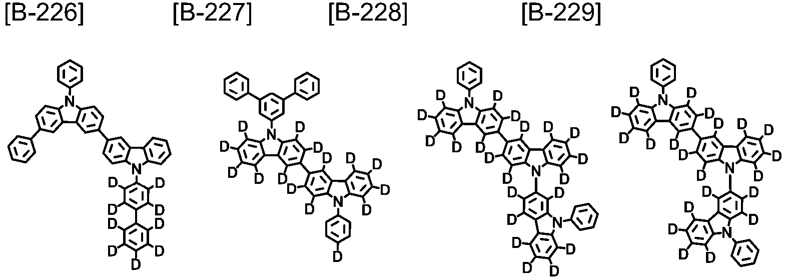

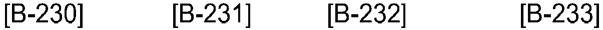

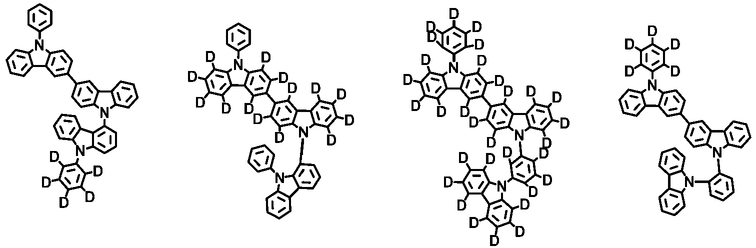

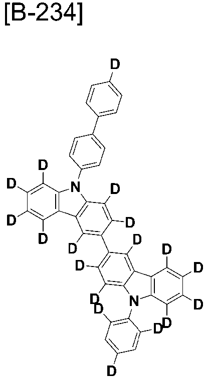

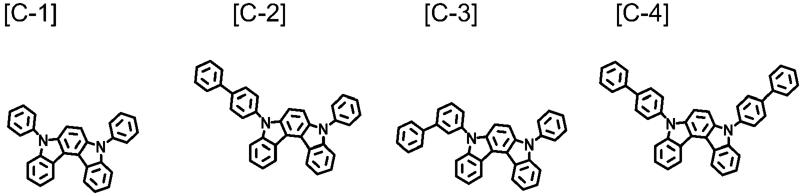

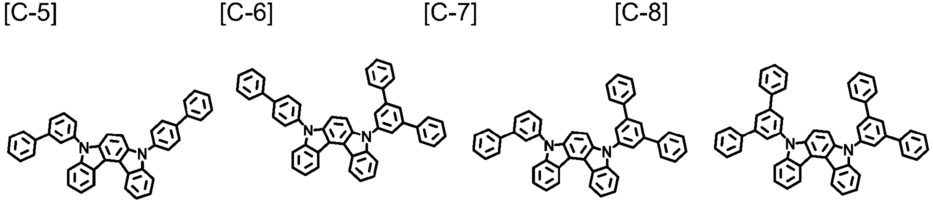

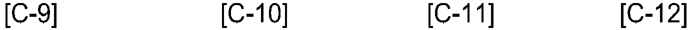

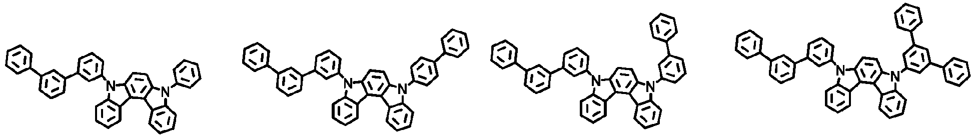

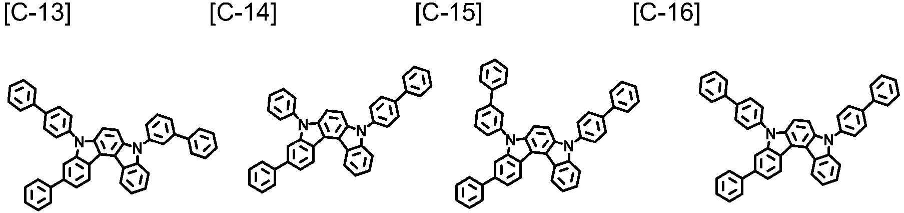

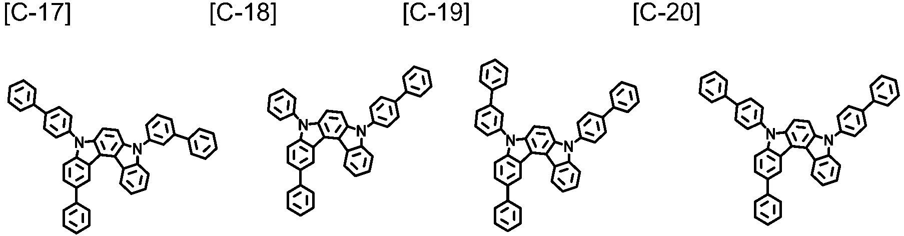

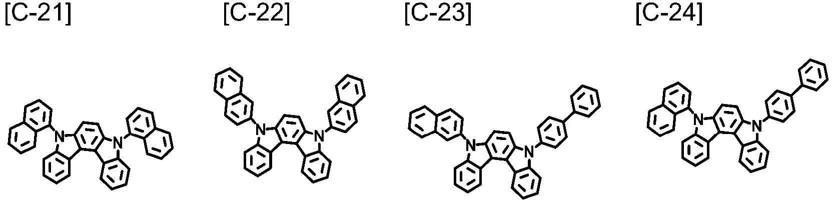

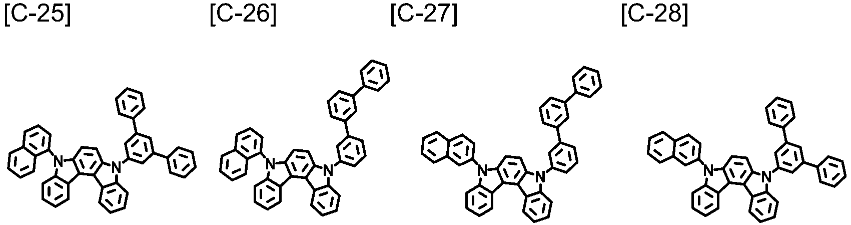

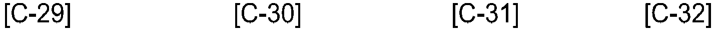

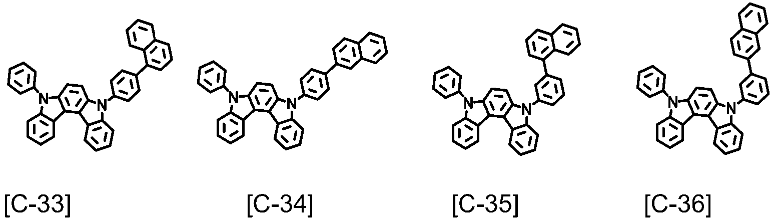

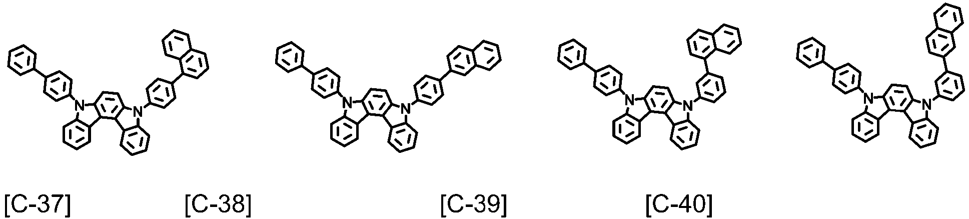

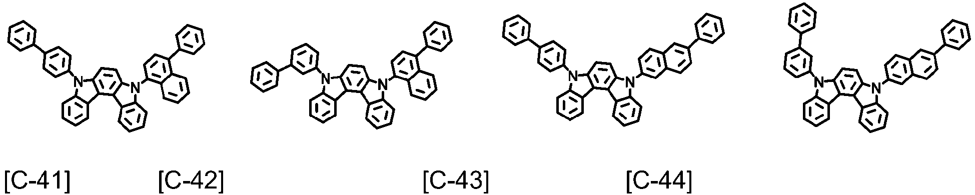

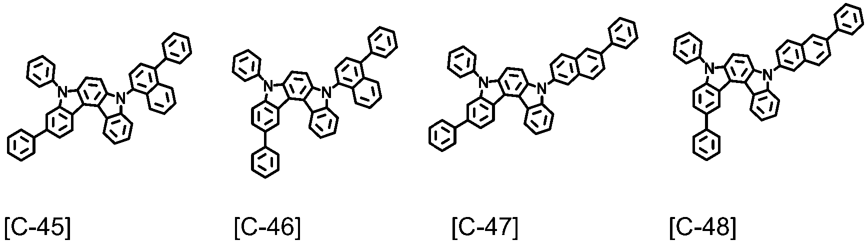

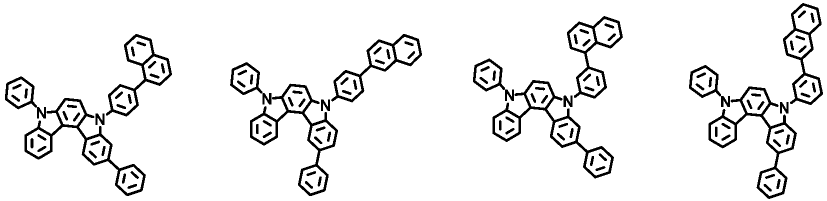

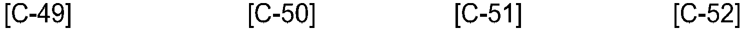

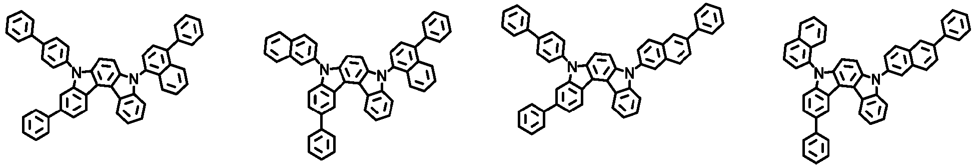

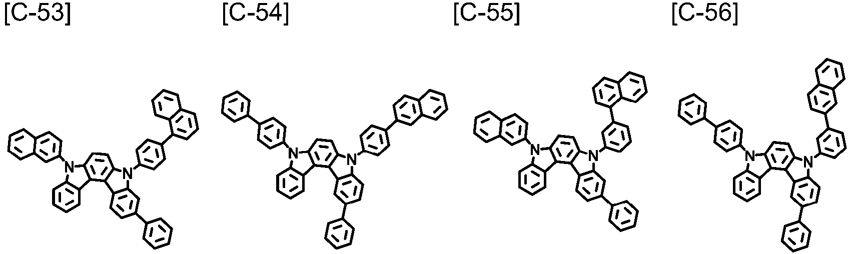

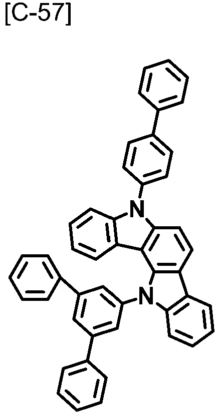

Additionally, examples of Compound C-1 to Compound C-57 listed in Group 2 in which at least one hydrogen is replaced with deuterium are shown below, but the present invention is not limited thereto.

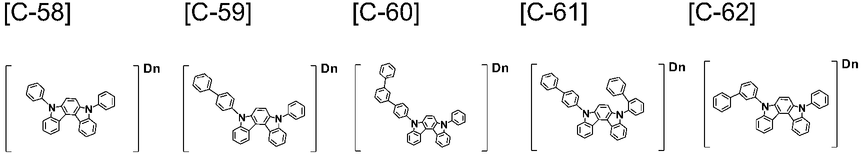

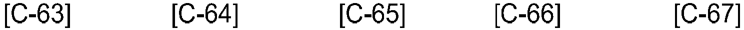

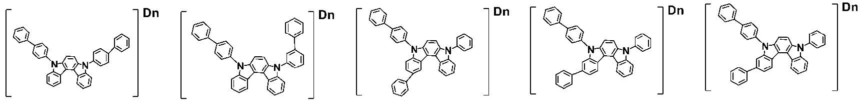

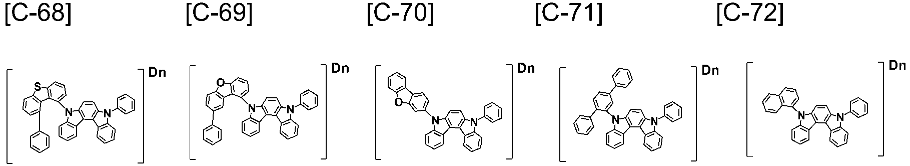

(Dn means the number of substituted deuteriums and indicates a structure in which one or more deuteriums are substituted)

For Compound C-58 to Compound C-72 of Group 2, the most specific structures are presented below only as examples according to the deuterium substitution position and substitution rate, and are not intended to limit the scope of rights to compounds not presented below.

The scope of the present invention is determined by the claims, and if substituted with deuterium, the present invention is not limited to the compounds exemplified below but may include all changeable ranges within the above range of Compound C-1 to Compound C-72 according to a deuterium substitution position and deuterium substitution rate, and the like.

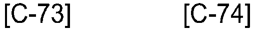

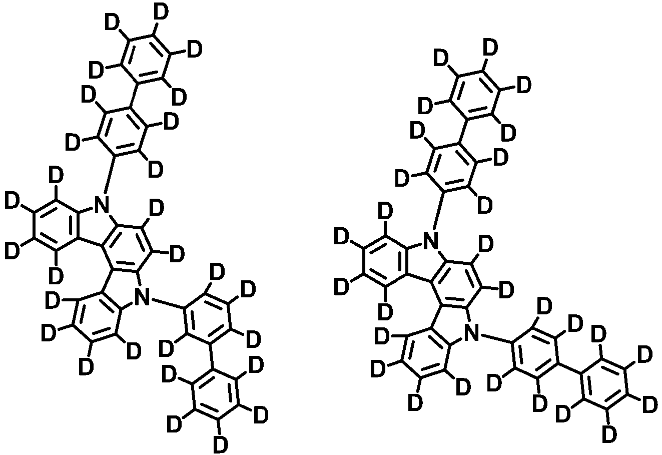

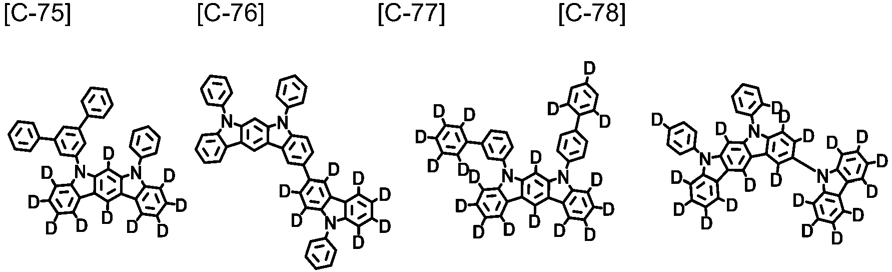

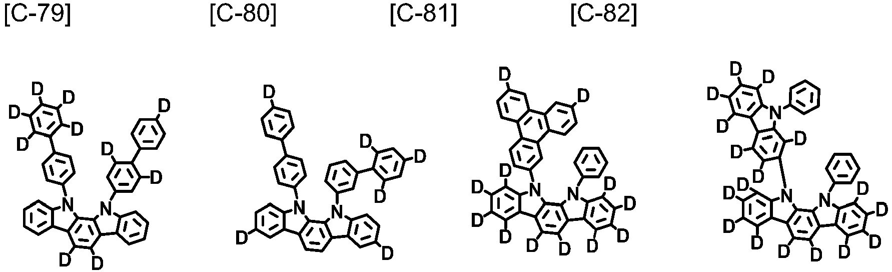

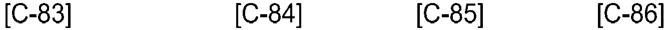

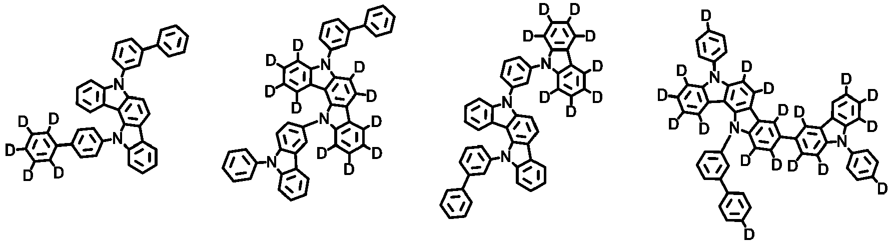

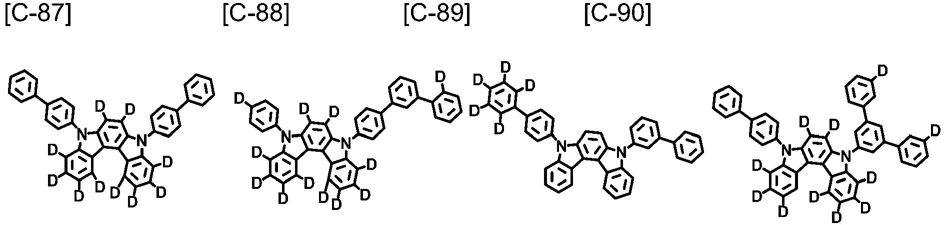

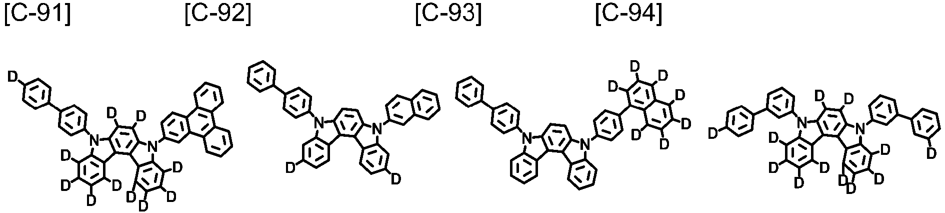

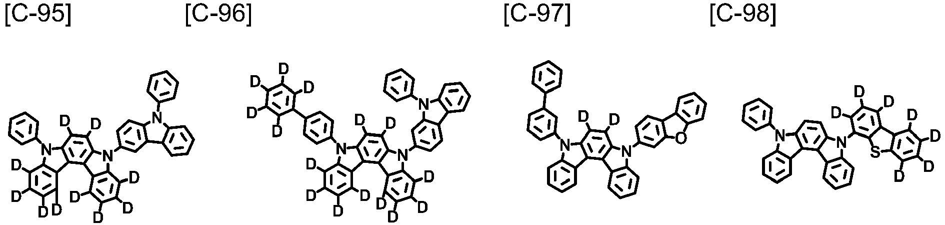

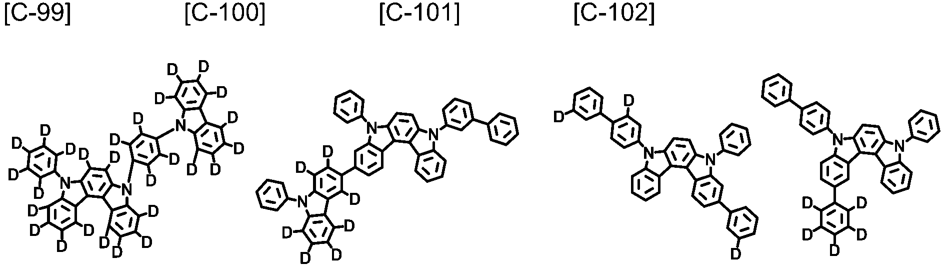

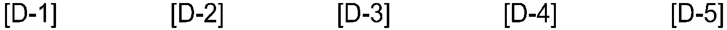

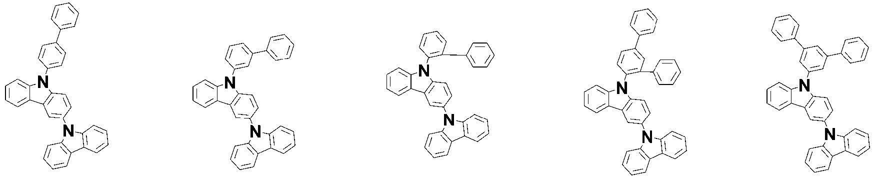

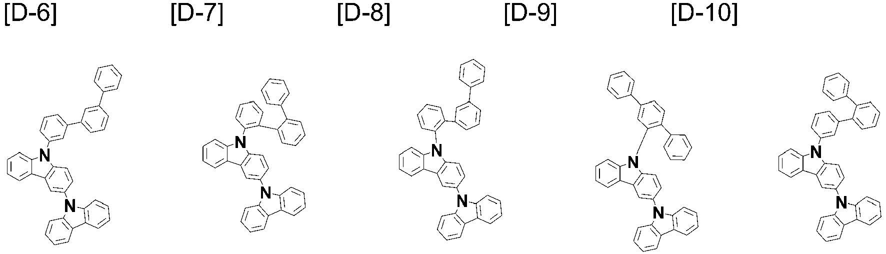

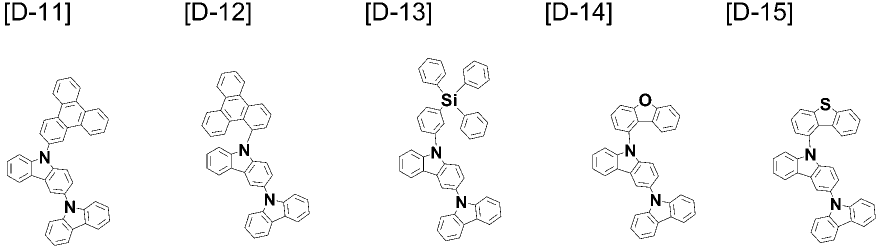

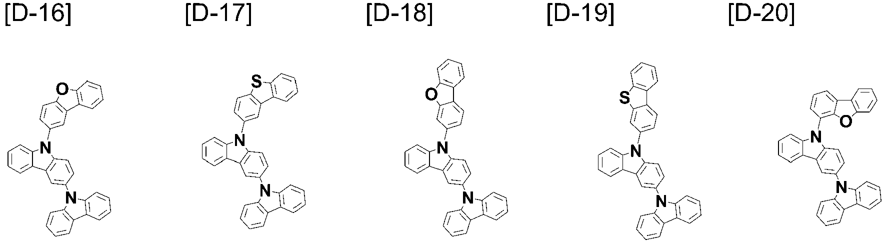

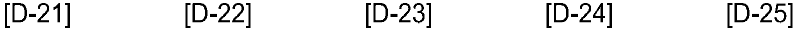

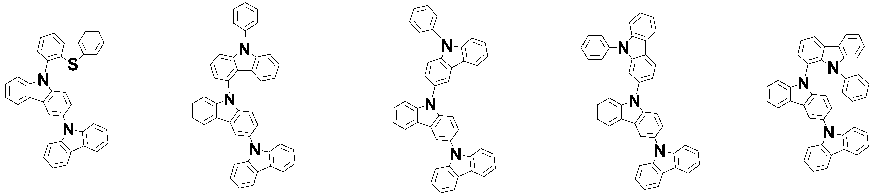

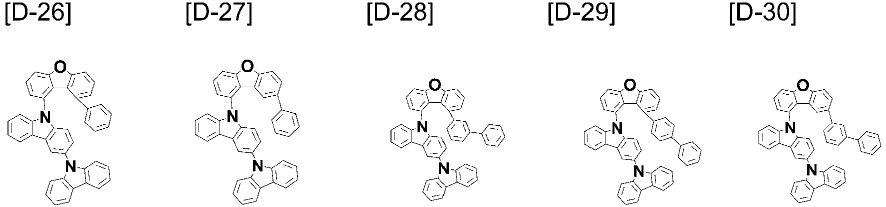

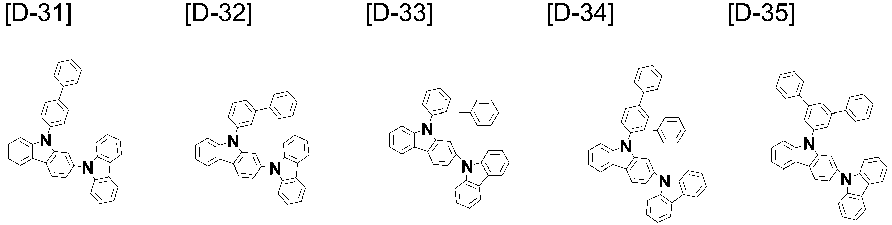

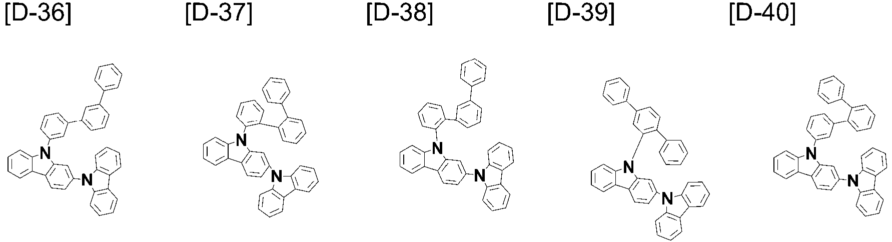

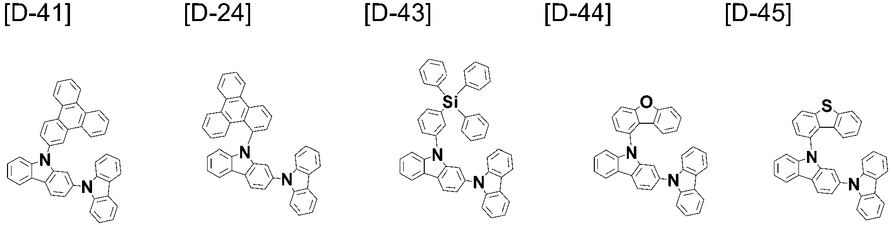

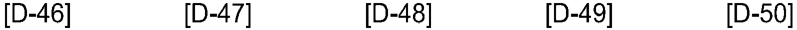

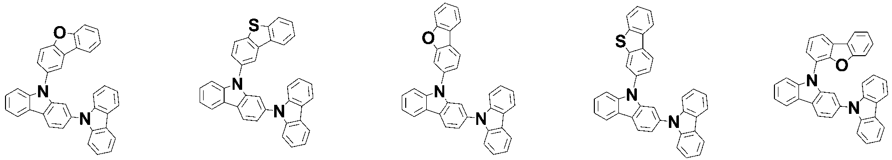

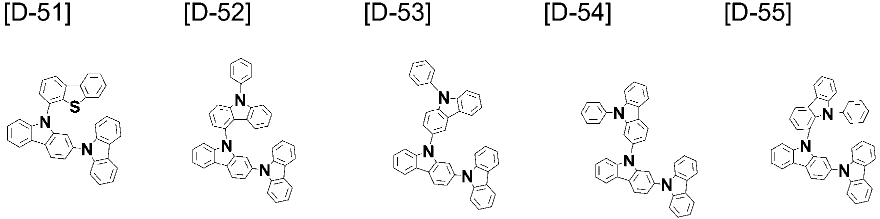

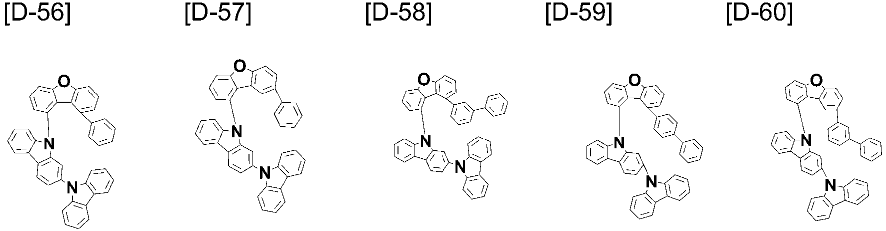

Additionally, examples of Compound D-1 to Compound D-60 listed in Group 2 in which at least one hydrogen is replaced with deuterium are shown below, but the present invention is not limited thereto.

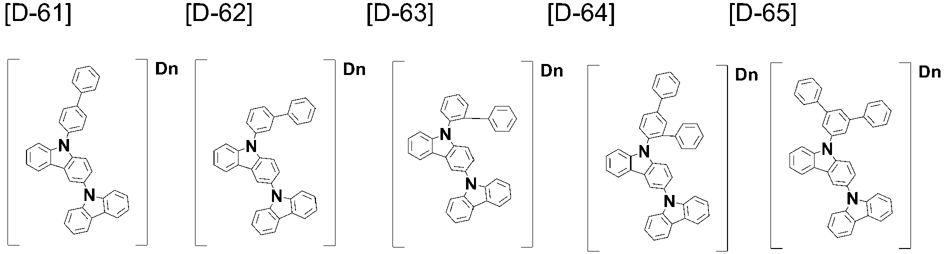

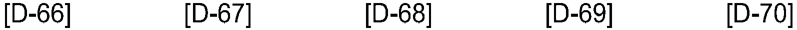

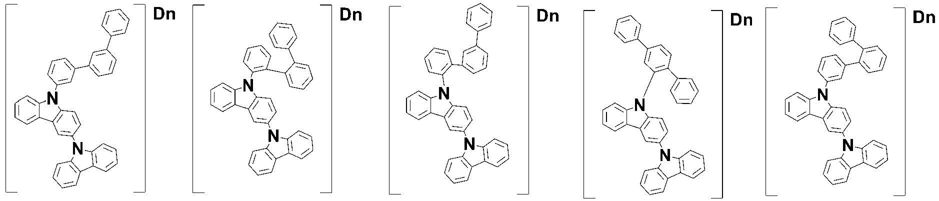

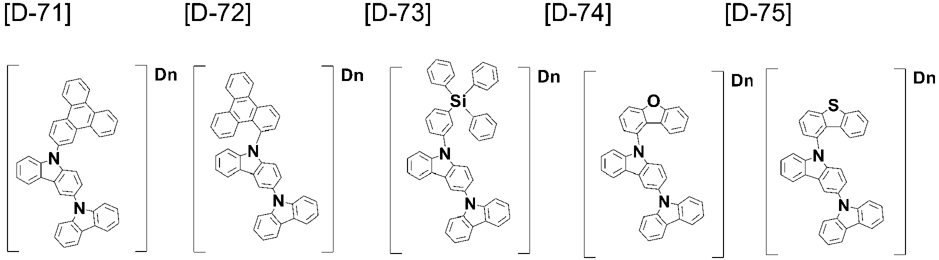

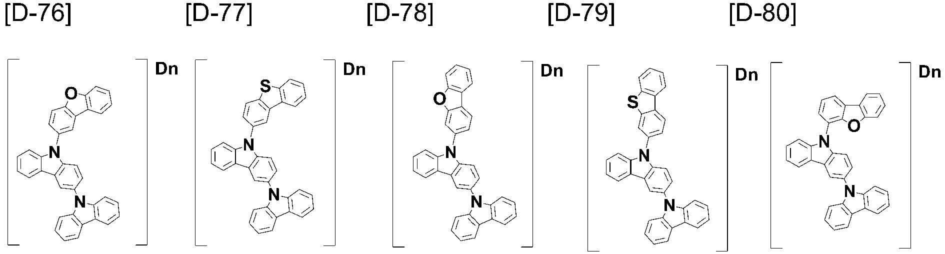

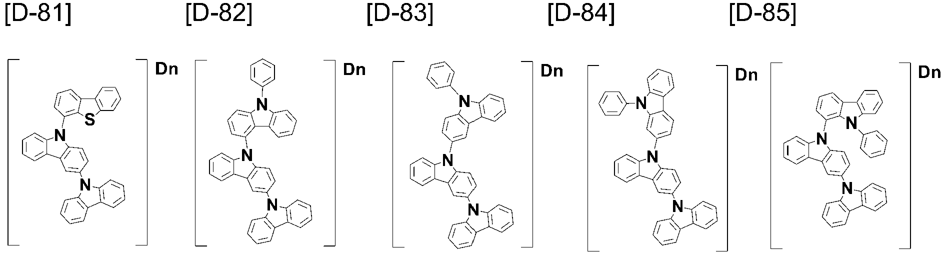

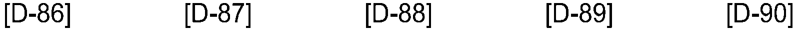

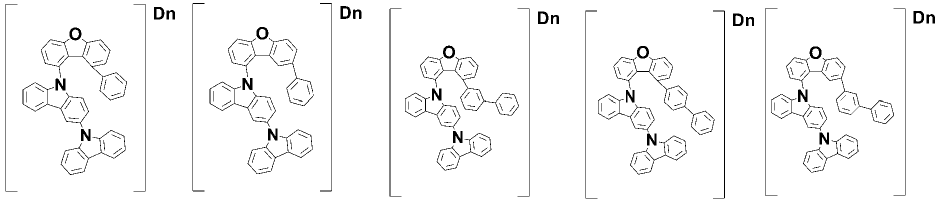

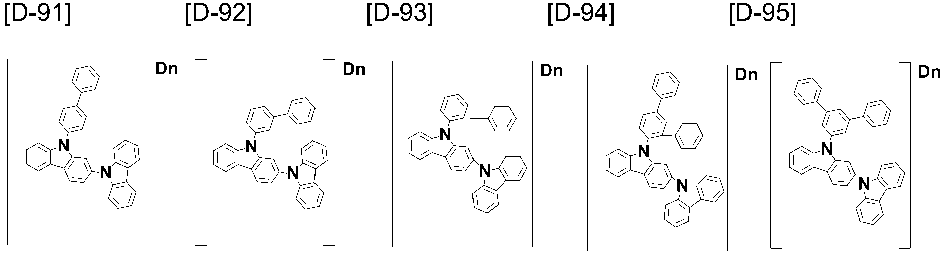

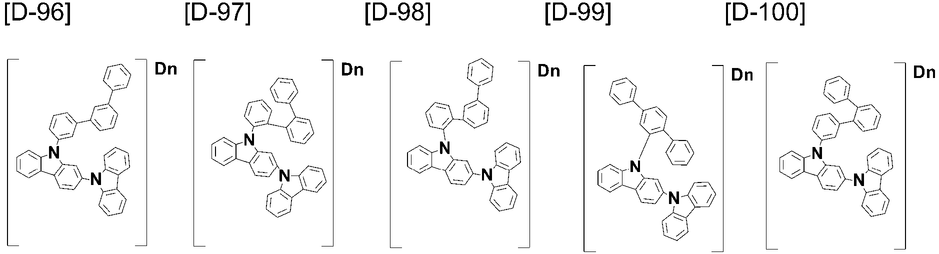

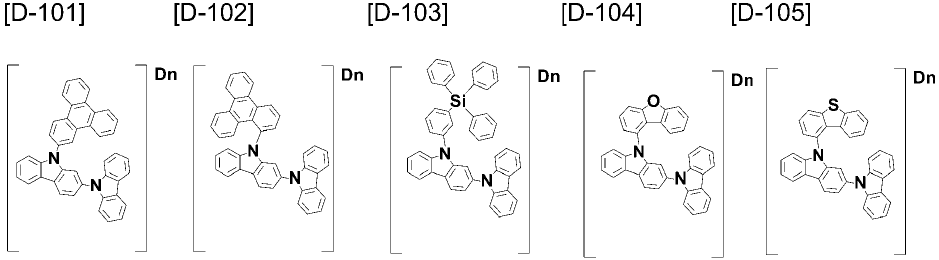

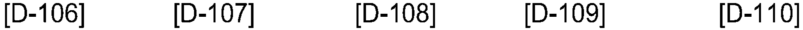

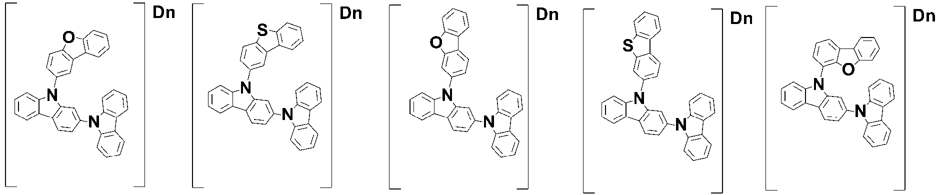

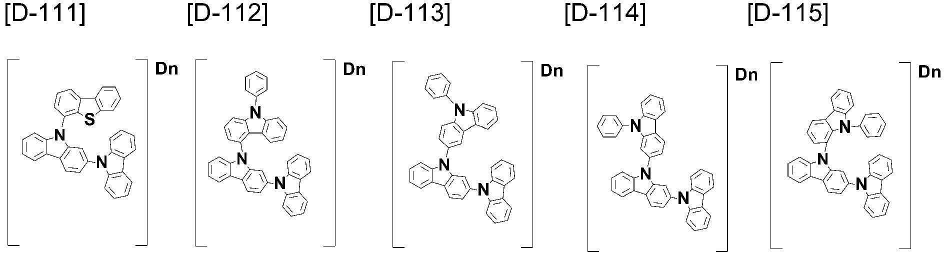

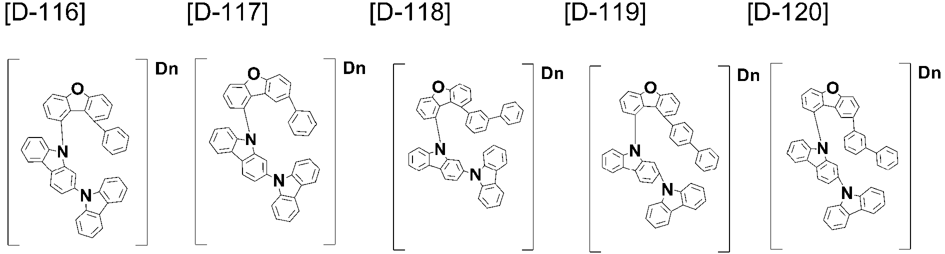

(Dn means the number of substituted deuteriums and indicates a structure in which one or more deuteriums are substituted)

**In** a most specific embodiment, the first compound may be represented by Chemical Formula 1A-3 or Chemical Formula 1B-3, and the second compound may be represented by Chemical Formula 2-8.

The first compound and the second compound may be included in a weight ratio of, for example, 1:99 to 99:1. Within the above range, bipolar characteristics may be realized by adjusting an appropriate weight ratio using electron transport capability of the first compound and hole transport capability of the second compound, thereby improving efficiency and life-span. Within the above range, they may be included in a weight ratio of, for example, about 10:90 to 90:10, about 20:80 to 80:20, and for example, they may be included in a weight ratio of, for example, about 20:80 to about 70:30, about 20:80 to about 60:40, and about 30:70 to about 60:40. As a specific example, they may be included in a weight ratio of 40:60, 50:50, or 60:40.

Hereinafter, an organic optoelectronic device to which the compound for the organic optoelectronic device or the composition for the organic optoelectronic device described above is applied is described.

The organic optoelectronic device may be a suitable device to convert electrical energy into photoenergy and vice versa, e.g., an organic photoelectric device, an organic light emitting diode, an organic solar cell, or an organic photoconductor drum.

Herein, an organic light emitting diode as one example of an organic optoelectronic device is described referring to drawings.

FIG. 1 is a cross-sectional view showing an organic light emitting diode according to an embodiment.

Referring to FIG. 1, an organic light emitting diode 100 according to an embodiment includes an anode 120 and a cathode 110 facing each other and an organic layer 105 between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a low work function to facilitate electron injection, for example, and may be made of, for example, a metal, a metal oxide, and/or a conductive polymer. Examples of the cathode 110 include for example, a metal such as magnesium, calcium, sodium, potassium, titanium, indium, yttrium, lithium, gadolinium, aluminum, silver, tin, lead, cesium, and barium or an alloy thereof; and a multi-layered structure material such as LiF/Al, LiO₂/Al, LiF/Ca, LiF/Al, and BaF₂/Ca, but is not limited thereto.

The organic layer 105 may include the aforementioned compound for the organic optoelectronic device or composition for the organic optoelectronic device.

The organic layer 105 includes a light emitting layer 130, the light emitting layer 130 includes a host and a dopant, the host may include the aforementioned compound for the organic optoelectronic device or composition for the organic optoelectronic device, and the dopant may be, for example, a phosphorescent dopant, for example, a red, green, or blue phosphorescent dopant, and for example, a red or green phosphorescent dopant.

The dopant is a material mixed with the compound or composition for an organic optoelectronic device in a small amount to cause light emission, and may be generally a material such as a metal complex that emits light by multiple excitation into a triplet or more. The dopant may be, for example an inorganic, organic, or organic/inorganic compound, and one or more types thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z, but is not limited thereto.

[Chemical Formula Z] L⁶MX¹

In Chemical Formula Z, M is a metal, and L⁶ and X¹ are the same as or different and are ligands to form a complex compound with M.

The M may be for example Ir, Pt, Os, Ti, Zr, Hf, Eu, Tb, Tm, Fe, Co, Ni, Ru, Rh, Pd, or a combination thereof, and L⁶ and X¹ may be for example a bidentate ligand.

The ligands represented by L⁶ and X¹ may be selected from chemical formulas listed in Group A, but are not limited thereto.

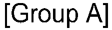

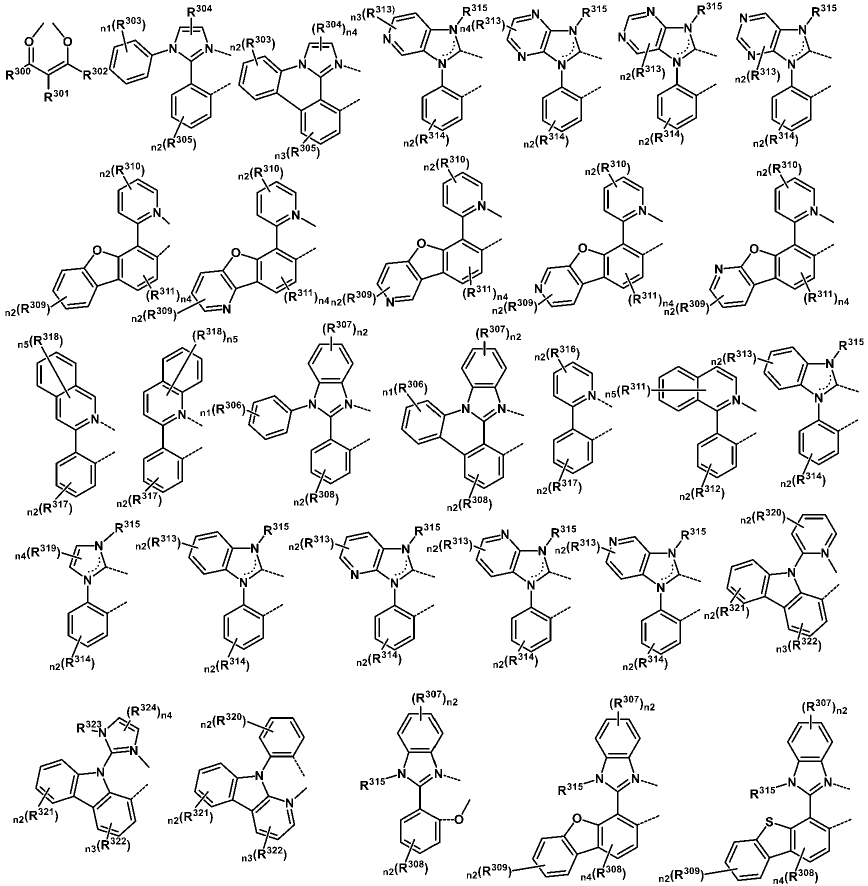

In Group A,
R³⁰⁰ to R¹⁰² are each independently hydrogen, deuterium, a C1 to C30 alkyl group that is substituted or unsubstituted with a halogen, a C6 to C30 aryl group that is substituted or unsubstituted with a C1 to C30 alkyl, or a halogen, and
are each independently hydrogen, deuterium, a halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SF₅, a trialkylsilyl group having a substituted or unsubstituted C1 to C30 alkyl group, a dialkylarylsilyl group having a substituted or unsubstituted C1 to C30 alkyl group and a C6 to C30 aryl group, or a triarylsilyl group having a substituted or unsubstituted C6 to C30 aryl group.

In an embodiment, the dopant may be an iridium complex, and may be represented by Chemical Formula 6-1 or Chemical Formula 6-2.

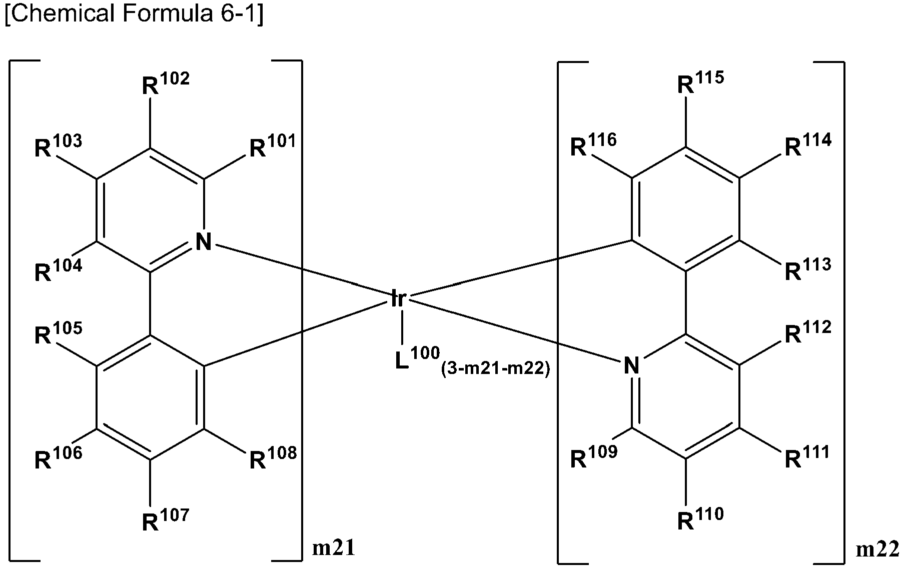

In Chemical Formula 6-1,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a substituted or unsubstituted C1 to C6 alkyl group,
at least one of R¹⁰¹ to R¹¹⁶ is a functional group represented by Chemical Formula V-1,
L¹⁰⁰ is a bidentate ligand of a monovalent anion, and is a ligand that coordinates to iridium through a lone pair of carbons or heteroatoms,
m21 and m22 are each independently any one of integers from 0 to 3, and m21 + m22 is any one of integers from 1 to 3, 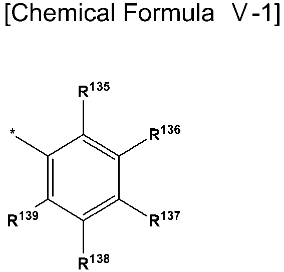
wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴, and
* refers to a portion linked to a carbon atom. 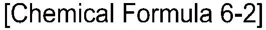 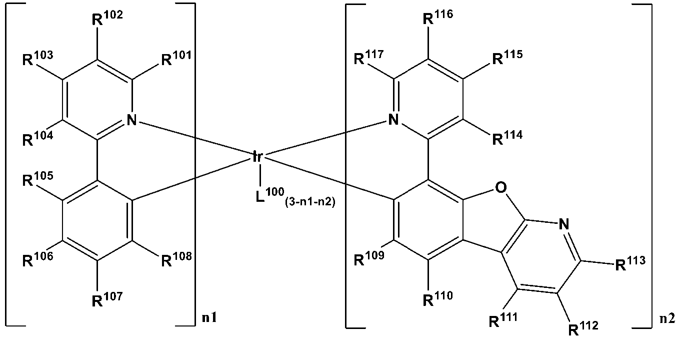 In Chemical Formula 6-2,
   R¹⁰¹ to R¹¹⁷ are each independently hydrogen, deuterium, a substituted or unsubstituted C1 to C10 alkyl group, a substituted or unsubstituted C6 to C20 aryl group, or -Si R¹³³R¹³⁴R¹³⁵,
   R¹³³ to R¹³⁵ are each independently a substituted or unsubstituted C1 to C6 alkyl group,
   L¹⁰⁰ is a bidentate ligand of a monovalent anion, and is a ligand that coordinates to iridium through a lone pair of carbons or heteroatoms,
   n1 and n2 are each independently any integer from 0 to 3, and n1 + n2 is any one of integers from 1 to 3.

In another embodiment, the dopant may be a platinum complex, for example represented by Chemical Formula Z-1.

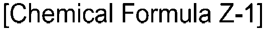

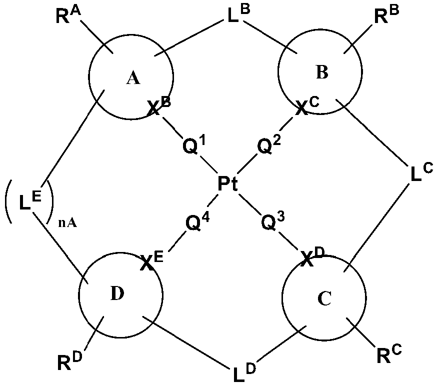

In Chemical Formula Z-1, rings A, B, C, and D independently represent a 5-membered or 6-membered carbocyclic or heterocyclic ring;
R^{A}, R^{B}, R^{C}, and R^{D} independently represent mono-, di-, tri-, or tetra-substitution, or unsubstitution;
L^{B}, L^{C}, and L^{D} are each independent selected from a direct bond, BR, NR, PR, O, S, Se, C=O, S=O, SO₂, CRR', SiRR', GeRR', and a combination thereof;
when nA is 1, L^{E} is selected from a direct bond, BR, NR, PR, O, S, Se, C=O, S=O, SO₂, CRR', SiRR', GeRR', and a combination thereof; when nA is 0, L^{E} does not exist; and
R^{A}, R^{B}, R^{C}, R^{D}, R, and R' are each independently selected from hydrogen, deuterium, a halogen, alkyl group, a cycloalkyl group, a heteroalkyl group, arylalkyl group, an alkoxy group, aryloxy group, an amino group, a silyl group, an alkenyl group, a cycloalkenyl group, a heteroalkenyl group, an alkynyl group, aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, and a combination thereof; any adjacent R^{A}, R^{B}, R^{C}, R^{D}, R, and R' are optionally linked to each other to provide a ring; X^{B}, X^{C}, X^{D}, and X^{E} are each independently selected from carbon and nitrogen; and Q¹, Q², Q³, and Q⁴ each represent oxygen or a direct bond.

The platinum complex may be represented, for example, by Chemical Formula 7-1 or Chemical Formula 7-2.

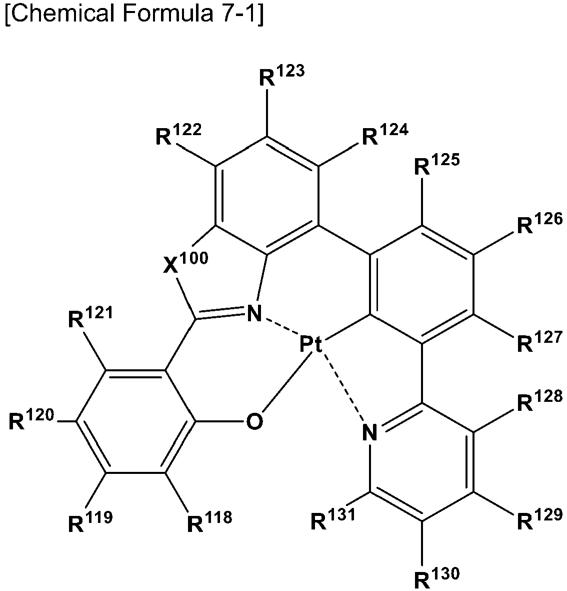

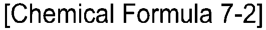

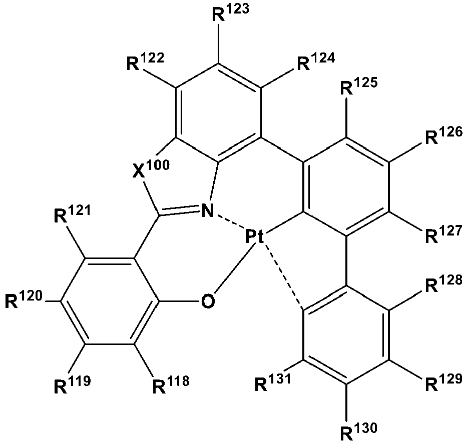

In Chemical Formula 7-1 and Chemical Formula 7-2,
X¹⁰⁰ is selected from O, S, and NR¹³²,
R¹¹⁸ to R¹³² are each independently hydrogen, deuterium, a substituted or unsubstituted C1 to C10 alkyl group, a substituted or unsubstituted C6 to C20 aryl group, or - SiR¹³³R¹³⁴R¹³⁵,
R¹³³ to R¹³⁵ are each independently a substituted or unsubstituted C1 to C6 alkyl group,
at least one of R¹¹⁸ to R¹³² is -SiR¹³³R¹³⁴R¹³⁵ or a tert-butyl group, and
R¹³³ to R¹³⁵ are each independently a substituted or unsubstituted C1 to C6 alkyl group.

The organic layer may further include a charge transport region in addition to the light emitting layer.

The charge transport region may be, for example, a hole transport region 140.

The hole transport region 140 may further increase hole injection and/or hole mobility between the anode 120 and the light emitting layer 130 and block electrons.

Specifically, the hole transport region 140 may include a hole transport layer between the anode 120 and the light emitting layer 130, and a hole transport auxiliary layer between the light emitting layer 130 and the hole transport layer, and at least one of compounds listed in Group B below may be included in at least one layer of the hole transport layer and the hole transport auxiliary layer.

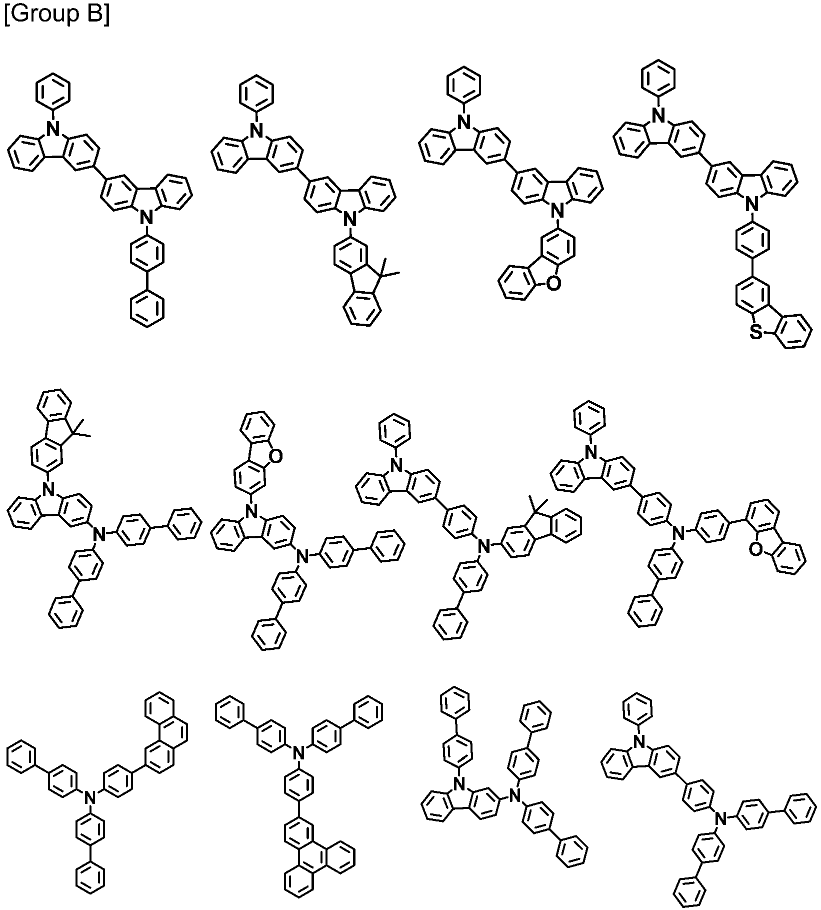

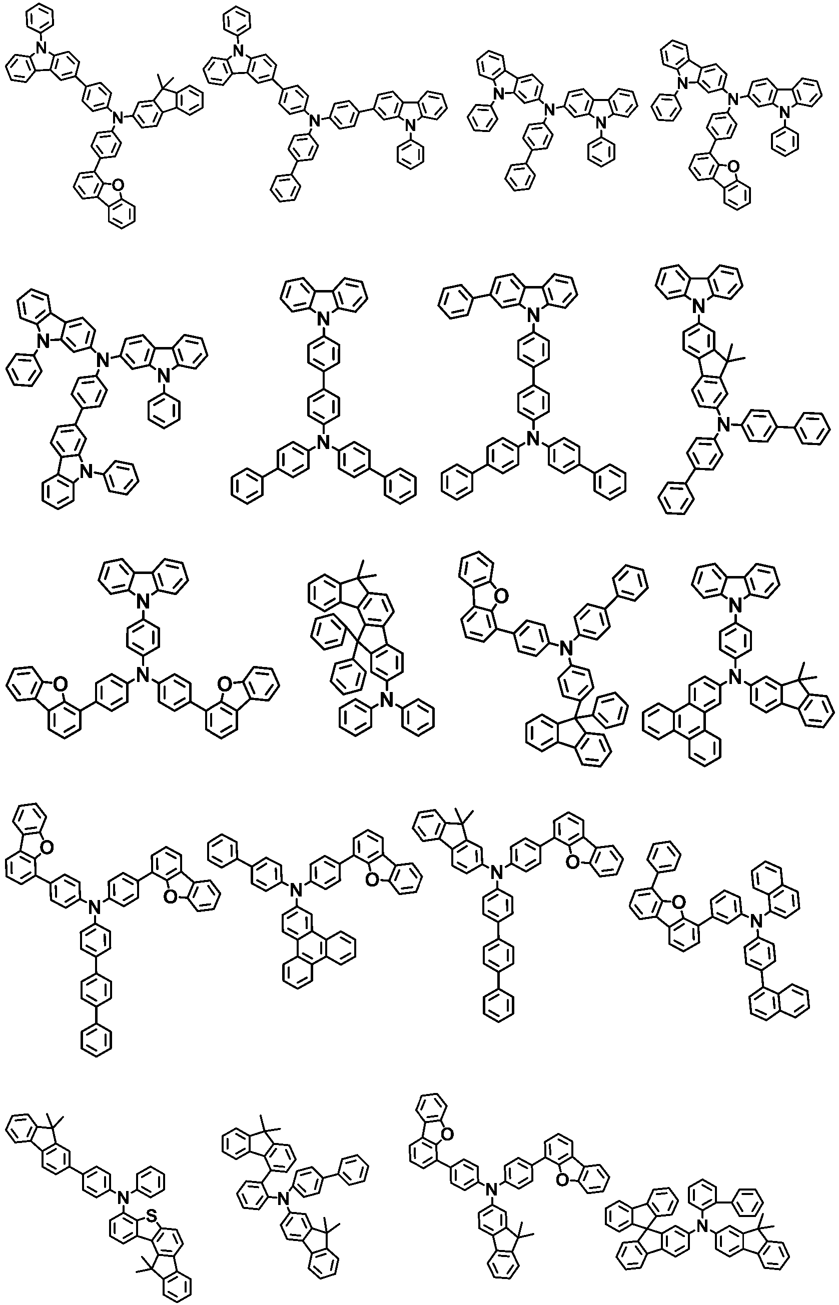

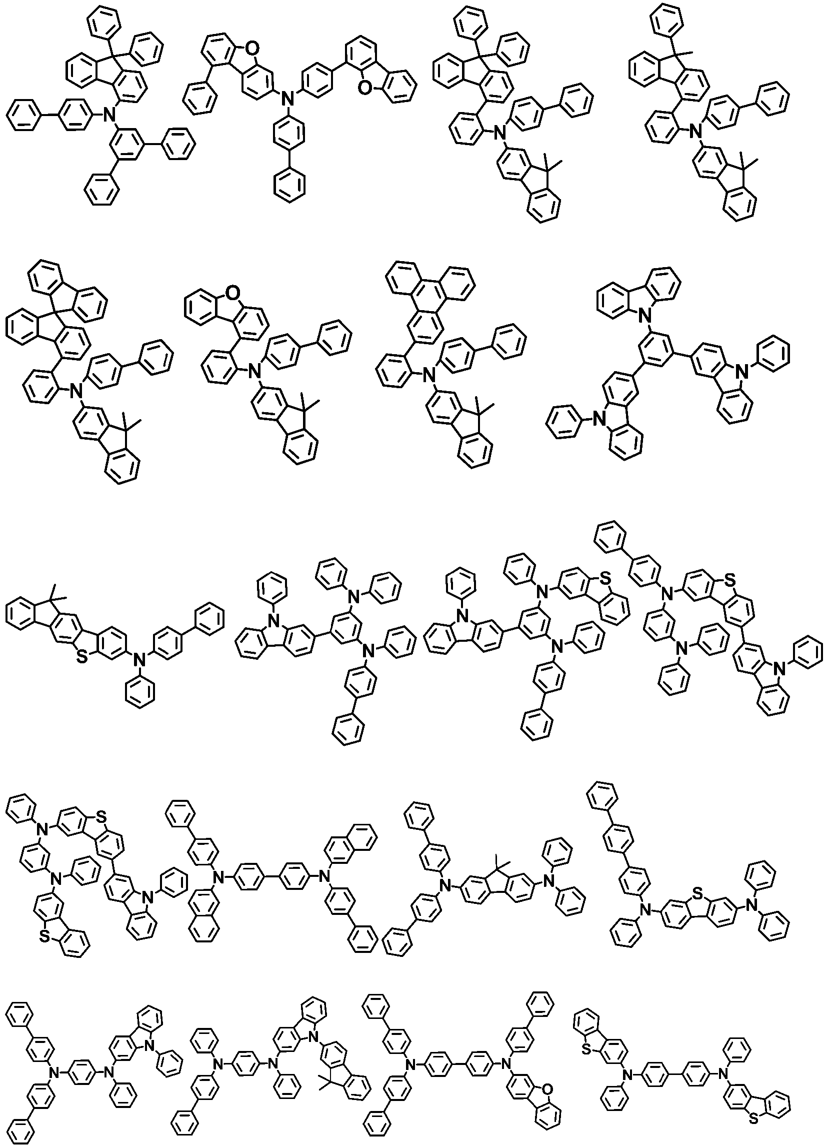

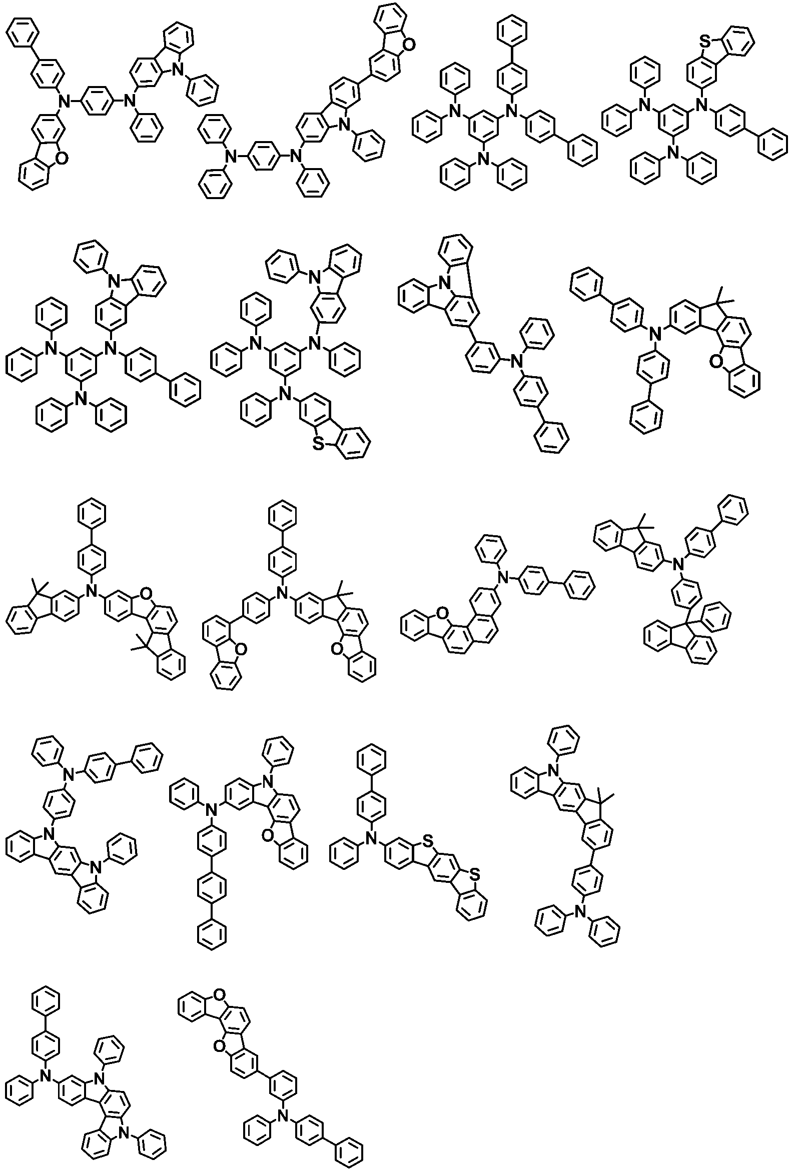

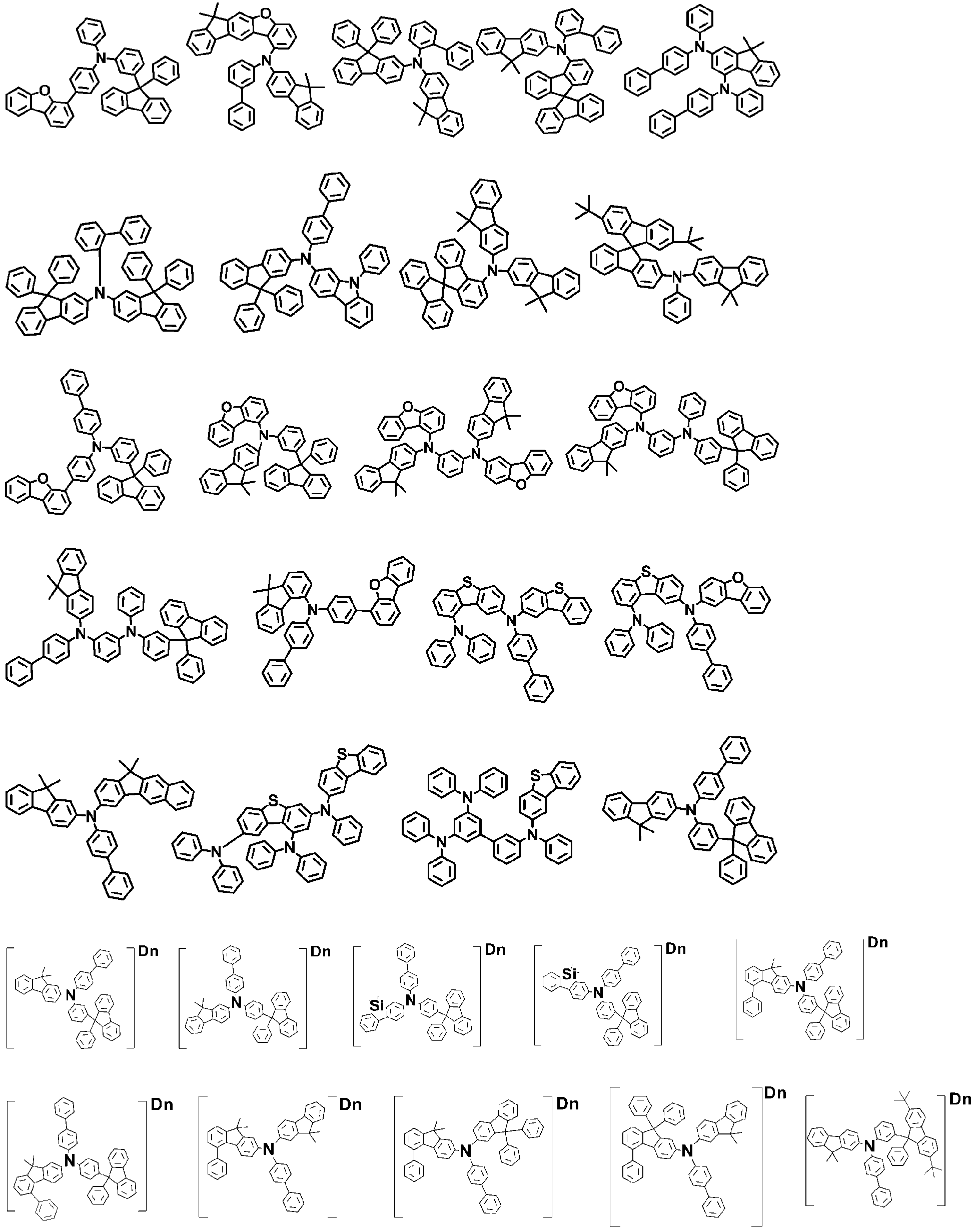

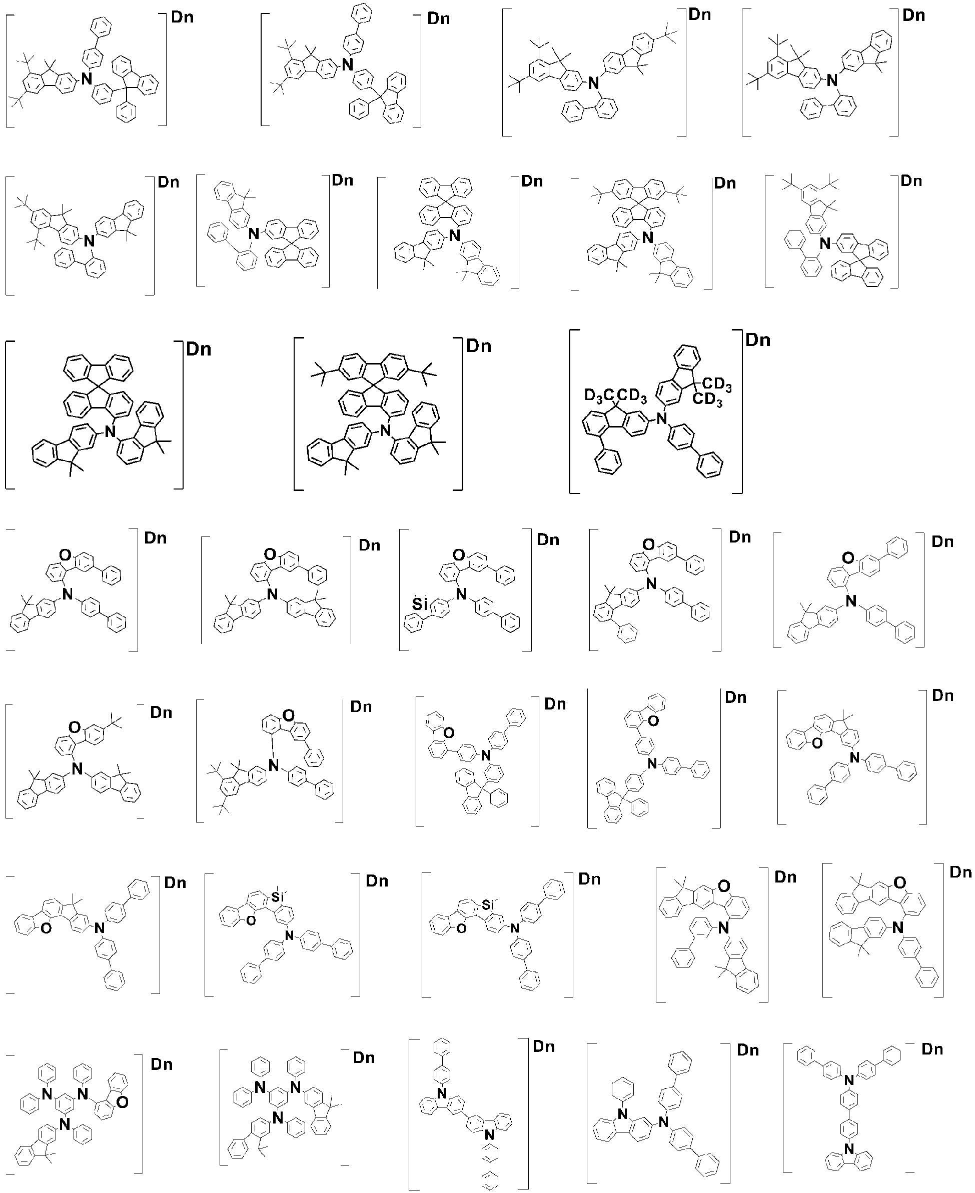

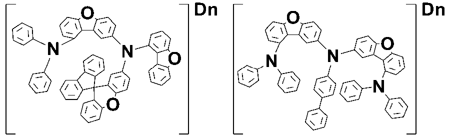

(Dn means the number of substituted deuteriums and indicates a structure in which one or more deuteriums are substituted)

**In** addition to the compounds described above, known compounds and compounds having structures similar thereto described in US5061569A, JP1993-009471A, WO1995-009147A1, JP1995-126615A, JP1998-095973A, and the like may also be used in the hole transport region 140.

Additionally, the charge transport region may be, for example, an electron transport region 150.

The electron transport region 150 may further increase electron injection and/or electron mobility between the cathode 110 and the light emitting layer 130 and block holes.

Specifically, the electron transport region 150 may include an electron transport layer between the cathode 110 and the light emitting layer 130, and an electron transport auxiliary layer between the light emitting layer 130 and the electron transport layer, and at least one of the compounds listed in Group C below may be included in at least one layer of the electron transport layer and the electron transport auxiliary layer.

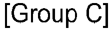

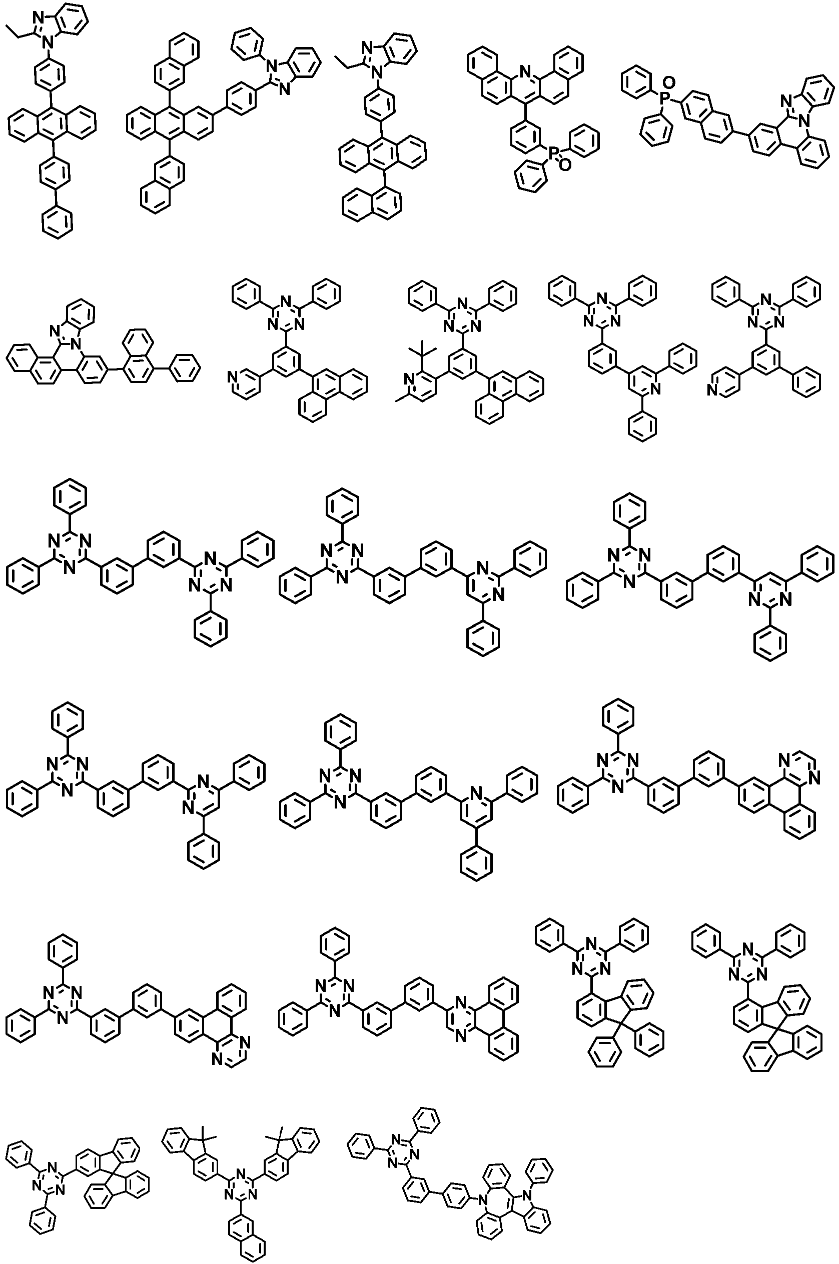

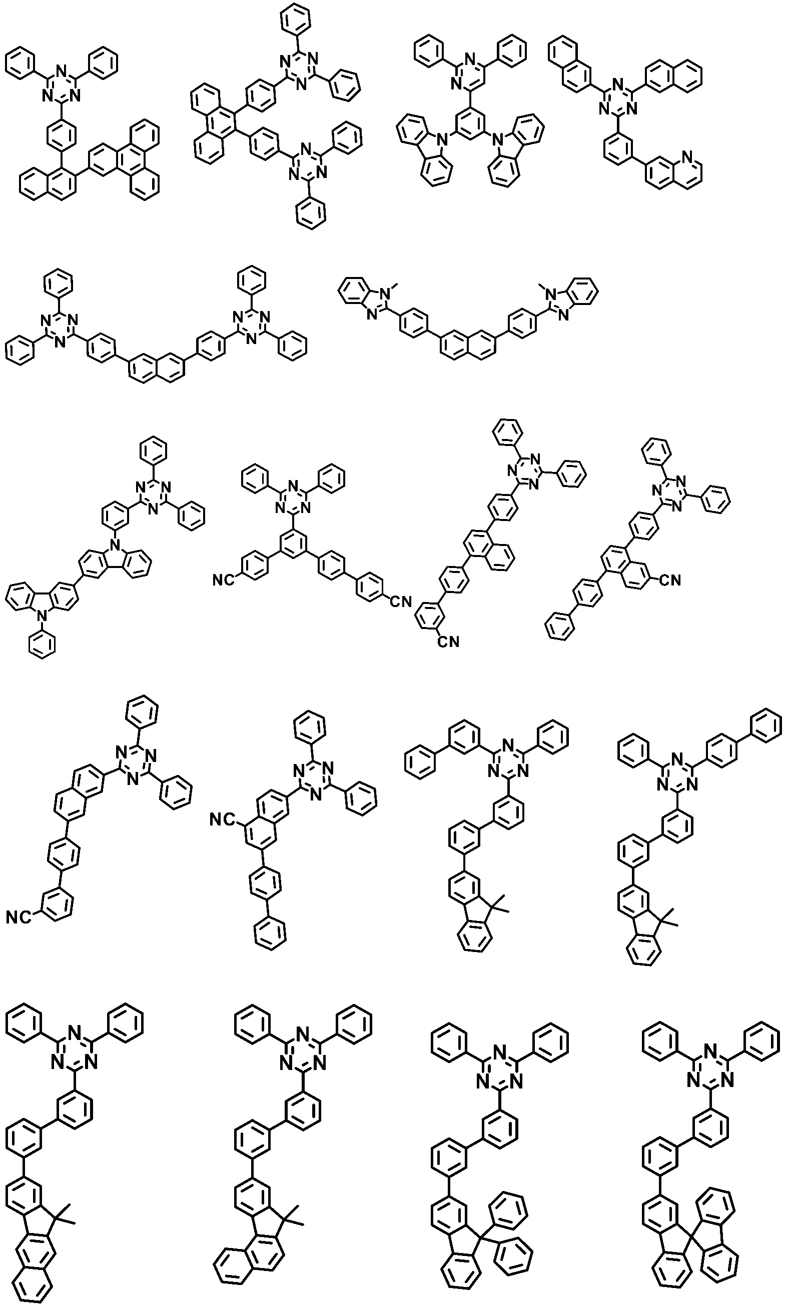

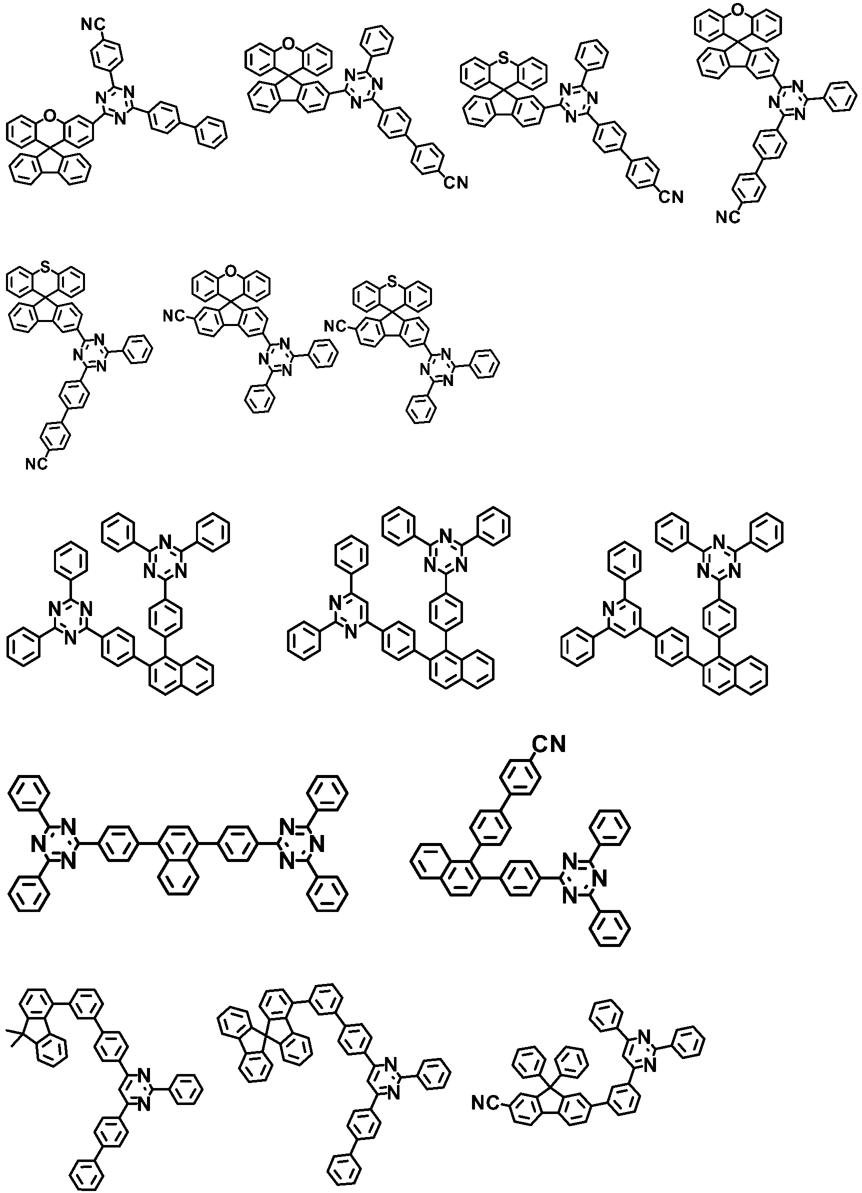

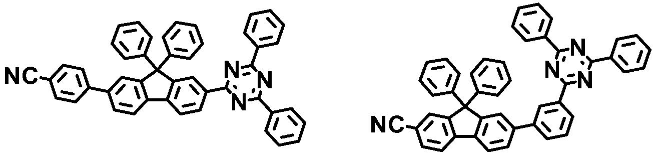

An embodiment may be an organic light emitting diode including a light emitting layer as an organic layer.

Another embodiment may be an organic light emitting diode including a light emitting layer and a hole transport region as an organic layer.

Another embodiment may be an organic light emitting diode including a light emitting layer and an electron transport region as an organic layer.

An organic light emitting diode according to an embodiment of the present invention may include a hole transport region 140 and an electron transport region 150 in addition to a light emitting layer 130 as an organic layer 105 as shown in FIG. 1.

Meanwhile, the organic light emitting diode may additionally include an electron injection layer (not shown), a hole injection layer (not shown), and the like. in addition to the light emitting layer as the aforementioned organic layer.

The organic light emitting diode 100 may be manufactured by forming an anode or a cathode on a substrate, and then forming an organic layer by a dry film method such as vacuum deposition, sputtering, plasma plating and ion plating, and forming a cathode or an anode thereon.

The aforementioned organic light emitting diode may be applied to an organic light emitting display device.

### [Mode for Invention]

Hereinafter, the embodiments are illustrated in more detail with reference to examples.. However, these examples are exemplary, and the present scope is not limited thereto.

Hereinafter, starting materials and reactants used in Examples and Synthesis Examples were purchased from Sigma-Aldrich Co. Ltd., TCI Inc., Tokyo chemical industry, or P&H tech as far as there is no particular comment or were synthesized by known methods.

### (Synthesis of Compound for Organic Optoelectronic Device)

### Synthesis Example 1: Synthesis of Compound A-1

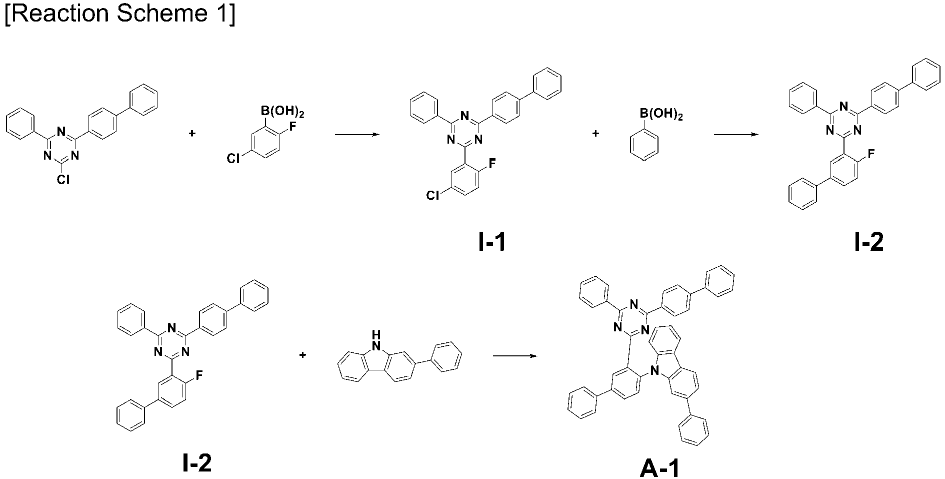

### 1st step: Synthesis of Intermediate I-1

2-([1,1'-biphenyl]-4-yl)-4-chloro-6-phenyl-1,3,5-triazine (58.18 g, 169.15 mmol), 5-chloro-2-fluorophenylboronic acid (28.10 g, 161.21 mmol), K₂CO₃ (44.55 g, 322.30 mmol), and Pd(PPh₃)₄ (9.31 g, 8.06 mmol) were put into a roundbottom flask, dissolved in THF (600 ml) and distilled water (160 ml), and then reflux stirred at 70 °C for 12 hours. After the reaction was completed, an excess of methanol and Ethyl Acetate (hereinafter EA) were added thereto and stirred at room temperature for 30 minutes. The precipitated solid was filtered, added to monochlorobenzene (hereinafter MCB), dissolved by heating, and then filtered through a silica pad. The filtrate was distilled under reduced pressure, and the precipitated solid was filtered to obtain 52.5 g (74%) of intermediate I-1.

### 2nd step : Synthesis of Intermediate I-2

Intermediate I-1 (52.5 g, 119.9 mmol), Phenylboronic acid (43.85 g, 359.67 mmol), Cs₂CO₃ (78.13 g, 239.78 mmol), tri-tert-butylphosphine (9.7 g, 23.98 mmol), and Pd₂(dba)₃ (5.49 g, 5.99 mmol) were put into a round-bottom flask, dissolved in 1,4-Dioxane (450 ml), and then reflux stirred at 120 °C for 8 hours. After the reaction was completed, an excess of distilled water was added to the reaction mixture and stirred for 30 minutes. The precipitated solid was filtered, added to MCB, dissolved by heating, and then filtered through a silica pad. The filtrate was distilled under reduced pressure, and the precipitated solid was filtered to obtain 39.67 g (69%) of intermediate I-2.

### 3rd step : Synthesis of Compound A-1

Intermediate I-2 (18.5 g, 38.58 mmol), 2-phenyl-9H-carbazole (14.08 g, 57.87 mmol), and K₃PO₄ (16.38 g, 77.16 mmol) were put into a round-bottom flask, dissolved in DMF (100 ml), and then reflux stirred at 150 °C for 6 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 24.4 g (90%) of Compound A-1.

### Synthesis Example 2: Synthesis of Compound A-29

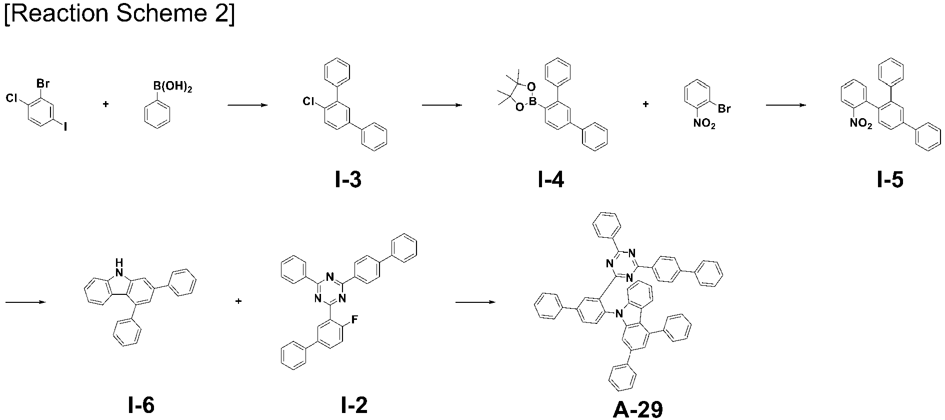

### 1st step : Synthesis of Intermediate I-3

2-bromo-1-chloro-4-iodobenzene (40.0 g, 126.04 mmol), Phenylboronic acid (38.42 g, 315.11 mmol), K₂CO₃ (34.84 g, 252.09 mmol), and Pd(PPh₃)₄ (7.28 g, 6.30 mmol) were put into a round-bottom flask, dissolved in THF (450 ml) and distilled water (120 ml), and then reflux stirred at 70 °C for 12 hours. After the reaction was completed, the aqueous layer was separated and removed, and then the organic solvent was removed under reduced pressure. Using column chromatography (Hexane:DCM (10% to 30%)), 24.03 g (72%) of Intermediate I-3 was obtained.

### 2nd step : Synthesis of Intermediate I-4

Intermediate I-3 (24.03 g, 90.76 mmol), Bis(pinacolato)diboron (29.96 g, 117.99 mmol), Tricyclohexylphosphine (5.46 g, 22.69 mmol), Potassium acetate (17.82 g, 181.53 mmol), and Pd(dppf)Cl₂ (4.16 g, 4.54 mmol) were put into a round-bottom flask, dissolved in Xylene (180 ml), and then reflux stirred at 150 °C for 8 hours. After the reaction was completed, the mixture was cooled to room temperature, filtered to remove salts, and then extracted with an excess of DCM and distilled water. The extracted mixture was subjected to column chromatography (Hexane:DCM (30% to 50%)) to obtain 25.87 g (80%) of intermediate I-4.

### 3rd step : Synthesis of Intermediate I-5

Intermediate I-4 (25.87 g, 72.61 mmol), 1-Bromo-2-nitrobenzene (14.67 g, 72.61 mmol), K₂CO₃ (20.07 g, 145.23 mmol), and Pd(PPh₃)₄ (4.20 g, 3.63 mmol) were put into a round-bottom flask, dissolved in THF (250 ml) and distilled water (80 ml), and then reflux stirred at 70 °C for 12 hours. After the reaction was completed, the aqueous layer was separated and removed, and then the organic solvent was removed under reduced pressure. Using column chromatography (Hexane:DCM (30% to 60%)), 17.86 g (70%) of Intermediate I-5 was obtained.

### 4th step : Synthesis of Intermediate I-6

Intermediate I-5 (17.86 g, 50.82 mmol) and triphenylphosphine (66.65 g, 254.12 mmol) were put into a round-bottom flask, dissolved in o-dichlorobenzene (hereinafter DCB) (170 ml), and then reflux stirred at 200 °C for 12 hours. After the reaction was completed, DCB was removed under reduced pressure, and then column chromatography (Hexane:DCM (20% to 50%)) was used to obtain 14.29 g (88%) of intermediate I-6.

### 5th step: Synthesis of Compound A-29

Intermediate I-2 (12.3 g, 25.65 mmol), intermediate I-6 (11.06 g, 34.63 mmol), and K₃PO₄ (10.89 g, 51.30 mmol) were put into a round-bottom flask, dissolved in DMF (100 ml), and then reflux stirred at 150 °C for 6 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 17.38 g (87%) of Compound A-29.

### Synthesis Example 3: Synthesis of Compound A-76

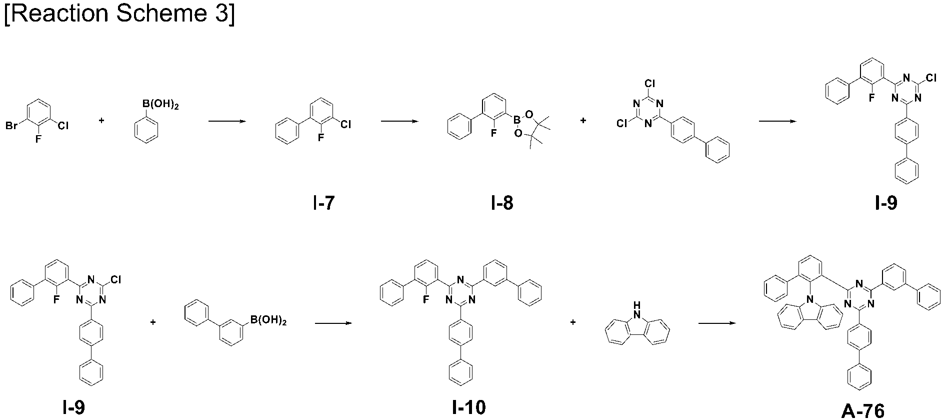

### 1st step : Synthesis of Intermediate I-7

1-Bromo-3-chloro-2-fluorobenzene (80.0 g, 381.97 mmol), Phenylboronic acid (51.23 g, 420.17 mmol), K₂CO₃ (105.58 g, 763.94 mmol), and Pd(PPh₃)₄ (22.07 g, 19.10 mmol) were put into a round-bottom flask, dissolved in THF (1000 ml) and distilled water (380 ml), and then reflux stirred at 70 °C for 12 hours. After the reaction was completed, the aqueous layer was separated and removed, and then the organic solvent was removed under reduced pressure. Using column chromatography (Hexane:DCM (10% to 25%)), 70.30 g (89%) of Intermediate I-7 was obtained.

### 2nd step : Synthesis of Intermediate I-8

Intermediate I-7 (70.30 g, 340.21 mmol), Bis(pinacolato)diboron (103.67 g, 408.25 mmol), tricyclohexylphosphine (16.36 g, 68.04 mmol), potassium acetate (66.78 g, 680.41 mmol), and Pd₂(dba)₃ (9.35 g, 10.21 mmol) were put into a round-bottom flask, dissolved in Xylene (950 ml), and then reflux stirred at 150 °C for 8 hours. After the reaction was completed, the mixture was cooled to room temperature, filtered to remove salts, and then extracted with an excess of DCM and distilled water. The extracted mixture was subjected to column chromatography (Hexane:DCM (30% to 50%)) to obtain 74.20 g (73%) of Intermediate I-8.

### 3rd step : Synthesis of Intermediate I-9

Intermediate I-8 (74.20 g, 248.86 mmol), 2-([1,1'-biphenyl]-4-yl)-4,6-dichloro-1,3,5-triazine (78.96 g, 261.30 mmol), K₂CO₃ (68.79 g, 497.72 mmol), and Pd(dppf)Cl₂ (9.10 g, 12.44 mmol) were put into a round-bottom flask, dissolved in toluene (800 ml) and distilled water (250 ml), and then reflux stirred at 55 °C for 8 hours. After the reaction was completed, the mixture was cooled to room temperature, and the precipitated solid was filtered. The solid was added to MCB, dissolved by heating, and then filtered through a silica pad. The filtrate was distilled under reduced pressure, and the precipitated solid was filtered to obtain 62.93 g (55%) of Intermediate I-9.

### 4th step : Synthesis of Intermediate I-10

Intermediate I-9 (31.9 g, 72.85 mmol), 3-Biphenylboronic acid (15.87 g, 80.13 mmol), K₂CO₃ (20.14 g, 145.70 mmol), and Pd(PPh₃)₄ (4.21 g, 3.64 mmol) were put into a round-bottom flask, dissolved in THF (360 ml) and distilled water (80 ml), and then reflux stirred at 70 °C for 12 hours. After the reaction was completed, the mixture was cooled to room temperature, and the precipitated solid was filtered. The solid was added to MCB, dissolved by heating, and then filtered through a silica pad. The filtrate was distilled under reduced pressure, and the precipitated solid was filtered to obtain 37.24 g (92%) of Itermediate I-10.

### 5th step: Synthesis of Compound A-76

Intermediate I-10 (37.24 g, 67.02 mmol), 9H-Carbazole (16.81 g, 100.53 mmol), and K₃PO₄ (28.45 g, 134.04 mmol) were put into a round-bottom flask, dissolved in DMF (260 ml), and then reflux stirred at 150 °C for 8 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 39.57 g (84%) of Compound A-76.

### Synthesis Example 4: Synthesis of Compound A-81

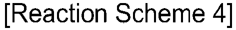

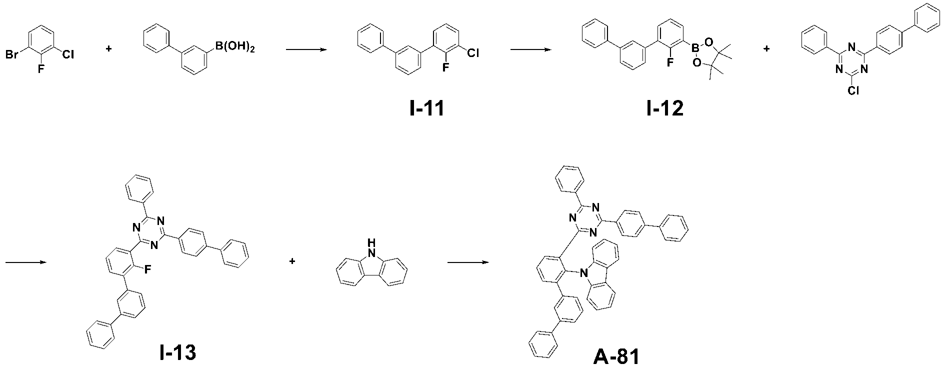

### 1st step : Synthesis of Intermediate I-11

1-Bromo-3-chloro-2-fluorobenzene (40.0 g, 190.99 mmol), 3-Biphenylboronic acid (41.60 g, 210.08 mmol), K₂CO₃ (52.79 g, 381.97 mmol), and Pd(PPh₃)₄ (11.03 g, 9.55 mmol) were put into a round-bottom flask, dissolved in THF (550 ml) and distilled water (190 ml), and then reflux stirred at 70 °C for 12 hours. After the reaction was completed, the aqueous layer was separated and removed, and then the organic solvent was removed under reduced pressure. Using column chromatography (Hexane:DCM (10% to 35%)), 43.20 g (80%) of Itermediate I-11 was obtained.

### 2nd step : Synthesis of Intermediate I-12

Intermediate I-11 (43.20 g, 152.79 mmol), bs(pinacolato)diboron (58.20 g, 229.19 mmol), ticyclohexylphosphine (8.57 g, 30.56 mmol), ptassium acetate (29.99 g, 305.58 mmol), and Pd(dppf)Cl₂ (5.59 g, 7.64 mmol) were put into a round-bottom flask, dissolved in Xylene (750 ml), and then reflux stirred at 150 °C for 8 hours. After the reaction was completed, the mixture was cooled to room temperature, filtered to remove salts, and then extracted with an excess of DCM and distilled water. The extracted mixture was subjected to column chromatography (Hexane:DCM (30% to 50%)) to obtain 51.47 g (90%) of Intermediate I-12.

### 3rd step : Synthesis of Intermediate I-13

Intermediate I-12 (51.47 g, 137.52 mmol), 2-([1,1'-biphenyl]-4-yl)-4-chloro-6-phenyl-1,3,5-triazine (49.65 g, 144.40 mmol), K₂CO₃ (38.01 g, 275.05 mmol), and Pd(PPh₃)₄ (7.95 g, 6.88 mmol) were put into a round-bottom flask, dissolved in THF (500 ml) and distilled water (140 ml), and then reflux stirred at 70 °C for 12 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 64.95 g (85%) of Intermediate I-13.

### 4th step : Synthesis of Compound A-81

Intermediate I-13 (29.55 g, 53.18 mmol), 9H-Carbazole (13.34 g, 79.77 mmol), and K₃PO₄ (22.58 g, 106.36 mmol) were put into a round-bottom flask, dissolved in DMF (250 ml), and then reflux stirred at 150 °C for 8 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 33.64 g (90%) of Compound A-81.

### Synthesis Example 5: Synthesis of Compound A-94

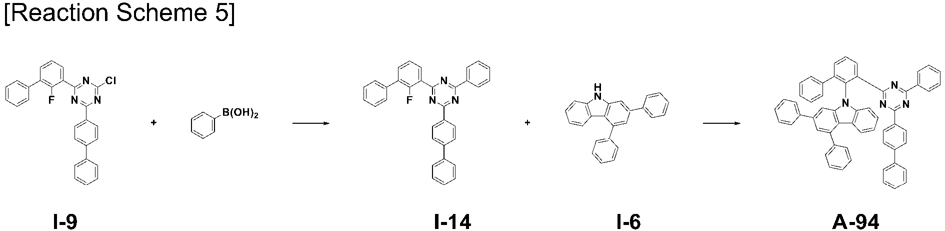

### 1st step : Synthesis of Intermediate I-14

Intermediate I-9 (31.1 g, 71.02 mmol), Phenylboronic acid (10.39 g, 85.22 mmol), K₂CO₃ (19.63 g, 142.04 mmol), and Pd(PPh₃)₄ (4.10 g, 3.55 mmol) were put into a round-bottom flask, dissolved in THF (250 ml) and distilled water (80 ml), and then reflux stirred at 70 °C for 12 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 30.99 g (91%) of Intermediate I-14.

### 2nd step : Synthesis of Compound A-94

Intermediate I-14 (15.9 g, 33.16 mmol), intermediate I-6 (15.89 g, 49.73 mmol), and K₃PO₄ (14.08 g, 66.31 mmol) were put into a round-bottom flask, dissolved in DMF (150 ml), and then reflux stirred at 150 °C for 8 hours. After the reaction was completed, the reactant was slowly poured into an excess of water and then stirred for 30 minutes. The precipitated solid was filtered, added to MCB, dissolved by heating, and then filtered through a silica pad. The filtrate was distilled under reduced pressure, and the precipitated solid was filtered to obtain 23.24 g (90%) of Compound A-94.

### Comparative Synthesis Example 1: Synthesis of Compound R-1

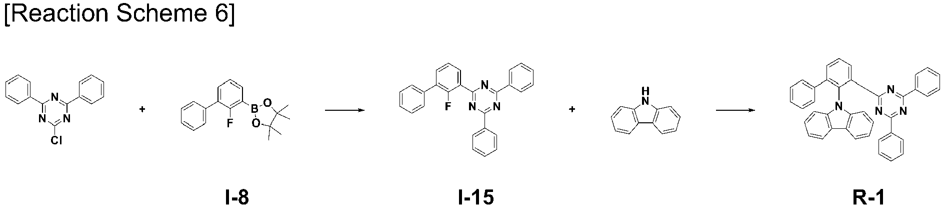

### 1st step: Synthesis of Intermediate I-15

Intermediate I-15 was synthesized in the same manner as in 3rd step of Synthesis Example 4 using 2-chloro-4,6-diphenyl-1,3,5-triazine and Intermediate I-8.

### 2nd step: Synthesis of Compound R-1

Compound R-1 was synthesized in the same manner as in 4th step of Synthesis Example 4 using Intermediate I-15 and 9H-Carbazole.

### Comparative Synthesis Example 2: Synthesis of Compound R-2

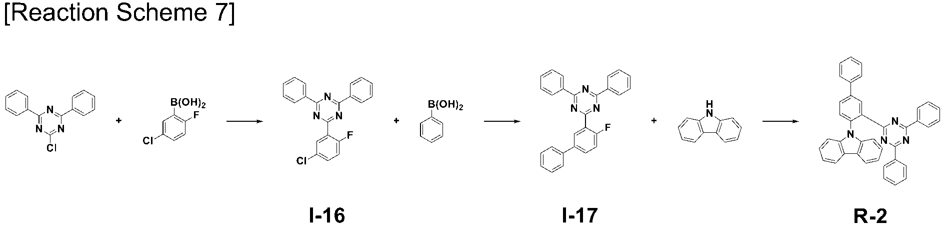

### 1st step : Synthesis of Intermediate I-16

Intermediate I-16 was synthesized in the same manner as in 1st step of Synthesis Example 1 using 2-chloro-4,6-diphenyl-1,3,5-triazine and 5-chloro-2-fluorophenylboronic acid.

### 2nd step: Synthesis of Intermediate I-17

Intermediate I-17 was synthesized in the same manner as in 2nd step of Synthesis Example 1 using Intermediate I-16 and phenylboronic acid.

### 3rd step : Synthesis of Compound R-2

Compound R-2 was synthesized in the same manner as in 4th step of Synthesis Example 4 using Intermediate I-17 and 9H-carbazole.

### Comparative Synthesis Example 3: Synthesis of Compound R-3

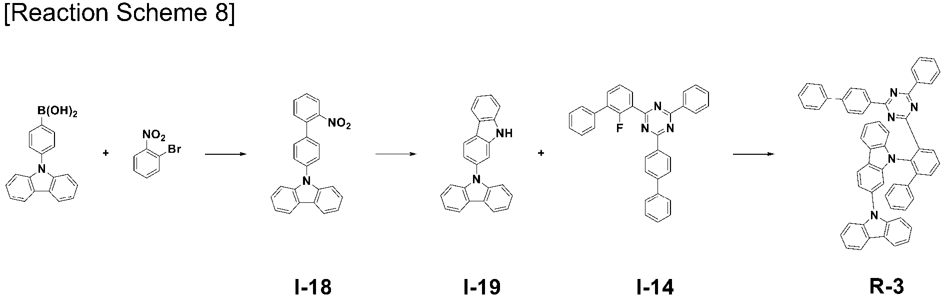

### 1st step : Synthesis of Intermediate I-18

(4-(9H-carbazol-9-yl)phenyl)boronic acid (57.28 g, 199.50 mmol), 1-Bromo-2-nitrobenzene (40.30 g, 199.50 mmol), K₂CO₃ (55.14 g, 398.99 mmol), and Pd(PPh₃)₄ (11.53 g, 9.97 mmol) were put into a round-bottom flask, dissolved in THF (500 ml) and distilled water (200 ml), and then reflux stirred at 70 °C for 8 hours. After the reaction was completed, the aqueous layer was separated and removed, and then the organic solvent was removed under reduced pressure. Using column chromatography (Hexane:DCM (30% to 50%)), 65.43 g (90%) of Intermediate I-18 was obtained.

### 2nd step: Synthesis of Intermediate I-19

Intermediate I-18 (65.43 g, 179.56 mmol) and triphenylphosphine (235.48 g, 897.78 mmol) were put into a round-bottom flask, dissolved in DCB (600 ml), and then reflux stirred at 190 °C for 12 hours. After the reaction was completed, the solvent was removed under reduced pressure, and then an appropriate amount of DCM was added thereto to dissolve the solid. An excess of ethanol was added thereto and stirred for 1 hour, and then the precipitated solid was filtered. The solid was added to MCB, dissolved by heating, and then filtered through a silica pad. The filtrate was distilled under reduced pressure, and the precipitated solid was filtered to obtain 44.76 g (75%) of Intermediate I-19.

### 3rd step : Synthesis of Compound R-3

Compound R-3 was synthesized in the same manner as in 4th step of Synthesis Example 4 using Intermediate I-19 and Intermediate I-14.

### Comparative Synthesis Example 4: Synthesis of Compound R-4

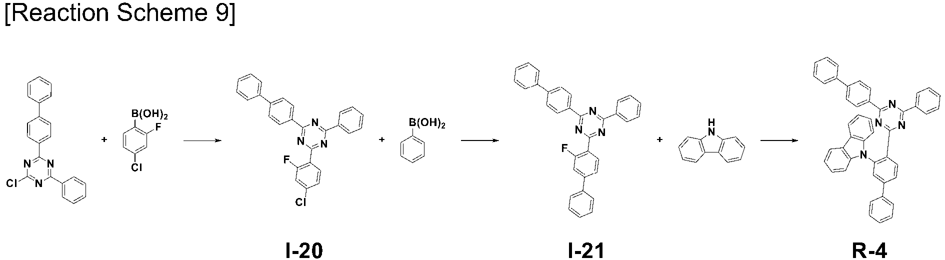

### 1st step : Synthesis of Intermediate I-20

Intermediate I-20 was synthesized in the same manner as in 1st step of Synthesis Example 1 using 2-([1,1'-biphenyl]-4-yl)-4-chloro-6-phenyl-1,3,5-triazine and 4-chloro-2-fluorophenylboronic acid.

### 2nd step : Synthesis of Intermediate I-21

Intermediate I-21 was synthesized in the same manner as in 2nd step of Synthesis Example 1 using Intermediate I-20 and penylboronic acid.

### 3rd step : Synthesis of Compound R-4

Compound R-4 was synthesized in the same manner as in 4th step of Synthesis Example 4 using Intermediate I-21 and 9H-crbazole.

### Example 1: Manufacture of Green Organic Light Emitting Diode (Single Host)

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. The prepared ITO transparent electrode was used as an anode, Compound A doped with 3% NDP-9 (Novaled GmbH) was vacuum-deposited on the ITO substrate to form a 100 Å-thick hole injection layer, and Compound A is deposited on the hole injection layer to a thickness of 1350 Å to form a hole transport layer. Compound B was deposited on the hole transport layer to a thickness of 350 Å to form a hole transport auxiliary layer. On the hole transport auxiliary layer, Compound A-29 was used as a host and PhGD was doped at 7 wt% as a dopant to form a 400 Å-thick light emitting layer by vacuum deposition. Subsequently, Compound C was deposited on the light emitting layer to a thickness of 50 Å to form an electron transport auxiliary layer, and Compound D and Liq were simultaneously vacuum-deposited in a weight ratio of 1:1 to form a 300 Å-thick electron transport layer. LiQ (15 Å) and Al (1200 Å) were sequentially vacuum-deposited on the electron transport layer to form a cathode, thereby manufacturing an organic light emitting diode.

It was manufactured with a structure of ITO/Compound A (3% NDP-9 doping, 100 Å)/Compound A (1350 Å)/Compound B (350 Å)/EML [Host (Compound A-29) : PhGD = 93 wt% : 7 wt%] (400 Å)/Compound C (50 Å)/Compound D:LiQ (300 Å)/LiQ (15 Å)/AI (1200 Å).
Compound A: N-(biphenyl-4-yl)-9,9-dimethyl-N-(4-(9-phenyl-9H-carbazol-3-yl)phenyl)-9H-fluoren-2-amine
Compound B: N-[4-(4-Dibenzofuranyl)phenyl]-N-[4-(9-phenyl-9H-fluoren-9-yl)phenyl][1,1'-biphenyl]-4-amine
Compound C: 2,4-Diphenyl-6-(4',5',6'-triphenyl[1,1':2',1":3",1"':3"',1""-quinquephenyl]-3""-yl)-1,3,5-triazine
Compound D: 2-(1,1'-Biphenyl-4-yl)-4-(9,9-diphenylfluoren-4-yl)-6-phenyl-1,3,5-triazine

### [PhGD]

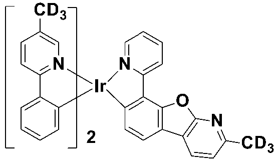

### Examples 2 to 3 and Comparative Examples 1 to 4

Organic light emitting diodes were manufactured in the same method as in Example 1 except for changing to compositions listed in Table 1 below.

### Example 4: Manufacture of Green Organic Light Emitting Diode (Mixed Host)

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. The prepared ITO transparent electrode was used as an anode, Compound E doped with 3% NDP-9 (Novaled GmbH) was vacuum-deposited on the ITO substrate to form a 100 Å-thick hole injection layer, and Compound E is deposited on the hole injection layer to a thickness of 1350 Å to form a hole transport layer. Compound F was deposited on the hole transport layer to a thickness of 320 Å to form a hole transport auxiliary layer. On the hole transport auxiliary layer, Compound A-1 and compound C-4 were simultaneously used as hosts in a weight ratio of 4:6 and PtGD was doped at 10 wt% as a dopant to form a 380 Å-thick light emitting layer by vacuum deposition. Subsequently, Compound G was deposited on the light emitting layer to a thickness of 50 Å to form an electron transport auxiliary layer, and Compound H and Liq were simultaneously vacuum-deposited in a weight ratio of 1:1 to form a 300 Å-thick electron transport layer. LiQ (15 Å) and Al (1200 Å) were sequentially vacuum-deposited on the electron transport layer to form a cathode, thereby manufacturing an organic light emitting diode.

It was manufactured with a structure of ITO/Compound E (3% NDP-9 doping, 100 Å)/Compound E (1350 Å)/Compound F (320 Å)/EML [Host (Compound A-1 : Compound C-4 = 4 : 6 wt%/wt%) : PhGD = 90 wt% : 10 wt%] (380 Å)/Compound G (50 Å)/Compound G: LiQ (300 Å)/LiQ (15 Å)/AI (1200 Å.
Compound E: N-(9,9-diphenyl-9H-fluoren-2-yl)-N,9-diphenyl-9H-carbazol-2-amine
Compound F: 9,9-dimethyl-N-[3-(9-phenyl-9H-fluoren-9-yl)phenyl]-4-(4-phenylphenyl)-9H-fluoren-2-amine
Compound G: 4-{4-[4-(9,9-dimethyl-9H-fluoren-4-yl)phenyl]phenyl}-2-phenyl-6- (4-phenylphenyl)pyrimidine
Compound H: 2-(4-{1-[4-(diphenyl-1,3,5-triazin-2-yl)phenyl]naphthalene-2-yl}-4,6-diphenyl-1,3,5-triazine

### Examples 5 to 7 and Comparative Examples 5 to 8

Organic light emitting diodes were manufactured in the same method as in Example 4 except for changing to compositions listed in Table 2 below.

### Evaluation

Driving voltage, luminous efficiency, and life-span characteristics of the organic light emitting diodes according to Examples 1 to 7 and Comparative Examples 1 to 8 were evaluated.

Specific measurement methods are as follows, and the results are shown in Table 1 to Table 2.

### (1) Measurement of Current Density Change Depending on Voltage Change

The obtained organic light emitting diodes were measured regarding a current value flowing in the unit diode, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

The luminance and the current density measured from (1) and (2) above and a voltage were used to calculate the current efficiency (cd/A) at the same current density (10 mA/cm²).

The luminous efficiency values of Examples 1 to 3 and Comparative Examples 1 to 4 were calculated as relative values based on Comparative Example 1 and listed in Table 1 below.

The luminous efficiency values of Examples 4 to 7 and Comparative Examples 5 to 8 were calculated as relative values based on Comparative Example 5 and listed in Table 2 below.

### (4) Life-span Measurement

The luminance (cd/m²) was maintained at 24000 cd/m², and the time for the current efficiency (cd/A) to decrease to 97% was measured to obtain the results.

The life-span measurement values of Examples 1 to 3 and Comparative Examples 1 to 4 were calculated as relative values based on Comparative Example 1 and are shown in Table 1 below.

The life-span measurement values of Examples 4 to 7 and Comparative Examples 5 to 8 were calculated as relative values based on Comparative Example 5 and are shown in Table 2 below.

### (5) Measurement of Driving Voltage

The driving voltage of each diode was measured at 15 mA/cm² using a current-voltage meter (Keithley 2400) to obtain results.

The driving voltages of Examples 1 to 3 and Comparative Examples 1 to 4 were calculated as relative values based on Comparative Example 1 and listed in Table 1 below.

**(Table 1)**

| No. | Compound | Driving voltage (%) | Luminous efficiency (%) | Life-span (%) |
|---|---|---|---|---|
| Example 1 | A-29 | 94 | 104 | 112 |
| Example 2 | A-76 | 95 | 105 | 122 |
| Example 3 | A-81 | 95 | 107 | 116 |
| Comparative Example 1 | R-1 | 100 | 100 | 100 |
| Comparative Example 2 | R-2 | 99 | 95 | 105 |
| Comparative Example 3 | R-3 | 110 | 101 | 88 |
| Comparative Example 4 | R-4 | 97 | 102 | 90 |

**(Table 2)**

| No. | Compound | | Luminous efficiency (%) | Life-span (%) |
|---|---|---|---|---|
| | First compound | Second compound | | |
| Example 4 | A-1 | C-4 | 106 | 115 |
| Example 5 | A-76 | C-5 | 109 | 132 |
| Example 6 | A-81 | C-5 | 113 | 107 |
| Example 7 | A-94 | C-4 | 107 | 120 |
| Comparative Example 5 | R-1 | C-5 | 100 | 100 |
| Comparative Example 6 | R-2 | C-5 | 98 | 84 |
| Comparative Example 7 | R-3 | C-4 | 99 | 50 |
| Comparative Example 8 | R-4 | C-4 | 102 | 78 |

Referring to Table 1 and Table 2, it can be confirmed that the organic light emitting diodes according to Examples 1 to 7 exhibited significantly improved driving voltage, luminous efficiency, and life-span characteristics compared to the organic light emitting diodes according to Comparative Examples 1 to 8.

While this disclosure has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

## Claims

1. A compound for an organic optoelectronic device represented by Chemical Formula 1A or Chemical Formula 1B: 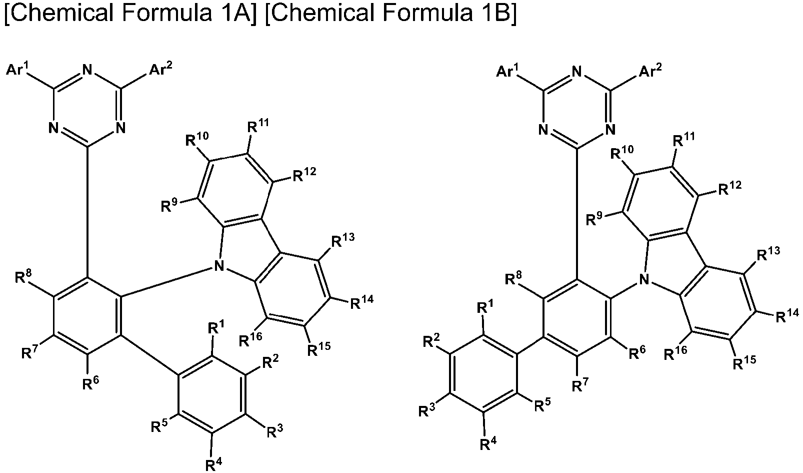 wherein, in Chemical Formula 1,
Ar¹ and Ar² are each independently a phenyl group unsubstituted or substituted with deuterium or a C6 to C20 aryl group; a biphenyl group unsubstituted or substituted with deuterium or a C6 to C20 aryl group; or a terphenyl group unsubstituted or substituted with deuterium or a C6 to C20 aryl group,
provided that at least one of Ar1 and Ar2 is a biphenyl group unsubstituted or substituted with deuterium or a C6 to C20 aryl group; or a terphenyl group unsubstituted or substituted with deuterium or a C6 to C20 aryl group,
R¹ to R⁵ are each independently hydrogen, deuterium, or a substituted or unsubstituted phenyl group,
R⁶ to R⁸ are each independently hydrogen or deuterium, and
R⁹ to R¹⁶ are each independently hydrogen, deuterium or a substituted or unsubstituted C6 to C20 aryl group.

2. The compound for the organic optoelectronic device as claimed in claim 1, wherein
Ar¹ and Ar² are each independently a phenyl group unsubstituted or substituted with deuterium, a phenyl group, or a biphenyl group; a biphenyl group unsubstituted or substituted with deuterium, a phenyl group, or a biphenyl group; or a terphenyl group unsubstituted or substituted with deuterium, a phenyl group, or a biphenyl group.

3. The compound for the organic optoelectronic device as claimed in claim 1, wherein
R⁹ to R¹⁶ are each independently hydrogen, deuterium, or a substituted or unsubstituted C6 to C12 aryl group.

4. The compound for the organic optoelectronic device as claimed in claim 1, wherein
R⁹ to R¹⁶ are each independently hydrogen, deuterium, a substituted or unsubstituted phenyl group, or a substituted or unsubstituted biphenyl group.

5. The compound for the organic optoelectronic device as claimed in claim 1, wherein
the compound is one selected from compounds listed in Group 1: 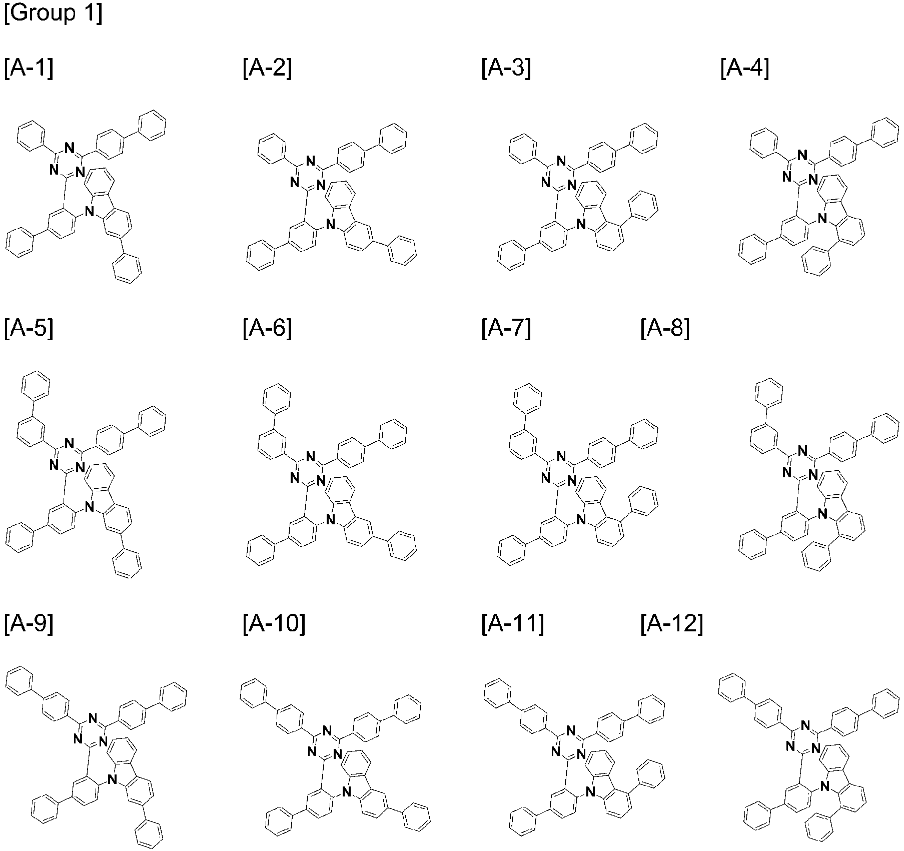 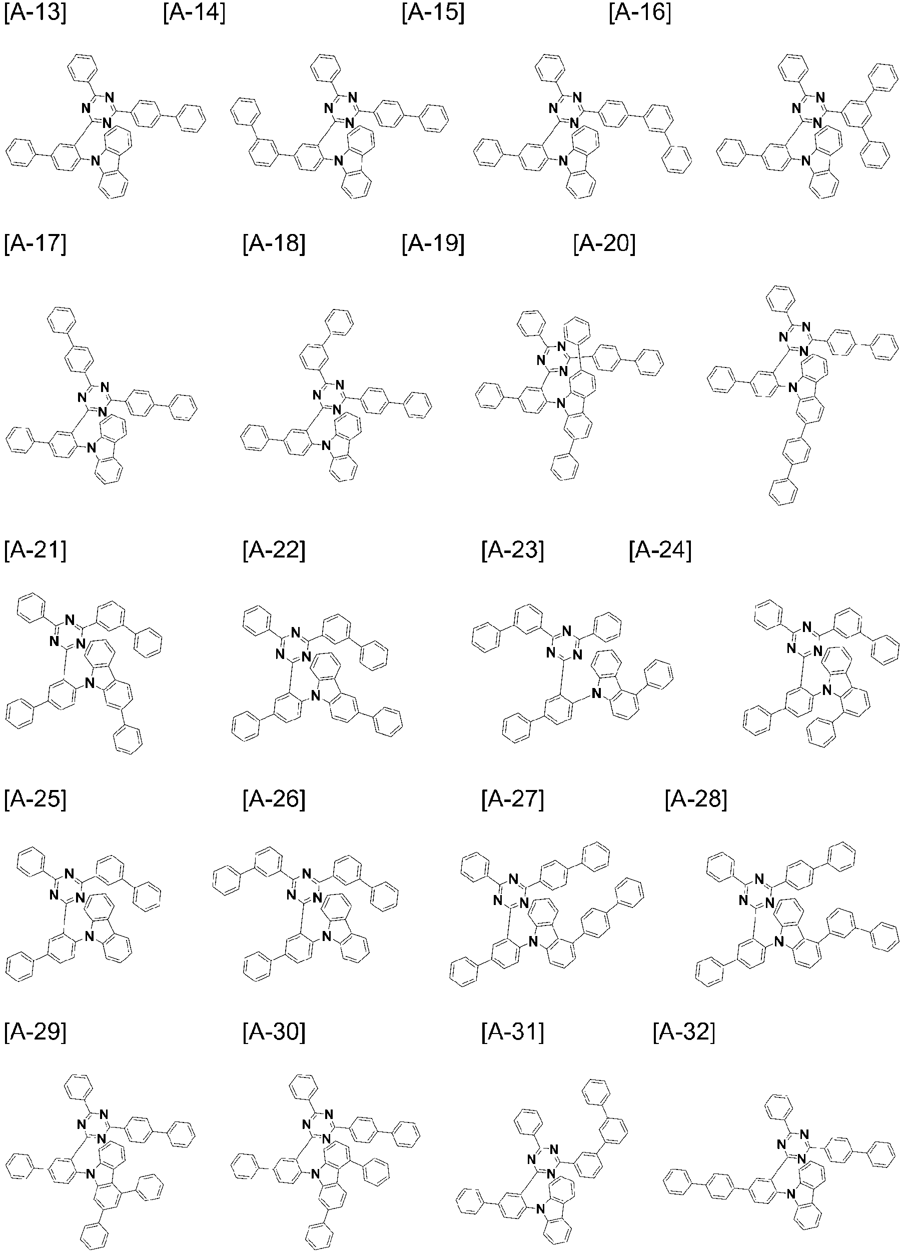 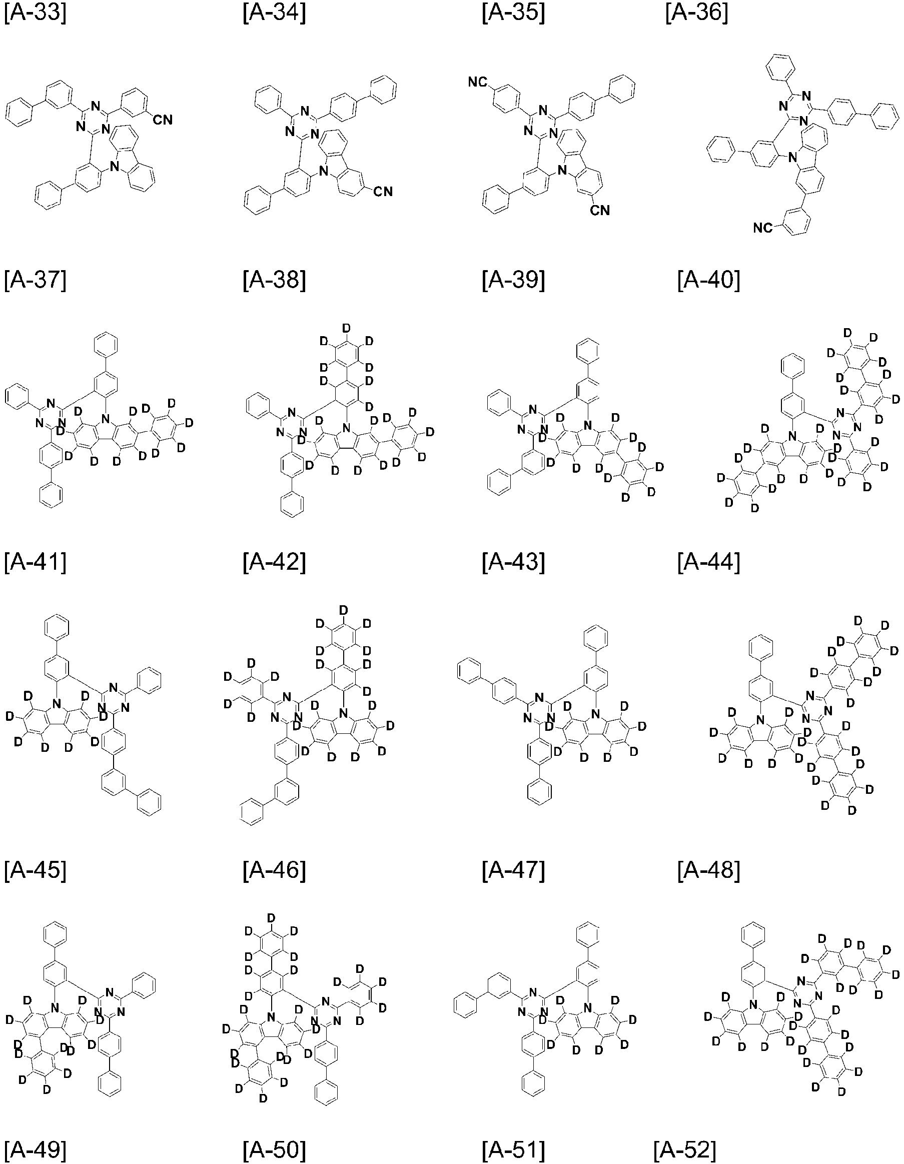 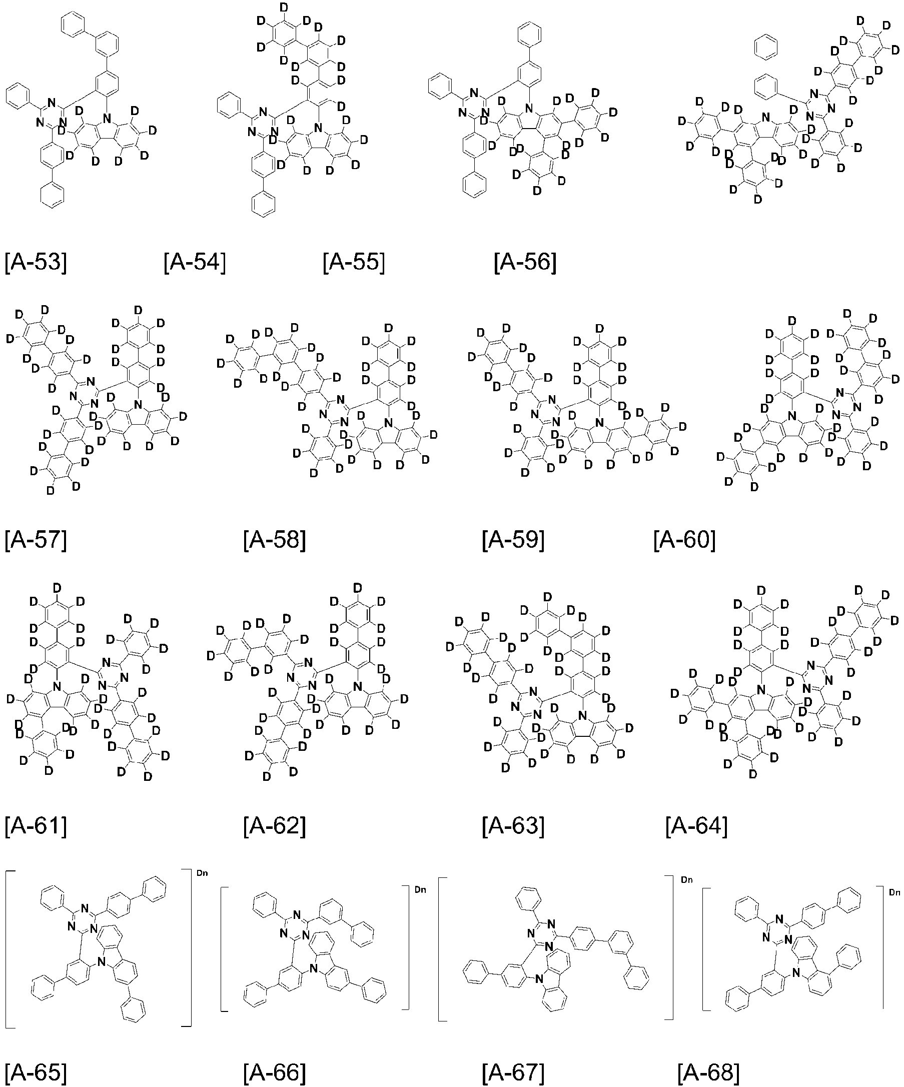 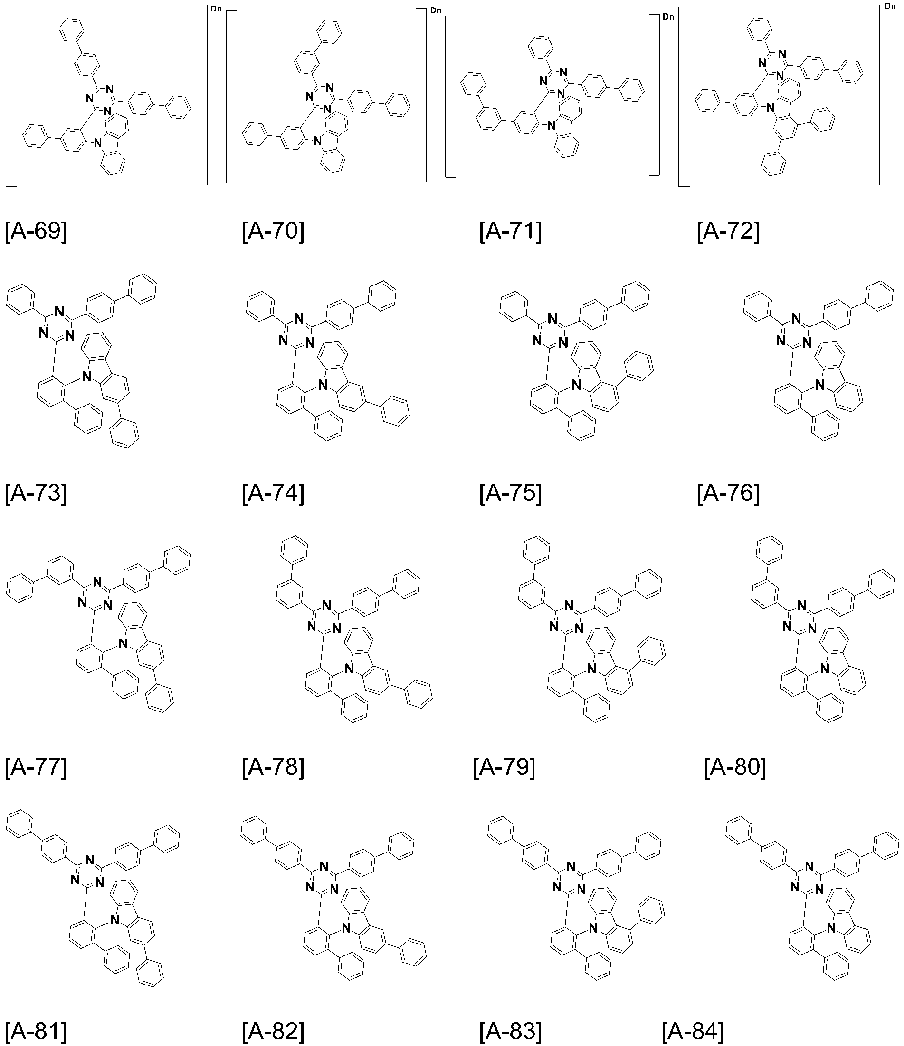 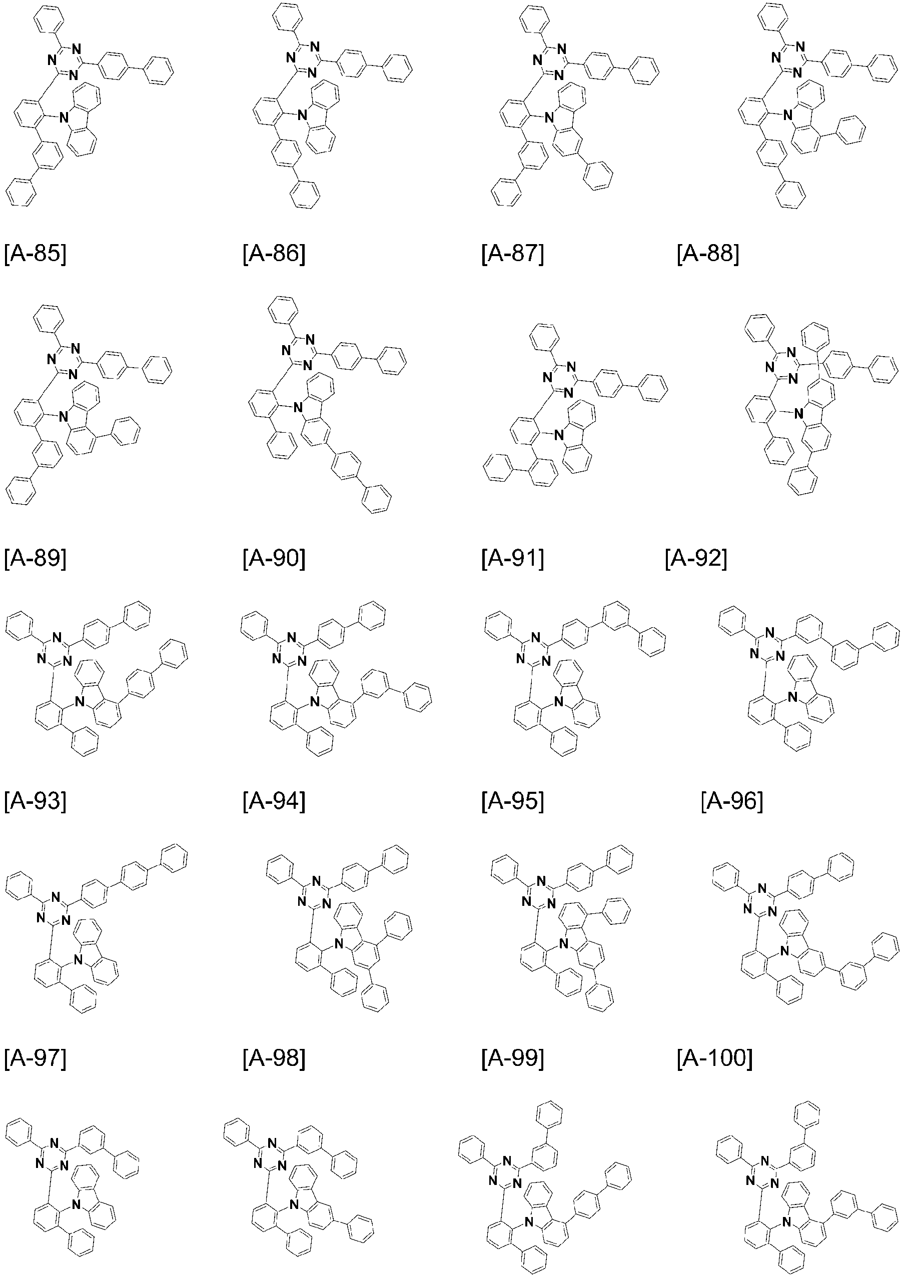 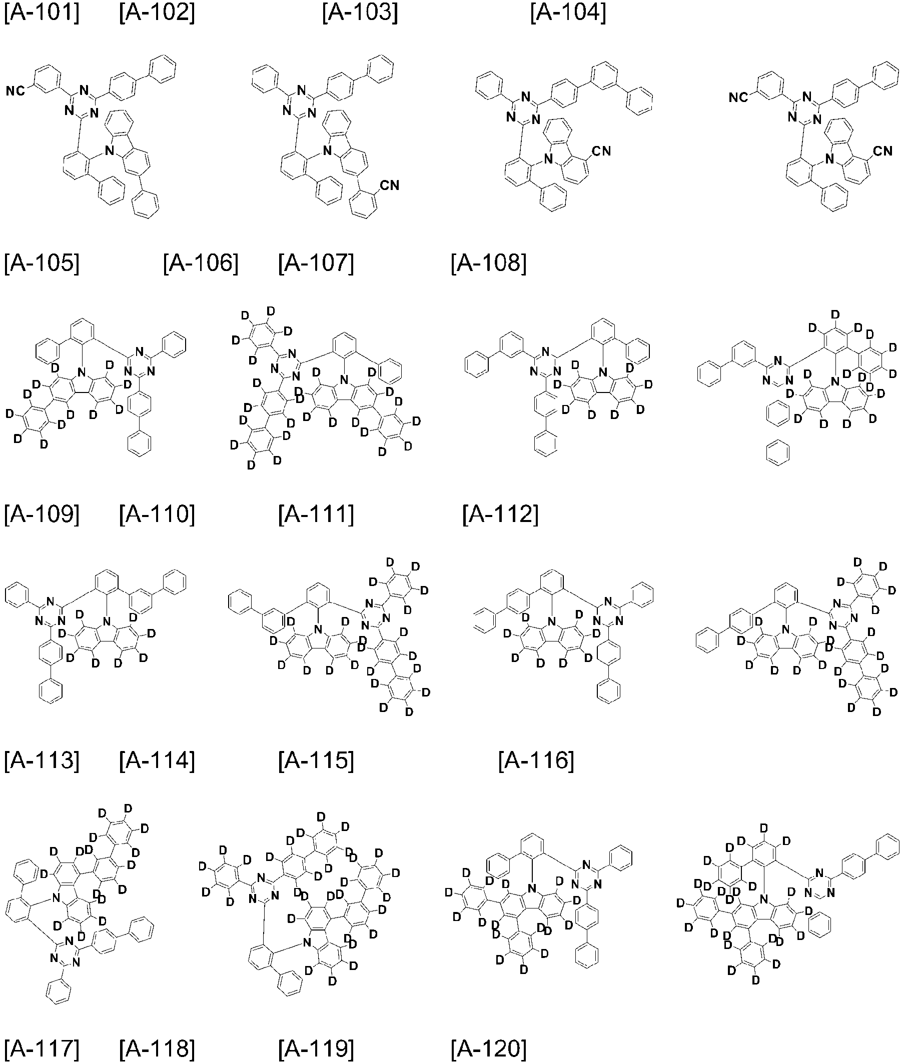 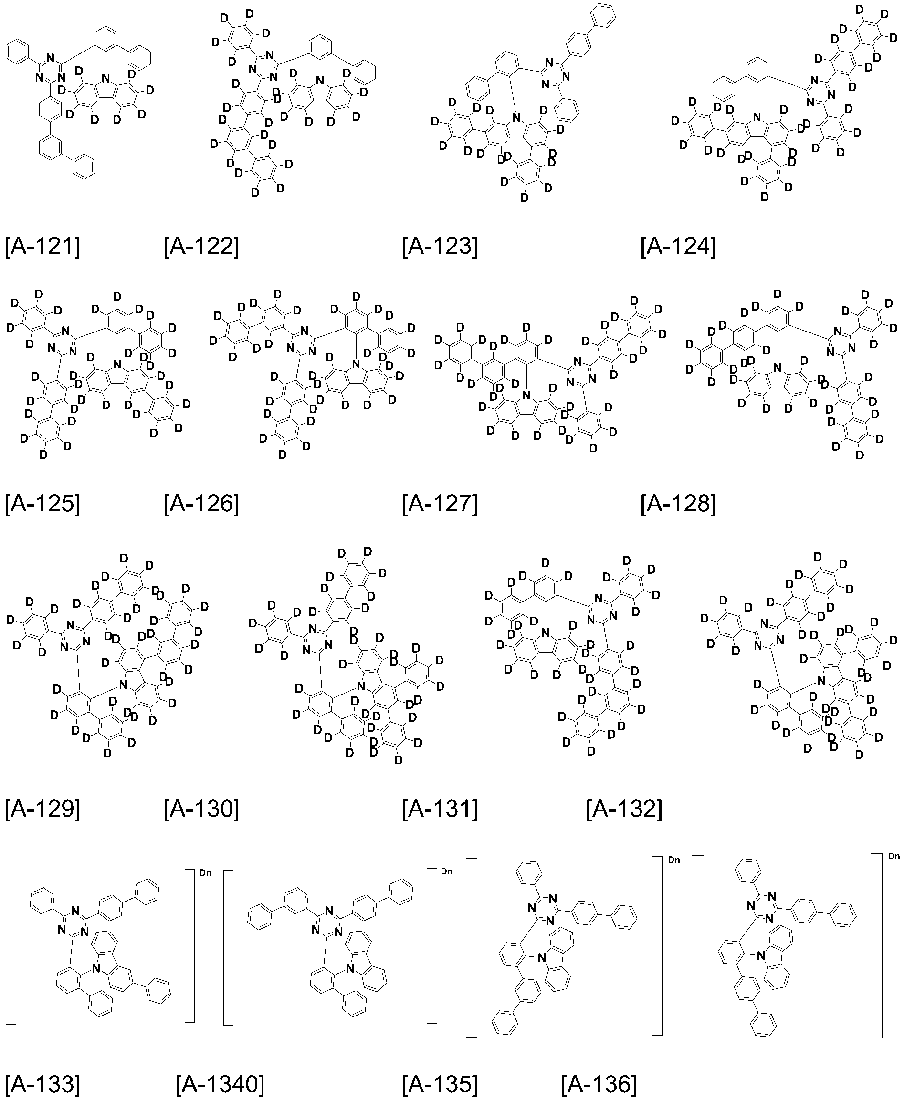 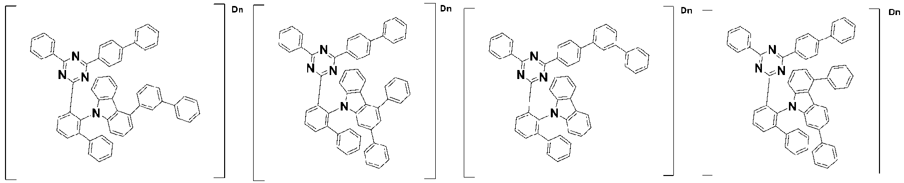
(Dn means the number of substituted deuteriums and indicates a structure in which one or more deuteriums are substituted).

6. A composition for an organic optoelectronic device, comprising
a first compound and a second compound,
wherein the first compound is the compound for the organic optoelectronic device according to claim 1, and
the second compound is represented by Chemical Formula 2; a combination of Chemical Formula 3 and Chemical Formula 4; or Chemical Formula 5: 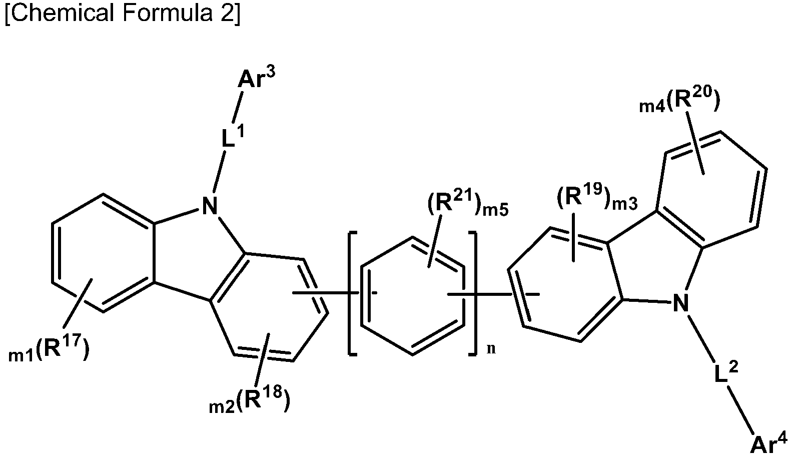
wherein, in Chemical Formula 2,
R¹⁷ to R²¹ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L¹ and L² are each independently a single bond or a substituted or unsubstituted C6 to C20 arylene group,
m1, m4, and m5 are each independently one of integers from 1 to 4,
m2 and m3 are each independently one of integers from 1 to 3, and
n is one of integers from 0 to 2; 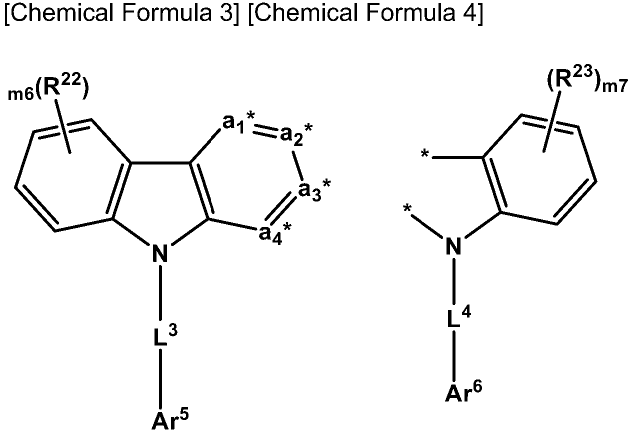
wherein, in Chemical Formula 3 and Chemical Formula 4,
among a₁* to a₄* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L³, and L⁴ are each independently a single bond or a substituted or unsubstituted C6 to C20 arylene group,
R^{a}, R²², and R²³ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m6 and m7 are each independently one of integers from 1 to 4; 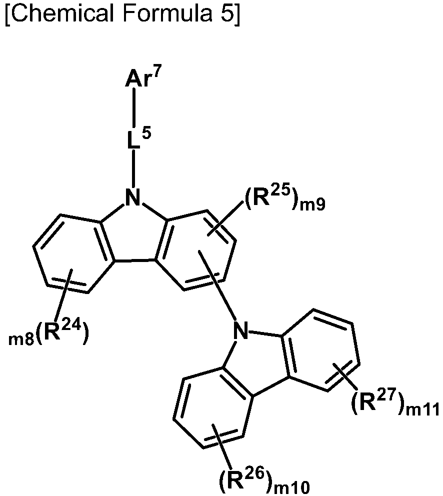
wherein, in Chemical Formula 5,
L⁵ is each independently a single bond or a substituted or unsubstituted C6 to C20 arylene group,
R²⁴ to R²⁷ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m8, m10, and m11 are each independently one of integers from 1 to 4, and
m9 is one of integers from 1 to 3.

7. The composition for the organic optoelectronic device as claimed in claim 6, wherein
Chemical Formula 2 is represented by Chemical Formula 2-8: 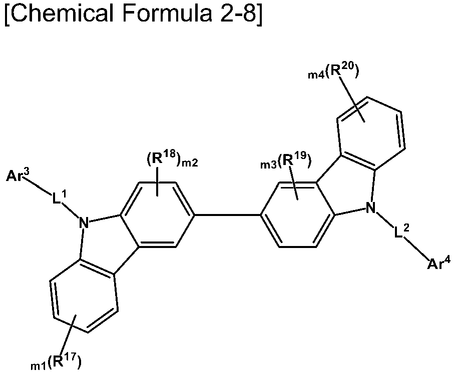
wherein, in Chemical Formula 2-8,
R¹⁷ to R²⁰ are each independently hydrogen, deuterium, or a substituted or unsubstituted C6 to C12 aryl group,
m1 and m4 are each independently one of integers from 1 to 4,
m2 and m3 are each independently one of integers from 1 to 3, and
L¹-Ar³ and L²-Ar⁴ are each independently one of the substituents listed in Group I , 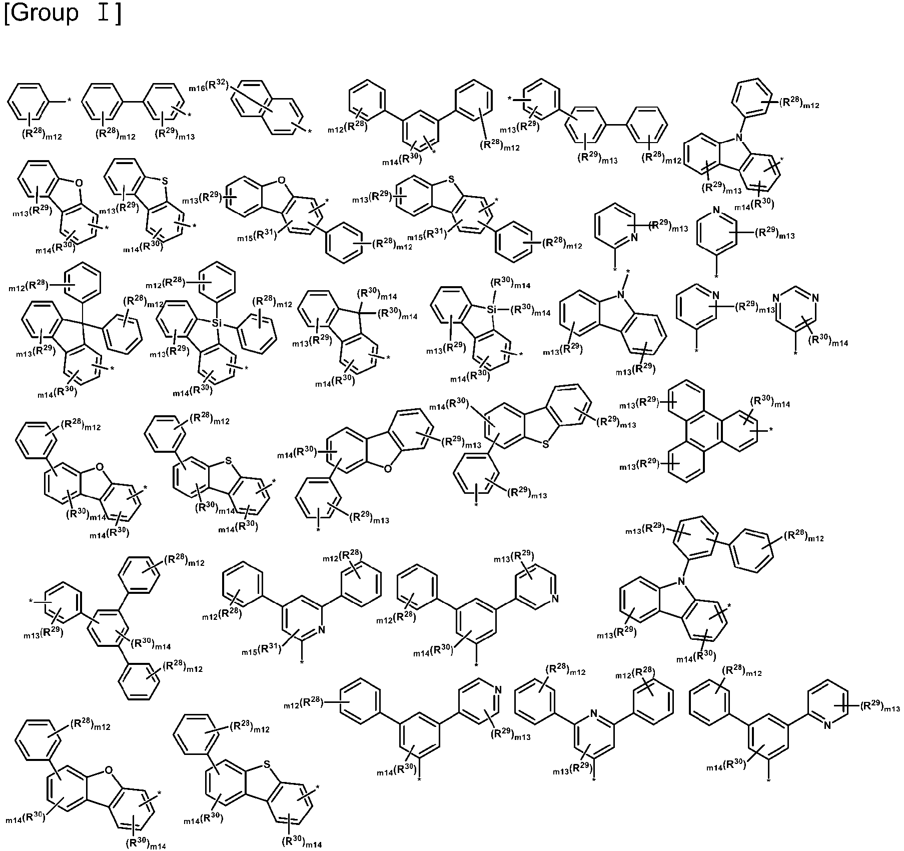 wherein, in Group I,
R²⁸ to R³² are each independently hydrogen, deuterium, a cyano group, a C1 to C10 alkyl group, or a C6 to C12 aryl group,
m12 is one of integers from 1 to 5,
m13 is one of integers from 1 to 4,
m14 is one of integers from 1 to 3,
m15 is an integer of 1 or 2,
m16 is one of integers from 1 to 7, and
* is a linking point.

8. The composition for the organic optoelectronic device as claimed in claim 6, wherein
the combination of Chemical Formula 3 and Chemical Formula 4 is represented by Chemical Formula 3C: 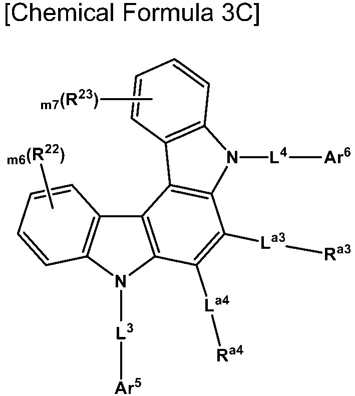
wherein, in Chemical Formula 3C,
L^{a3} and L^{a4} are a single bond,
R²², R²³, R^{a3}, and R^{a4} are each independently hydrogen, deuterium, or a C6 to C12 aryl group,
m6 and m7 are each independently one of integers from 1 to 4, and
L³-Ar⁵ and L⁴-Ar⁶ are each independently one of the substituents listed in Group I , 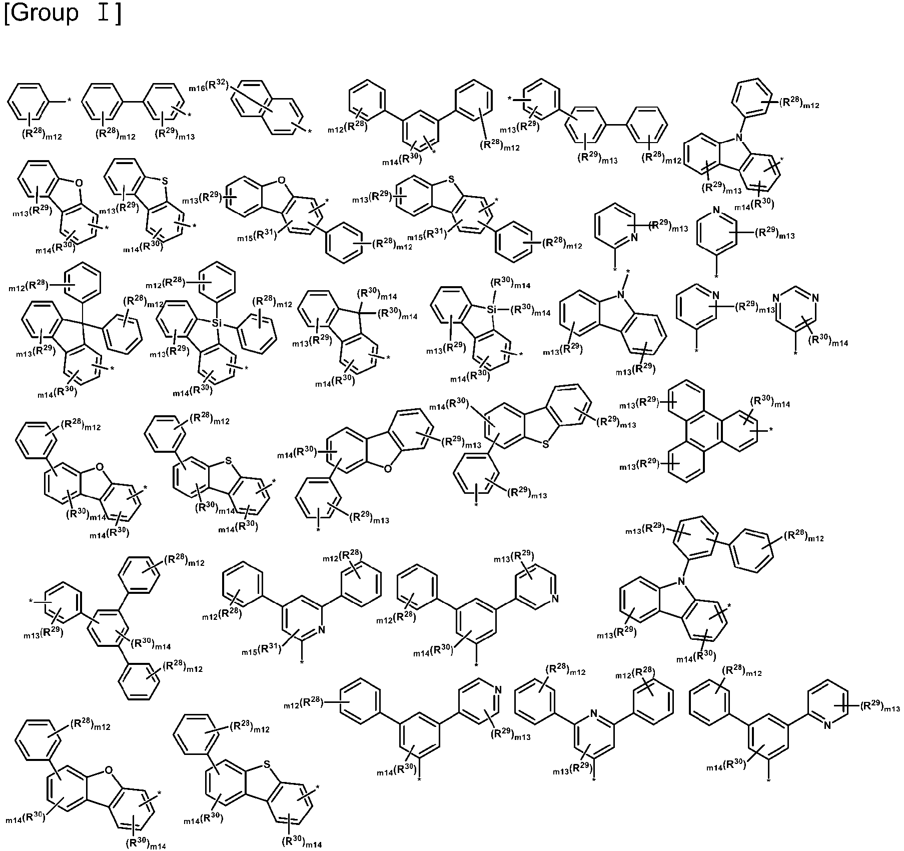 wherein, in Group I,
R²⁸ to R³² are each independently hydrogen, deuterium, a cyano group, a C1 to C10 alkyl group, or a C6 to C12 aryl group,
m12 is one of integers from 1 to 5,
m13 is one of integers from 1 to 4,
m14 is one of integers from 1 to 3,
m15 is an integer of 1 or 2,
m16 is one of integers from 1 to 7, and
* is a linking point.

9. An organic optoelectronic device, comprising
an anode and a cathode facing each other, and
at least one organic layer between the anode and the cathode,
wherein the organic layer comprises
the compound for the organic optoelectronic device as claimed in any one of claims 1 to 5; or
the composition for the organic optoelectronic device as claimed in any one of claims 6 to 8.

10. The organic optoelectronic device as claimed in claim 9, wherein
the organic layer comprises a light emitting layer, and
the light emitting layer comprises the compound for the organic optoelectronic device or the composition for the organic optoelectronic device.

11. A display device comprising the organic optoelectronic device as claimed in claim 9.
